# EUROPÄISCHE PATENTSCHRIFT

(11) **EP 1 317 427 B1**
(45) Veröffentlichungstag und Bekanntmachung des Hinweises auf die Patenterteilung: **15.12.2004**
(21) Anmeldenummer: 01980386.5
(22) Anmeldetag: 14.09.2001
(51) Int. Cl.: C07C 323/60, A61K 31/10, A61P 25/04

(54) **VERFAHREN ZUR HERSTELLUNG CHIRALER VERBINDUNGEN**
METHOD FOR PRODUCING CHIRAL COMPOUNDS
PROCEDE POUR PRODUIRE DES COMPOSES CHIRAUX

(30) Priorität: 14.09.2000 DE 10045832
(43) Veröffentlichungstag der Anmeldung: 11.06.2003
(73) Patentinhaber: Grünenthal GmbH, 52078 Aachen (DE)
(72) Erfinder: GERLACH, Matthias, 63636 Brachttal (DE); PÜTZ, Claudia, 52349 Düren (DE); ENDERS, D., 52074 Aachen (DE); GAUBE, Gero, 52062 Aachen (DE)
(86) Internationale Anmeldenummer: PCT/EP2001/010626
(87) Internationale Veröffentlichungsnummer: WO 2002/022569

(56) Entgegenhaltungen:
- GB-A- 2 217 706
- K. TOMIOKA ET AL.: "Diastereoselective Thiophenol Addition ..." J. ORG. CHEM., Bd. 60, 1995, Seiten 6188-6190, XP002190371 in der Anmeldung erwähnt

## Beschreibung

Die Erfindung betrifft Verfahren zur Herstellung chiraler Verbindungen unter den Bedingungen einer 1,4-Michael-Addition sowie entsprechende Verbindungen.

### Asymmetrische Synthese

Zentrales Thema dieser Anmeldung ist die asymmetrische Synthese. Ein Kohlenstoffatom kann vier Bindungen ausbilden, die räumlich tetraedrisch ausgerichtet sind. Wenn ein Kohlenstoffatom vier unterschiedliche Substituenten trägt, sind zwei Anordnungen möglich, die sich zueinander wie Bild und Spiegelbild verhalten. Diese werden Enantiomere genannt. Die chiralen (von gr. cheir = Hand abgeleitet) Moleküle besitzen keine Drehspiegelachse. Sie unterscheiden sich lediglich durch eine ihrer physikalischen Eigenschaften, nämlich die Richtung in der sie linear polarisiertes Licht um den gleichen Betrag drehen. in achiraten Umgebungen besitzen die beiden Enantiomere die gleichen chemischen, biologischen und physikalischen Eigenschaften. Dagegen können in chiralen Umgebungen, wie z.B. im menschlichen Körper, ihre Eigenschaften sehr unterschiedlich sein.

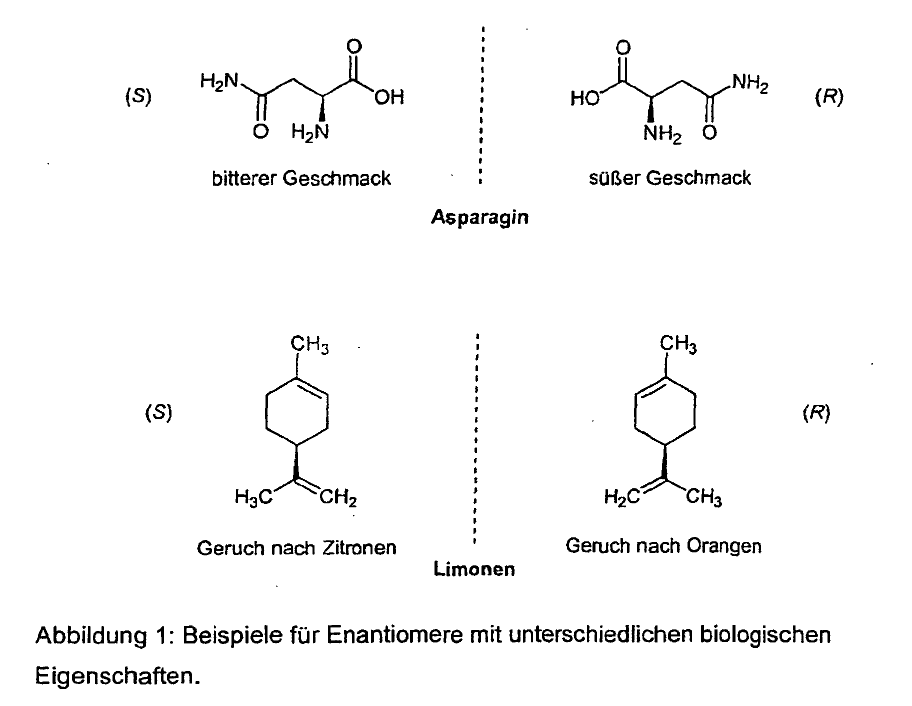

Dort wechselwirken die Enantiomere jeweils unterschiedlich mit Rezeptoren und Enzymen, so daß es in der Natur zu unterschiedlichen physiologischen Auswirkungen kommen kann (siehe Abbildung 1)[1]. Beispielsweise besitzt die (S)-Form (S von lat. sinister = links) von Asparagin einen bitteren Geschmack, während die (R)-Form (R von lat. rectus = rechts) süß schmeckt. Ein alltägliches Beispiel stellt Limonen dar, welches in Zitrusfrüchten vorkommt. Die (S)-Form erinnert im Geruch an Zitronen, die (R)-Form dagegen riecht nach Orangen. Allgemein werden im Beschreibungstext Literaturstellen durch in eckigen Klammem stehende arabische Bezugskennzahlen, die sich auf die hinter Abkürzugsliste und vor den Patentansprüchen befindliche Literaturliste beziehen. Sollte eine römische Ziffer hinter einer meist durch Nennung des Erstautors zitierten Literaturstelle stehen, so ist der entsprechende Wert (in arabischen Zahlen) gemeint, ebenso wie in Fällen, in denen keine eckige Klammer um den Wert ist.

Die Herstellung enantiomerenreiner Substanzen kann nach drei verschiedenen Methoden erfolgen:
- Klassische Racematspaltung
- Verwendung chiraler Bausteine der Natur ("chiral pool")
- Asymmetrische Synthese

Vor allem die asymmetrische Synthese ist inzwischen von großer Bedeutung. Zu ihr zählen enzymatische, stöchiometrische und auch katalytische Methoden. Die asymmetrische Katalyse ist die weitaus effektivste Methode, da mit einem Minimum an chiralem Katalysator ein Maximum an optisch aktiven Substanzen hergestellt werden kann.
Durch die Entdeckungen von Pasteur[2], LeBel[3] und van't Hoff[4] stieg das Interesse an optisch aktiven Substanzen, weil man deren Bedeutung für die komplexe Chemie des Lebens erkannt hatte.

D. Enders und W. Hoffmann[1] definieren die asymmetrische Synthese wie folgt:
"Eine asymmetrische Synthese ist eine Reaktion, bei der aus einer prochiralen eine chirale Gruppierung so erzeugt wird, daß die stereoisomeren Produkte (Enantiomere oder Diastereomere) in ungleichen Mengen entstehen."

Um ein Gelingen einer asymmetrischen Synthese zu erreichen, müssen diastereomorphe Übergangszustände mit unterschiedlichen Energien während der Reaktion durchlaufen werden. Diese bestimmen, welches Enantiomer im Überschuß gebildet wird. Diastereomorphe Übergangszustände mit unterschiedlichen Energien können durch zusätzliche Chiralitätsinformationen erzeugt werden. Diese können wiederum durch chirale Solventien, chiral modifizierte Reagenzien oder chirale Katalysatoren zur Bildung der diastereomorphen Übergangszustände eingebracht werden.

Ein Beispiel für eine asymmetrische Katalyse stellt die Sharpless-Epoxidierung dar.[5] Hierbei wird aus der Lewis-Säure Ti(O-i-Pr)4 und (-)-Diethyltartrat der in Abbildung 2 aufgezeigte chirale Katalysator gebildet.

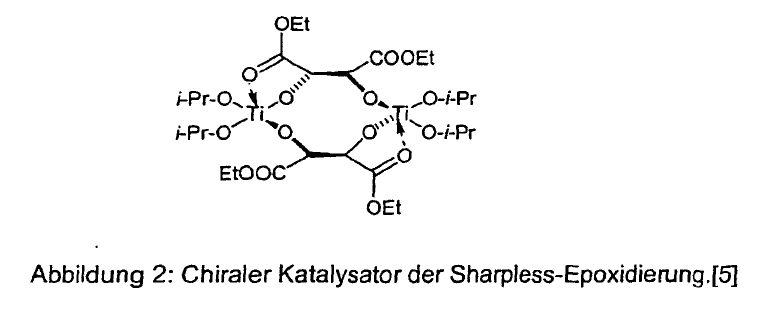

Mit Hilfe dieses Katalysators können Allylalkohole **1** mit hoher Enantionselektivität zu **2** epoxidiert werden (siehe Abbildung 3), wobei tert.-Butylhydroperoxid als Oxidationsmittel eingesetzt wird.

Allgemein werden im Beschreibungstext zur Bezeichnung und Kennzeichnung von Verbindungen, insbesondere solchen Verbindungen, die in einer Abbbildung gezeigt sind oder als allgemeine Formel bezeichnet sind, überwiegend, aber nicht immer, korrespondierende fette und unterstrichene Zahlen verwendet.

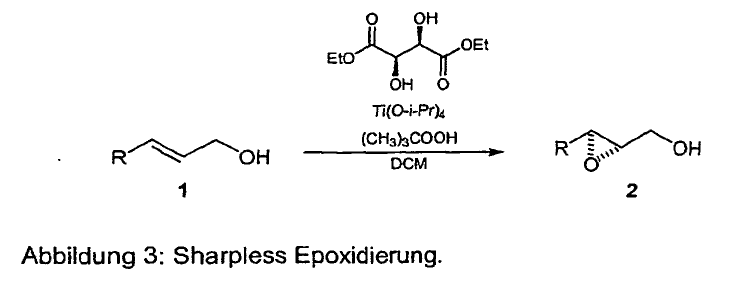

Die Sharpless-Reaktion ist inzwischen eine weit verbreitete Reaktion, besonders in der Naturstoffchemie. Durch nucleophile Ringöffnung lassen sich leicht Verbindungen wie Alkohole, Ether oder vicinale Alkohole mit einer optischen Reinheit von > 90% darstellen.

### Die Michael-Reaktion

Die Michael-Reaktion hat eine sehr große Bedeutung in der organischen Synthese und ist eine der wichtigsten C-C-Verknüpfungsreaktionen. Sie besitzt ein enormes Synthesepotential.

Da eine Vielzahl verschiedener Michael-Additionen existieren, werden in den nächsten Kapiteln einige Beispiele aufgeführt. Dabei wird ein Schwerpunkt auf die Michael-Additionen mit Thiolen durch asymmetrische Katalyse gelegt.

### Die klassische Michael-Addition

Die klassische Michael-Reaktion[6], wie in Abbildung 4 dargestellt, wird in protischen Lösungsmitteln durchgeführt. Dabei wird zuerst eine Carbonylverbindung **3** in α-Stellung mit einer Base unter Bildung des Enolates **4** deprotoniert.

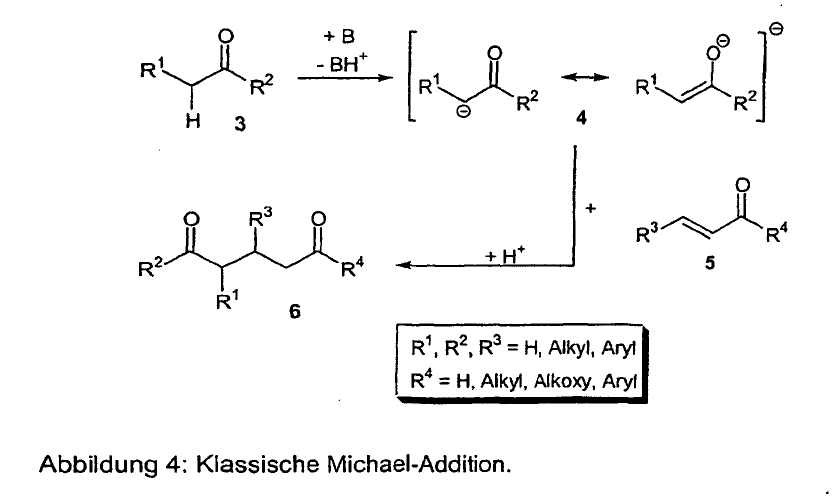

Dieses Enolat-Anion **4** (Michael-Donator) greift in Form einer 1,4-Addition an eine α,β-ungesättigte Carbonyl-verbindung **5** (Michael-Akzeptor) an. Nach Reprotonierung entsteht dann das Michael-Addukt **6**, ein 1,5-Diketon.
Die wichtigste Nebenreaktion, die hierbei auftreten kann, ist die Aldol-Reakfion.[5] Das gebildete Enolat-Anion greift dann nicht in β-Stellung sondem direkt am Carbonylsauerstoff des Michael-Akzeptors in Form einer 1,2-Addition an. Die Aldol-Reaktion ist dabei der kinetisch favorisierte Prozeß, aber diese 1,2-Addition ist reversibel. Da die Michael-Addition irreversibel ist, erhält man bei höheren Temperaturen das thermodynamisch stabilere 1,4-Addukt.

### Allgemeine Michael-Addition

Inzwischen gibt es viele verwandte 1,4-Additionen, in denen der Michael-Akzeptor und/oder -Donator von denen der klassischen Michael-Addition abweichen. Sie werden häufig 'Michael-artig' genannt oder unter dem Oberbegriff 'Michael-Addition' gefaßt Als allgemeine Michael-Addition werden heute alle 1,4-Additionen eines Nucleophils (Michael-Donator) an eine durch elektronenziehende Gruppen aktivierte C-C-Mehrfachbindung (Michael-Akzeptor) bezeichnet. Hierbei wird das Nucleophil an die aktivierte C-C-Mehrfachbindung **7** unter Bildung des Adduktes **8** 1,4-addiert (siehe Abbildung 5). [7]

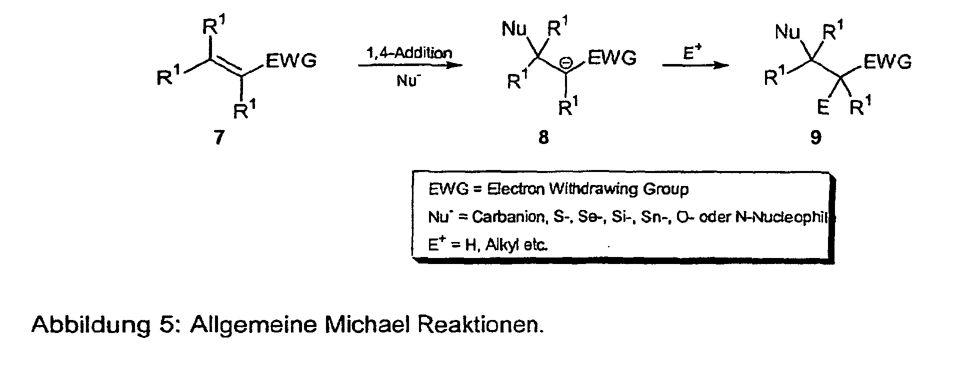

Beim Arbeiten in aprotischen Lösungsmitteln kann das intermediär entstehende Carbanion **8** mit Elektrophilen unter Bildung von **9** (E=H) umgestetzt werden. Wenn es sich beim Elektrophil um ein Proton handelt, spricht man von einer "normalen" Michael-Addition. Handelt es sich hingegen um Kohlenstoff-Elektrophile, spricht man von einer "Michael-Tandem-Reaktion", da nach der 1,4-Addition als zweiter Schritt die Addition des Elektrophils erfolgt.[8]

Es können neben den α,β-ungesättigten Carbonylverbindungen auch vinyloge Sulfone[9], Sulfoxide[10], Phosphonate[11] und Nitroolefine[12] als Michael-Akzeptor eingesetzt werden. Als Nuclephile dienen nicht nur Enolate sondern auch andere Carbanionen sowie andere Hetero-Nucleophile wie Stickstoff[13], Sauerstoff[14], Silicium[15], Zinn[16], Selen[17] und Schwefel[18].

### Intramolekulare Steuerung der Michael-Additionen

Eine Möglichkeit, eine asymmetrische Induktion in die Michael-Addition von Thiolen an Michael-Akzeptoren einzuführen, ist die intramolekulare Steuerung. Hierbei befindet sich schon vor der Reaktion entweder im Michael-Akzeptor oder im Thiol ein stereogenes Zentrum, welches die Stereochemie der Michael-Reaktion steuert.

Mit der Hilfe von enantiopuren N-Acrylsäure-Pyrrolidinonen haben ähnlich wie Evans mit Oxazolidinonen K. Tomioka et al.[19] wie aus Abbildung 6 ersichtlich eine induzierte Michael-Addition mit Thiolen an 2-Alkylacrylsäuren durchgeführt:

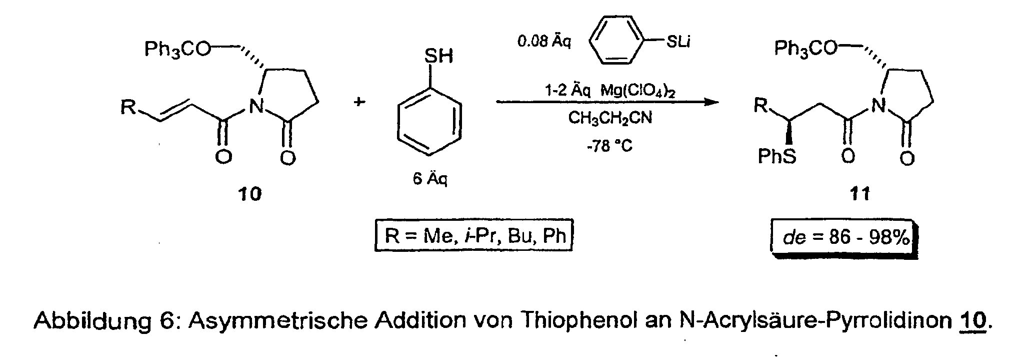

Die Reaktion wurde durch die (E/Z)-Geometrie der Acryl-Pyrrolidinone vorgesteuert. Die asymmetrische Induktion erfolgt durch die (R)-Triphenylmethoxymethyl-Gruppe in 5-Position des Pyrrolidinons. Dieser raumfüllende 'Henkel' deckt während der Reaktion die Re-Halbseite der Doppelbindung ab, so daß nur ein Angriff von der entgegengesetzten Si-Seite möglich ist. Bei einzelner Zugabe von 0.08 Äquivalenten Thiolat oder Mg(ClO₄)₂ konnte ein de-Wert von bis zu 70% erreicht werden. Durch die kombinierte Zugabe stieg der de-Wert sogar bis 98%. Dabei versteht man unter dem de-Wert den Anteil des reinen Enantiomers am Produkt, wobei der verbleibende Rest, der an 100% fehlt, das racemische Gemisch ist. Der ee-Wert ist identisch definiert.

Für den Aufbau eines neuen stereogenen Zentrums gibt es viele weitere Beispiele, jedoch Michael-Additionen von Thiolen mit intramolekularer Steuerung, bei denen zwei stereogene Zentren in einem Schritt gebildet werden, sind selten.

T. Naito et al [20] haben für eine Michael-Addition, bei der zwei neue Zentren gebildet wurden, die Oxazolidinone von Evans[21] zur Einführung der Chiralitätsinformation in den Michael-Akzeptor benutzt (Abbildung 7):

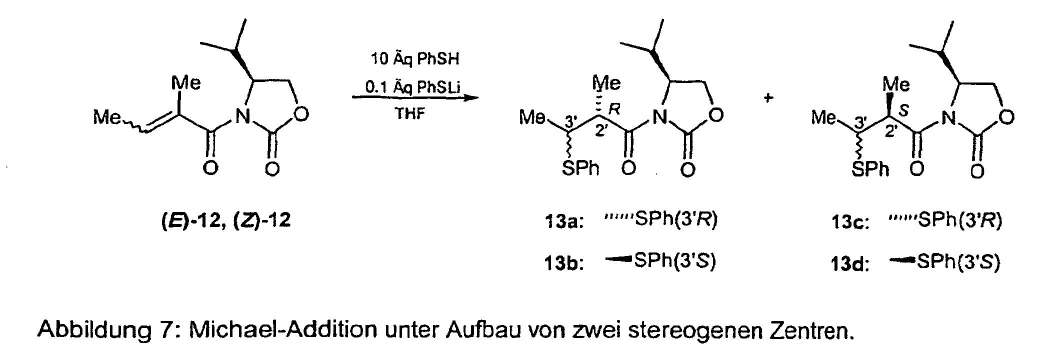

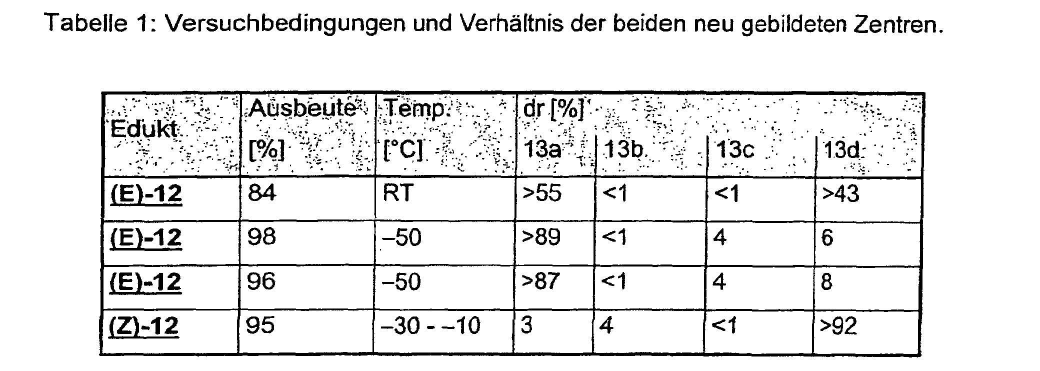

Um hohe Diastereomeren- (80 - 86%) und Enantionmeren-Überschüsse (98%) zu erzielen, wurde eine Lösung von 10 Äquivalenten Thiophenol und 0.1 Äquivalenten Lithiumthiophenolat in THF bei niedrigen Temperaturen (-50 - -10 °C) zu 1 Äquivalent des chiralen Imids **12** hinzugegeben. Als die Methylgruppe von **12** in 3'-Position gegen eine Phenylgruppe ausgetauscht wurde, wurden in der gleichen Reaktion immer noch Diastereomerenüberschüsse von >80% erreicht. Die Enantiomerenüberschüsse lagen jedoch nur noch zwischen 0 und 50%. Das Stereozentrum in 2'-Position konnte auch in diesem Fall selektiv gesteuert werden, dagegen beim Zentrum in 3'-Position waren nur noch geringe Selektivitäten zu erzielen.

### Michael-Addition katalysiert durch chirale Basen

Die Michael-Addition von Thiolen an α,β-ungesättigte Carbonylverbindungen unter Basenkatalyse wie Triethylamin oder Piperidin ist schon länger bekannt.[22] Beim Einsatz achiraler Edukte werden jedoch enantiopure Basen benötigt, um optisch aktive Substanzen zu gewinnen.

T. Mukaiyama et al.[23] untersuchte den Einsatz von Hydroxyprolin-Derivaten **14** als chiralen Katalysator:

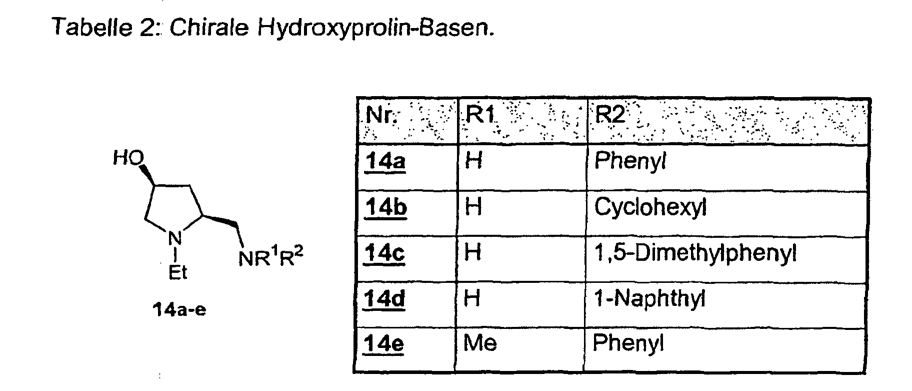

Es wurde die Addition von Thiophenol (0.8 Äquivalente) und Cyclohexanon (1 Äquivalent) mit den Hydroxyprolin-Derivaten **14a-e** (0.008 Äquivalente) in Toluol untersucht. Dabei stellte sich heraus, daß unter Verwendung von **14d** ein ee-Wert von 72% erreicht werden konnte.

Zur chiralen Basenkatalyse wurden ebenfalls viele Alkaloide getestet. Besonders oft und ausführlich wurden Cinchona-Alkaloide[24],[25] und Ephedrin-Alkaloide verwendet.

So hat H. Wynberg[26] sehr ausführlich die Michael-Addition von Thiophenolen an α,β-ungesättigte Cylohexanone mit Cinchona- und Ephedrin-Alkaloiden (siehe Abbildung 8) zur Katalyse und Steuerung getestet:

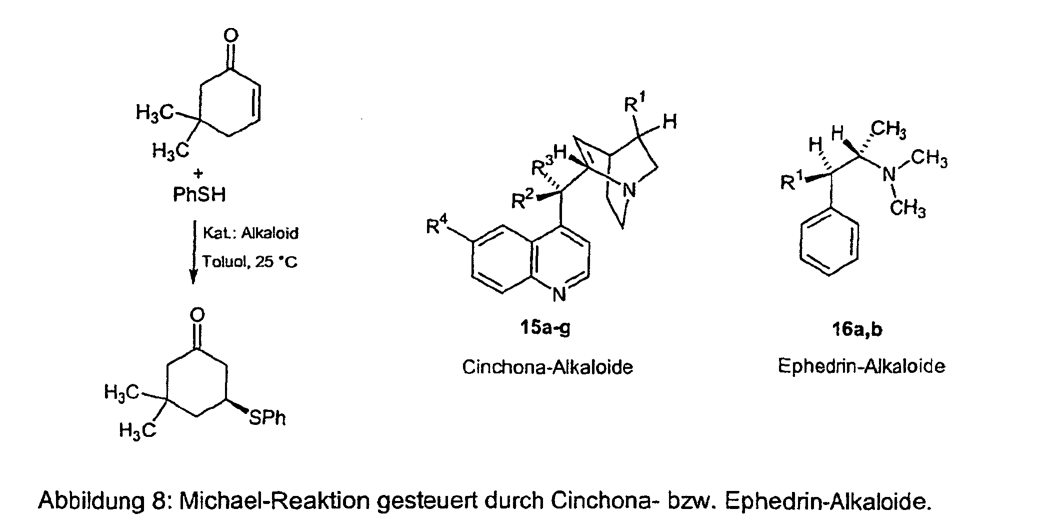

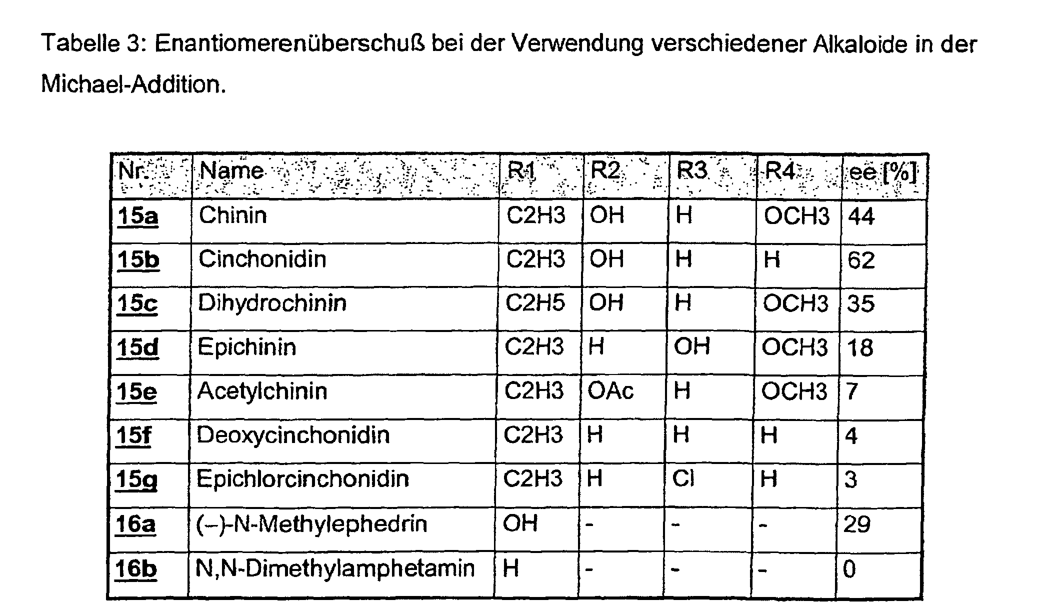

Wie aus Tabelle 3 ersichtlich bewirkte schon eine geringe Änderung der Reste R1 - R4 im Alkaloid **15, 16** eine deutliche Änderung des Enantiomerenüberschusses. Das heißt, daß der Katalysator den Edukten angepaßt werden muß. Wenn z.B. anstatt Thiophenol p-Methylthiophenol verwendet wurde, war eine deutliche Verschlechterung des Enantiomerenüberschusses bei gleichem Katalysator zu erkennen.

### Michael-Addition unter chiraler Lewis-Säure-Katalyse

Einfache Katalysen der Michael-Addition von Thiolen an Michael-Akzeptoren durch einfache Lewis-Säuren wie z.B. TiCl4 sind mit z.T. guten Ausbeuten schon länger bekannt.[27]

Für die Katalyse durch chirale Lewis-Säuren gibt es einige Beispiele, bei denen, wie auch bei der intramolekularen Steuerung (Kapitel 1.2.3), N-Acrylsäure-Oxazolidinone verwendet worden sind. Diese enthalten diesmal jedoch kein chirales Zentrum. Die weitere Carbonylgruppe des eingeführten Oxazolidinon-Rings wird zur Chelatisierung des Metallatoms der chiralen Lewis-Säure benötigt → **17.** Die Lewis-Säure **18** wurde von D.A. Evans zur Addition von Silylenolethern an den N-Acrylsäure-Oxazolidinon **17** + Lewis-Säure-Komplex **18** mit Diastereomeren-überschüssen von 80 - 98% und Enantiomerenüberschüssen von 75 - 99% verwendet (siehe Abbildung 9). [28]

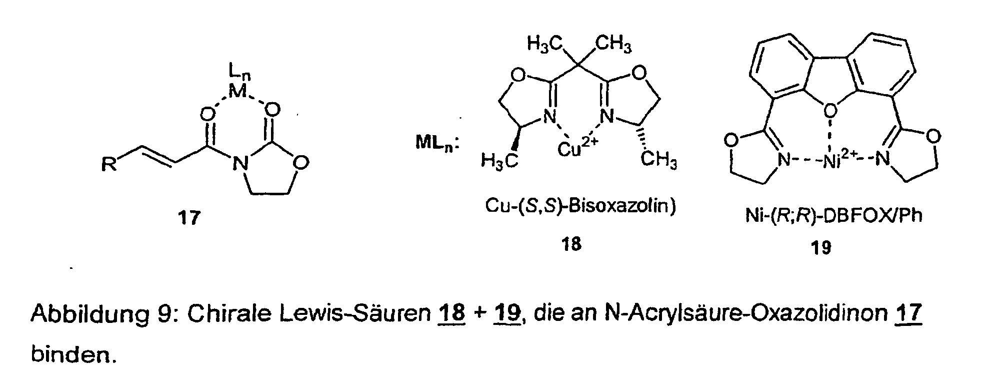

Die Lewis-Säure Ni-(R,R)-DBFOX/Ph (DBFOX/Ph = 4,6-Dibenzofurandiyl-2,2'-bis-(4-phenyloxazolin)) **19** wurde von S. Kanemasa zur Addition von Thiolen an **17** verwendet.[29] Er erreichte Enantiomerenüberschüsse von bis zu 97% mit guten Ausbeuten.

Des öfteren wurden auch 1,1-Binaphthole (Binol) an Metall-lonen gebunden, um chirale Lewis-Säuren zu bilden (siehe Abbildung 10). So hat B. L. Feringa[30] einen LiAlBinol-Komplex 20 synthetisiert, welchen er in einer Michael-Addition von α-Nitroestern an α,β-ungesättigte Ketone einsetzte. Bei -20 °C in THF erhielt er bei Einsatz von 10 mol-% LiAlBinol 20 Michael-Addukte mit bis zu 71% ee.

Shibasaki[31] benutzt den NaSmBinol-Komplex 21 bei der Michael-Addition von Thiolen an α,β-ungesättigte acyclische Ketone. Bei -40 °C erhielt er die Michael-Addukte mit Enantiomerenüberschüssen von 75 - 93%.

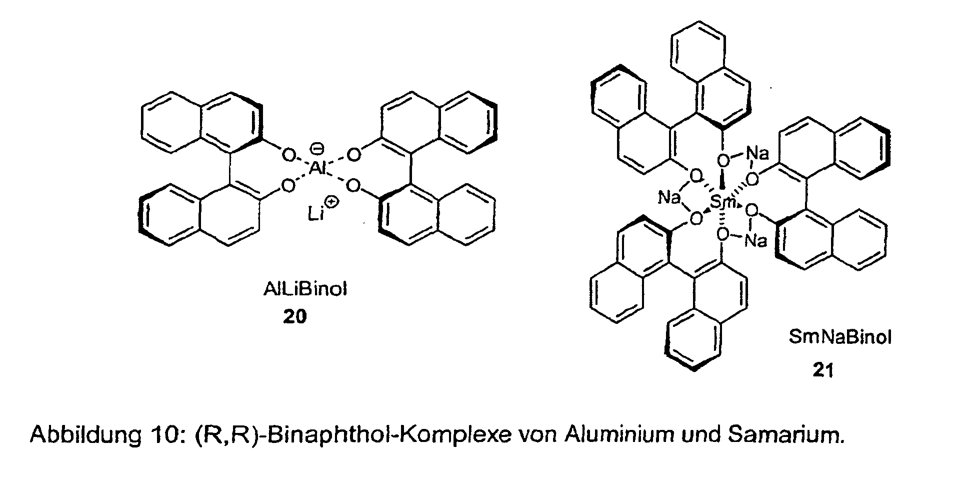

Diese chiralen Lewis-Säuren bilden bei Zugabe des Michael-Donators und -Akzeptors einen diastereomorphen Übergangszustand, wodurch dann die Steuerung der Reaktion erfolgt.

### Steuerung der Michael-Addition durch Komplexierung des lithiierten Nucleophils

Eine weitere Art den Angriff eines Nucleophils (*Michael*-Donators) in einer Reaktion zu steuern, ist die Komplexierung des lithiierten Nucleophils durch einen externen chiralen Liganden.

*Tomioka et al*.^{*[*}³²^{*]*} haben hierzu viele externe chirale Liganden für den gesteuerten Angriff von Metallorganylen in verschiedenen Reaktionen getestet, wie z.B. Aldol-Additionen, Alkylierungen von Enolaten, *Michael*-Additionen, etc.. In Abbildung 11 sind einige Beispiele von enantionmerenreinen Verbindungen aufgelistet, mit denen *Tomioka* Metallorganyle komplexierte.

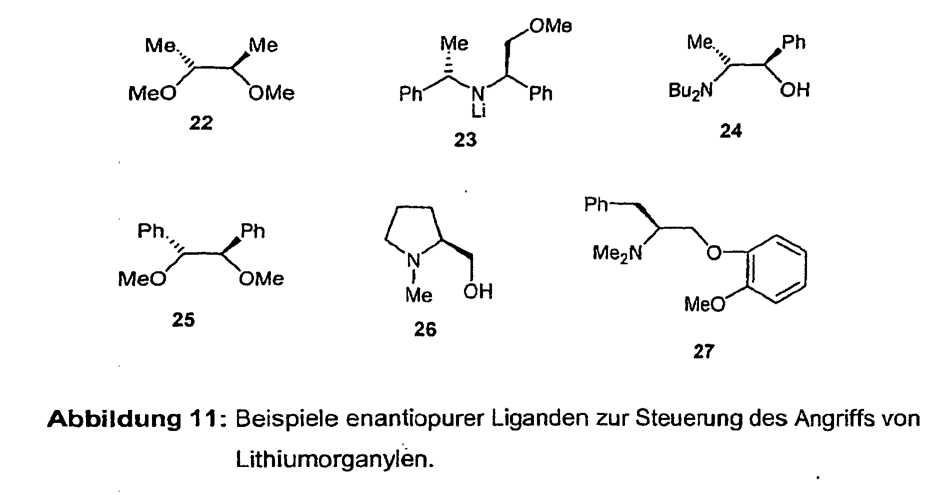

So steuerte er z.B. mit Dimethylether **22** die Aldol-Addition von Dimethylmagnesium an Benzaldehyd und erhielt einen Enantiomerenüberschuß von 22%. Dagegen erreichte er mit Lithiumamid **23** bei der Addition von BuLi an Benzaldehyd einen Enantiomerenüberschuß von 90%. Bei der Addition von Diethylzink an Benzaldehyd erreichte er mit **24** Enantiomerenüberschüsse von 90%. Mit dem Prolinderivat **26** steuerte er die Addition von Metallorganylen an *Michael*-Systeme mit Enantiomerenüberschüssen von bis zu 90%. Mit **27** konnte er bei der Alkylierung von cyclischen Enaminen nur 50% *ee* erreichen.

Später erweiterte *Tomioka* seine Synthese, indem er nicht nur Lithiumorganyle, sondern auch Lithiumthiolate verwendete.^{[33]} Dabei setzte er chirale Dimethylether wie z.B. **25,** Spartein oder chirale Diether ein. Dieser ist mit **27** verwandt und besitzt durch einen Phenylsubstituenten in 2-Position ein weiteres Chiralitätszentrum. In einer Michael-Addition von Lithiumthiolaten an Methylacrylate konnten Enatiomerenüberschüsse von 90% für diesen chiralen Diether, für **25** jedoch nur 6% erreicht werden.

Wenn man bedenkt, daß in allen Fällen die chiralen Verbindungen nur in katalytischen Mengen von 5 - 10 mol-% eingesetzt wurden, sind die Enantiomerenüberschüsse als z.T. sehr gut anzusehen.

*Tomioka* postulierte für die Dimethylether **28** in unpolaren Lösungsmitteln das Konzept des asymmetrischen Sauerstoffatoms^{[34]}:

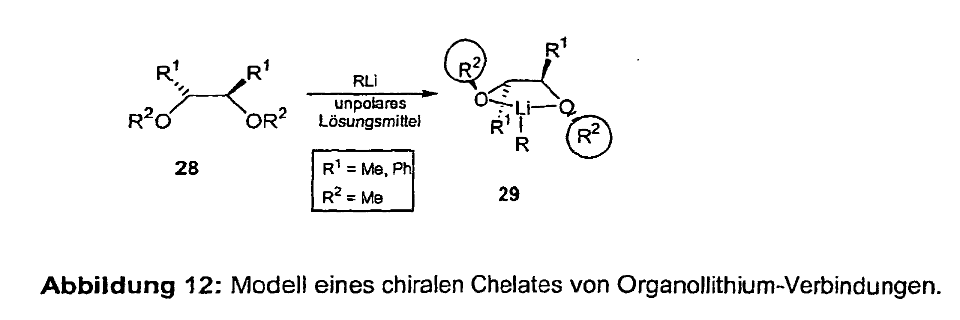

Wie in Abbildung 12 gezeigt, stehen die Reste von **28** im Komplex **29** durch sterische Effekte in *all*-*trans*-Stellung. Durch die asymmetrischen Kohlenstoffatome in der Ethylenbrücke werden die benachbarten Sauerstoffatome zu asymmetrischen Zentren. Laut Röntgenstrukturanalyse sind diese Sauerstoffatome, die das Lithium chelatisieren, in **29** tetraedrisch koordiniert. Daher ist die Chiralitätsinformation in direkter Nachbarschaft zum chelatisierten Lithium-Atom durch die raumfüllenden Reste R2 gegeben.

Aufgabe der Erfindung war allgemein, eine asymetrische Synthese unter den Bedingungen einer Micheladdition zu entwickeln, die bestimmte Nachteile des Standes der Technik vermeidet und gute Ausbeuten bringt.

Im speziellen sollte ein einfacher Syntheseweg zur Herstellung von 2-Formylamino-3-dialkylacrylsäureester **30** gefunden und die (*E,Z*)-Gemische der gebildeten Acrylsäureester **30** voneinander getrennt werden. Ebenso sollte ausgehend von dem synthetisierten Michael-Akzeptor **30** ein Weg zur Michael-Addition mit Thiolen gefunden werden. Für diese Addition sollte zuerst ein Lewis-Säure-Katalysator gefunden werden, der später mit chiralen Liganden zur Steuerung versehen werden kann (siehe Abbildung 13). Dabei wurden die Diastereomeren- und Enantiomerenüberschüsse von den Michael-Addukten **31** direkt bestimmt.

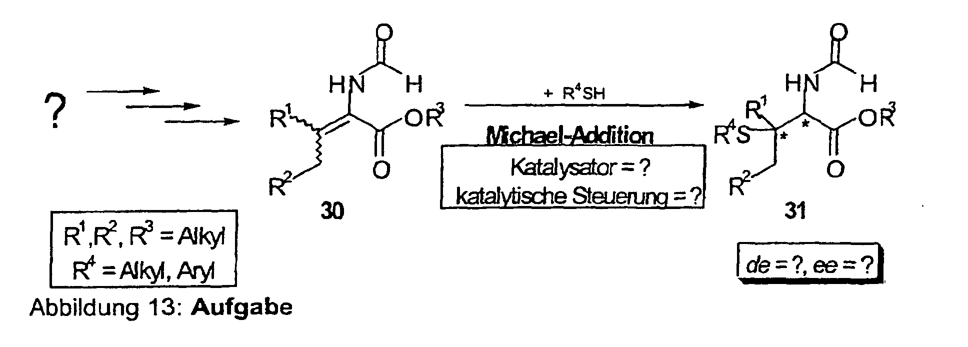

Daher ist genereller Gegenstand der Erfindung ein Verfahren zur Herstellung einer Verbindung der allgemeinen Formel **9** 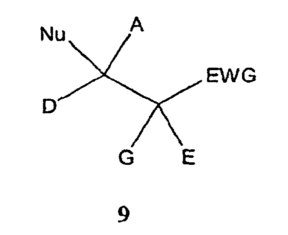 wobei eine Verbindung der allgemeinen Formel **7** unter den geeigneten Bedingungen einer 1,4-Michael-Addition mit einem Nucleophil Nu⁻ nach nachfolgendem Schema umgesetzt wird 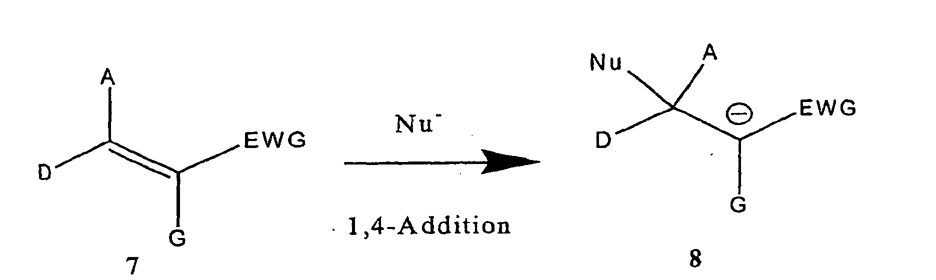 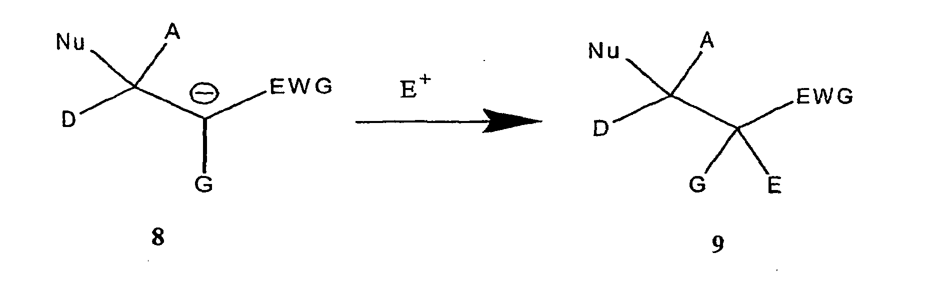 , worin die Reste
A, D und G unabhängig voneinander gleich oder verschieden sind und einen beliebigen Substituenten darstellen,
E ausgewählt ist aus H oder Alkyl,
Nu ausgewählt ist aus einem C-, S-, Se-, Si-, Si-, O- oder N-Nucleophil,
und EWG für eine elektronenziehende Gruppe steht,
dadurch gekennzeichnet, daß die Bedingungen so gewählt sind, daß die stereoisomeren, insbesondere enantiomeren und/oder diastereomeren, Produkte in ungleichen Mengen entstehen. Dabei ist es besonders bevorzugt, wenn das Nucleophil Nu- ein S-Nucleophil ist.

Ein weiterer spezieller Gegenstand der Erfindung ist ein Verfahren zur Herstellung einer Verbindung der allgemeinen Formel **31** 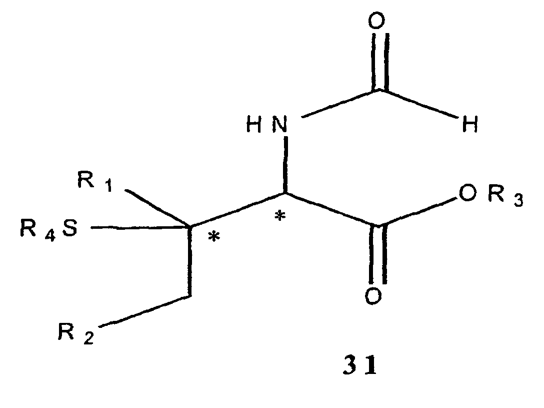 , worin
R1, R2 und R3 unabhängig voneinander ausgewählt sind aus
C₁₋₁₀-Alkyl, gesättigt oder ungesättigt, verzweigt oder unverzweigt, einfach oder mehrfach substituiert oder unsubstituiert; und
* ein stereoselektives Zentrum markiert,
R4 ausgewählt ist aus:
C1-10-Alkyl, gesättigt oder ungesättigt, verzweigt oder unverzweigt, einfach oder mehrfach substituiert oder unsubstituiert; C3-8-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C1-3-Alkyl gebundenem Aryl, C3-8-Cycloalkyl oder Heteroaryl, jeweils unsubstituiert oder einfach oder mehrfach substituiert;
bei dem eine Verbindung der allgemeinen Formel **30**, unter Bedingungen einer Michael-Addition mit einer Verbindung der allgemeinen Formel R₄SH, nach nachfolgender Reaktion I umgesetzt wird: 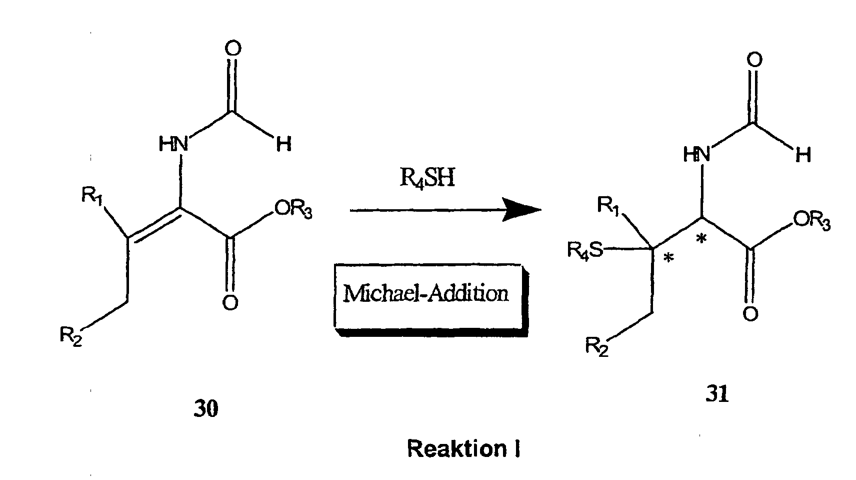 , wobei die Verbindungen der Formel R₄SH als Lithiumthiolate eingesetzt oder in oder vor der Reaktion I zu Lithiumthiolaten umgesetzt werden und/oder chirale Katalysatoren, ausgewählt aus: chiralen Hilfsreagenzien, insbesondere dem Diether (*S,S*)-1,2-Dimethoxy-1,2-diphenylethan; Lewis-Säuren und/oder Brønsted-Basen oder Kombinationen von diesen, eingesetzt werden und gegebenenfalls mit Basen, insbesondere NaOH, anschließend hydrolysiert und gegebenenfalls, vorzugsweise durch Säulenchromatographie, gereinigt wird.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht C₁₋₂-Alkyl für C1- oder C2-Alkyl, C₁₋₃-Alkyl für C1-, C2- oder C3-Alkyl, C₁₋₄-Alkyl für C1-, C2-, C3- oder C4-Alkyl, C₁₋₅-Alkyl für C1-, C2-, C3-, C4- oder C5-Alkyl, C₁₋₆-Alkyl für C1-, C2-, C3-, C4-, C5- oder C6-Alkyl, C₁₋₇-Alkyl für C1-, C2-, C3-, C4-, C5-, C6- oder C7-Alkyl, C₁₋₈-Alkyl für C1-, C2-, C3-, C4-, C5-, C6-, C7- oder C8-Alkyl, C₁₋₁₀-Alkyl für C1-, C2-, C3-, C4-, C5-, C6-, C7-, C8,- C9- oder C10-Alkyl und C₁₋₁₈-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht C₃₋₄-Cycloalkyl für C3- oder C4-Cycloalkyl, C₃₋₅-Cycloalkyl für C3-, C4- oder C5-Cycloalkyl, C₃-₆-Cycloalkyl für C3-, C4-, C5- oder C6-Cycloalkyl, C₃₋₇-Cycloalkyl für C3-, C4-, C5-, C6- oder C7-Cycloalkyl, C₃₋₈-Cycloalkyl für C3-, C4-, C5-, C6-, C7- oder C8-Cycfoalkyl, C₄₋₅-Cycloalkyl für C4- oder C5-Cycloalkyl, C₄₋₆-Cycloalkyl für C4-, C5- oder C6-Cycloalkyl, C₄₋₇-Cycloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CHF₂, CF₃ oder CH₂OH sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines Wasserstoffrestes durch F, Cl, Br, I, NH₂, SH oder OH, wobei unter "mehrfach substituiert" Resten zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂. Besonders bevorzugte Substituenten sind hier F, Cl und OH. In Bezug auf Cycloalkyl kann der Wasserstoffrest auch durch OC₁₋₃-Alkyl oder C₁₋₃-Alkyl (jeweils ein- oder mehrfach substituiert oder unsubstituiert), insbesondere Methyl, Ethyl, n-Propyl, i-Propyl, CF₃, Methoxy oder Ethoxy, ersetzt sein.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH_{z}-CH₂- zu verstehen, unter (CH₂)₁₋₄ ist -CH₂-, -CH₂-CH₂-, -CH₂-CH₂-CH₂- und -CH₂-CH₂-CH₂-CH₂- zu verstehen, unter (CH₂)₄₋₅ ist -CH₂-CH₂-CH₂-CH₂- und -CH₂-CH₂-CH₂-CH₂-CH₂- zu verstehen, etc.

Unter einem Aryl-Rest werden Ringsysteme mit mindestens einem armomatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo-1,2,5 thiadiazol, Benzothiazol, Indol, Benzotriazol, Benzodioxolan, Benzodioxan, Carbazol, Indol und Chinazolin aufgeführt.

Dabei versteht man im Zusammenhang mit Aryl und Heteroaryl unter substituiert die Substitution des Aryls oder Heteroaryls mit R²³, OR²³ einem Halogen, vorzugsweise F und/oder Cl, einem CF₃, einem CN, einem NO₂, einem NR²⁴R²⁵, einem C₁₋₆-Alkyl (gesättigt), einem C₁₋₆-Alkoxy, einem C₃₋₈-Cycloalkoxy, einem C₃₋₈-Cycloalkyl oder einem C₂₋₆-Alkylen.

Dabei steht der Rest R²³ für H, einen C₁₋₁₀-Alkyl-, vorzugsweise einen C₁₋₆-Alkyl-, einen Aryl- oder Heteroaryl- oder für einen über eine C₁₋₃-Alkylen-Gruppe gebundenen Aryl- oder Heteroaryl-Rest, wobei diese Aryl und Heteroarylreste nicht selbst mit Aryloder Heteroaryl-Resten substituiert sein dürfen,
die Reste R²⁴ und R²⁵, gleich oder verschieden, für H, einen C₁₋₁₀-Alkyl-, vorzugsweise einen C₁₋₆-Alkyl-, einen Aryl-, einen Heteroaryl- oder einen über eine C₁₋₃-Alkylen-Gruppe gebundenen Aryl- oder Heteroaryl-Rest bedeuten, wobei diese Aryl und Heteroarylreste nicht selbst mit Aryl- oder Heteroaryl-Resten substituiert sein dürfen,
oder die Reste R²⁴ und R²⁵ bedeuten zusammen CH₂CH₂OCH₂CH₂, CH₂CH₂NR²⁶CH₂CH₂ oder (CH₂)₃₋₆, und
der Rest R²⁶ für H, einen C₁₋₁₀-Alkyl-, vorzugsweise einen C₁₋₆-Alkyl-, einen Aryl-, oder Heteroaryl- Rest oder für einen über eine C₁₋₃-Alkylen-Gruppe gebundenen Aryloder Heteroaryl-Rest, wobei diese Aryl und Heteroarylreste nicht selbst mit Aryl- oder Heteroaryl-Resten substituiert sein dürfen.

In einer bevorzugten Ausführungsform des erfindungsgemäßen Verfahrens gilt, daß die Verbindungen der Formel R₄SH als Lithiumthiolate eingesetzt oder in oder vor der Reaktion I zu Lithiumthiolaten umgesetzt werden.

in einer bevorzugten Ausführungsform des erfindungsgemäßen Verfahrens gilt, daß vor der Reaktion I zur Umsetzung der Verbindungen der Formel R₄SH zu Lithiumthiolaten Butyl-Lithium (BuLi) eingesetzt, vorzugsweise in einem Äquivalenten-Verhältnis BuLi : R4SH zwischen 1 : 5 und 1:20, insbesondere 1 : 10, und mit R₄SH umgesetzt wird und/oder die Umsetzung bei Temperaturen ≤ 0°C und/oder in organischem Lösungsmittel, insbesondere Toluol, Ether, THF oder DCM, besonders THF; stattfindet.

In einer bevorzugten Ausführungsform des erfindungsgemäßen Verfahrens gilt, daß zu Beginn der Reaktion I die Reaktionstemperatur bei Temperaturen ≤ 0°C, vorzugsweise bei zwischen -70 und -80°C, insbesondere -78°C, liegt und im Verlauf der Reaktion I die Temperatur auf Raumtemperatur gebracht wird oder die Reaktionstemperatur zu Beginn der Reaktion I bei Temperaturen ≤ 0°C, vorzugsweise bei zwischen -30 und -20°C, insbesondere -25°C, liegt und im Verlauf der Reaktion I die Temperatur auf zwischen -20°C und -10°C, insbesondere -15°C, gebracht wird.

In einer bevorzugten Ausführungsform des erfindungsgemäßen Verfahrens gilt, daß die Reaktion I in einem organischen Lösungsmittel, vorzugsweise Toluol, Ether, THF oder DCM, insbesondere in THF, bzw. einem unpolaren Lösungsmittel, insbesondere in DCM oder Toluol, stattfindet.

In einer bevorzugten Ausführungsform des erfindungsgemäßen Verfahrens gilt, daß die Diastereomeren nach der Reaktion I getrennt wurden, vorzugsweise durch präparative HPLC oder Kristallisation, insbesondere unter Einsatz des Lösungsmittels Pentan/Ethanol (10:1) und Kühlung.

In einer bevorzugten Ausführungsform des erfindungsgemäßen Verfahrens gilt, daß die Enantiomerentrennung vor der Diastereomerentrennung erfolgt.

In einer bevorzugten Ausführungsform des erfindungsgemäßen Verfahrens gilt, daß R¹ C₁₋₆ Alkyl, gesättigt oder ungesättigt, verzweigt oder unverzweigt, einfach oder mehrfach substituiert oder unsubstituiert; bedeutet und R² C₂₋₉-Alkyl, gesättigt oder ungesättigt, verzweigt oder unverzweigt, einfach oder mehrfach substituiert oder unsubstituiert; bedeutet,
vorzugsweise
R¹ C₁₋₂-Alkyl, einfach oder mehrfach substituiert oder unsubstituiert, insbesondere Methyl oder Ethyl; bedeutet und R² C₂₋₉-Alkyl, vorzugsweise C₂₋₇-Alkyl, gesättigt oder ungesättigt, verzweigt oder unverzweigt, einfach oder mehrfach substituiert oder unsubstituiert, insbesondere Ethyl, Propyl, n-Propyl, i-Propyl, Butyl, n-Butyl, i-Butyl, tert.-Butyl, Pentyl, Hexyl oder Heptyl; bedeutet
insbesondere
der Rest R¹ Methyl bedeutet und R² n-Butyl.

In einer bevorzugten Ausführungsform des erfindungsgemäßen Verfahrens gilt, daß R³ ausgewählt ist aus C₁₋₃-Alkyl, gesättigt oder ungesättigt, verzweigt oder unverzweigt, einfach oder mehrfach substituiert oder unsubstituiert; vorzugsweise Methyl oder Ethyl.

In einer bevorzugten Ausführungsform des erfindungsgemäßen Verfahrens gilt, daß R⁴ ausgewählt ist aus C₁₋₆-Alkyl, gesättigt oder ungesättigt, verzweigt oder unverzweigt, einfach oder mehrfach substituiert oder unsubstituiert; Phenyl oder Thiophenyl, unsubstituiert oder einfach (vorzugsweise mit OCH₃, CH₃, OH, SH, CF₃, F, Cl, Br oder I) substituiert; oder über gesättigtes CH₃-gebundenem Phenyl, unsubstituiert oder einfach (vorzugsweise mit OCH₃, CH₃, OH, SH, CF₃, F, Cl, Br oder I) substituiert;
vorzugsweise R⁴ ausgewählt ist aus C₁₋₆-Alkyl, gesättigt, unverzweigt und unsubstituiert, insbesondere Methyl, Ethyl, Propyl, n-Propyl, i-Propyl, Butyl, n-Butyl, i-Butyl, tert.-Butyl, Pentyl oder Hexyl; Phenyl oder Thiophenyl, unsubstituiert oder einfach (vorzugsweise mit OCH₃, CH₃, OH, SH, CF₃, F, Cl, Br oder I) substituiert; oder über gesättigtes CH₃-gebundenem Phenyl, unsubstituiert oder einfach (vorzugsweise mit OCH₃, CH₃, OH, SH, CF₃, F, Cl, Br oder I) substituiert,
insbesondere R⁴ ausgewählt ist aus Methyl, Ethyl oder Benzyl, unsubstituiert oder einfach (vorzugsweise mit OCH₃, CH₃, OH, SH, CF₃, F, Cl, Br oder I) substituiert

In einer bevorzugten Ausführungsform des erfindungsgemäßen Verfahrens gilt, daß das Thiolat stöchiometrisch eingesetzt wird, TMSCI verwendet wird und/oder dann ein chiraler Protonendonator R*-H eingesetzt wird,
oder
daß die Verbindung **30** vor der Reaktion I mit einer sterisch anspruchsvollen (großen) Gruppe, vorzugsweise TBDMS modifiziert wird.

In einer bevorzugten Ausführungsform des erfindungsgemäßen Verfahrens gilt, daß die Verbindung nach der allgemeinen Formel **31** 3-Ethylsulfanyl-2-formylamino-3-methyloctansäureethylester oder 3-Benzylsulfanyl-2-formylamino-3-methyloctansäureethylester ist, die Verbindung nach der allgemeinen Formel **30** 2-Formylamino-3-methyloct-2-ensäureethylester ist und R₄SH Ethylmercaptan oder Benzylmercaptan ist.

Weiter sind auch die anderen Bedingungen und Ausführungsformen der Michael Addition, wie Sie hier im folgenden dargestellt werden ebenfalls bevorzugte Ausführungsformen der erfindungsgemäßen Verfahren.

Ein weiterer Gegenstand der Erfindung ist eine Verbindung der allgemeinen Formel **31** 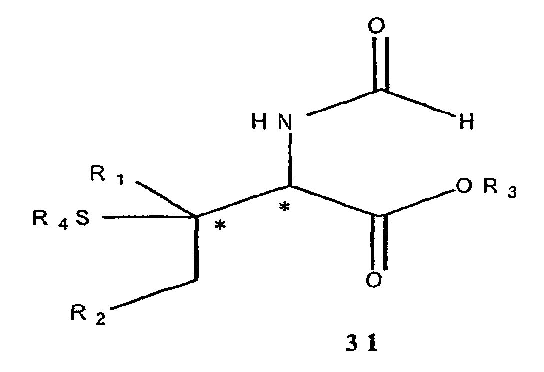 , worin
R1, R2 und R3 unabhängig voneinander ausgewählt sind aus C₁₋₁₀-Alkyl, gesättigt oder ungesättigt, verzweigt oder unverzweigt, einfach oder mehrfach substituiert oder unsubstituiert;
* ein stereoselektives Zentrum markiert, und
R⁴ ausgewählt ist aus:
   C₁₋₁₀-Alkyl, gesättigt oder ungesättigt, verzweigt oder unverzweigt, einfach oder mehrfach substituiert oder unsubstituiert; C₃₋₈-Cycloalkyl, gesättigt oder ungesättigt, unsubstituiert oder einfach oder mehrfach substituiert; Aryl-, oder
   Heteroaryl, jeweils unsubstituiert oder einfach oder mehrfach substituiert; oder über gesättigtes oder ungesättigtes C₁₋₃-Alkyl gebundenem Aryl, C₃₋₈-Cycloalkyl oder Heteroaryl, jeweils unsubstituiert oder einfach oder mehrfach substituiert;
   in Form ihrer Razemate; Enantiomere, Diastereomere, insbesondere Mischungen ihrer Enantiomere oder Diastereomere oder eines einzelnen Enantiomers oder Diastereomers; in Form ihrer physiologisch verträglichen sauren und basischen Salze bzw. Salze mit Kationen bzw. Basen oder mit Anionen bzw. Säuren oder in Form der freien Säuren oder Basen.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, a-Liponsäure, Acetylglycin, Acetylsalicylsäure, Hippursäure und/oder Asparaginsäure. Besonders bevorzugt ist das Hydrochlorid-Salz.

In einer bevorzugten Form der erfindungsgemäßen Verbindungen gilt, daß R¹ C₁₋₆-Alkyl, gesättigt oder ungesättigt, verzweigt oder unverzweigt, einfach oder mehrfach substituiert oder unsubstituiert; bedeutet und R² C₂₋₉-Alkyl, gesättigt oder ungesättigt, verzweigt oder unverzweigt, einfach oder mehrfach substituiert oder unsubstituiert; bedeutet,
vorzugsweise
R¹ C₁₋₂-Alkyl, einfach oder mehrfach substituiert oder unsubstituiert, insbesondere Methyl oder Ethyl; bedeutet und R² C₂₋₉-Alkyl, vorzugsweise C₂₋₇-Alkyl, gesättigt oder ungesättigt, verzweigt oder unverzweigt, einfach oder mehrfach substituiert oder unsubstituiert, insbesondere Ethyl, Propyl, n-Propyl, i-Propyl, Butyl, n-Butyl, 1-Butyl, tert.-Butyl, Pentyl, Hexyl oder Heptyl; bedeutet
insbesondere
der Rest R¹ Methyl bedeutet und R² n-Butyl.

In einer bevorzugten Form der erfindungsgemäßen Verbindungen gilt, daß R³ ausgewählt ist aus C₁₋₃-Alkyl, gesättigt oder ungesättigt, verzweigt oder unverzweigt, einfach oder mehrfach substituiert oder unsubstituiert; vorzugsweise Methyl oder Ethyl.

In einer bevorzugten Form der erfindungsgemäßen Verbindungen gilt, daß R⁴ ausgewählt ist aus C₁₋₆-Alkyl, gesättigt oder ungesättigt, verzweigt oder unverzweigt, einfach oder mehrfach substituiert oder unsubstituiert; Phenyl oder Thiophenyl, unsubstituiert oder einfach (vorzugsweise mit OCH₃, CH₃, OH, SH, CF₃, F, Cl, Br oder I) substituiert; oder über gesättigtes CH₃-gebundenem Phenyl, unsubstituiert oder einfach (vorzugsweise mit OCH₃, CH₃, OH, SH, CF₃, F, Cl, Br oder I) substituiert;
vorzugsweise R⁴ ausgewählt ist aus C₁₋₆-Alkyl, gesättigt, unverzweigt und unsubstituiert, insbesondere Methyl, Ethyl, Propyl, n-Propyl, i-Propyl, Butyl, n-Butyl, i-Butyl, tert.-Butyl, Pentyl oder Hexyl; Phenyl oder Thiophenyl, unsubstituiert oder einfach (vorzugsweise mit OCH₃, CH₃, OH, SH, CF₃, F, Cl, Br oder I) substituiert; oder über gesättigtes CH₃-gebundenem Phenyl, unsubstituiert oder einfach (vorzugsweise mit OCH₃, CH₃, OH, SH, CF₃, F, Cl, Br oder I) substituiert,
insbesondere R⁴ ausgewählt ist aus Methyl, Ethyl oder Benzyl, unsubstituiert oder einfach (vorzugsweise mit OCH₃, CH₃, OH, SH, CF₃, F, Cl, Br oder I) substituiert.

In einer bevorzugten Form der erfindungsgemäßen Verbindungen gilt, daß die Verbindung ausgewählt ist aus
- 3-Ethylsulfanyl-2-formylamino-3-methyloctansäureethylester oder
- 3-Benzylsulfanyl-2-formytamino-3-methyloctansäure-ethylester.

Die erfindungsgemäßen Verbindungen sind pharmakologisch, insbesondere als Analgetika, wirksam und toxikologisch unbedenklich, so daß ein weiterer Gegenstand der Erfindung Arzneimittel enthaltend die erfindungsgemäßen Verbindungen sowie gegebenenfalls geeignete Zusatz- und/oder Hilfsstoffe und/oder gegebenenfalls weitere Wirkstoffe sind. Außerdem ist ein weiterer Gegenstand die Verwendung der erfindungsgemäßen Verbindungen zur Herstellung eines Arzneimittels zur Behandlung von Schmerz, insbesondere von neuropathischem, chronischem oder akutem Schmerz, von Epilepsie und/oder von Migräne, sowie entsprechende Behandlungsmethoden.

Die folgenden Beispiele sollen die Erfindung erläutem ohne sie zu beschränken.

### Beispiele:

### Beispiel 1:

### Syntheseroute

Das Zielmolekül **32/33** soll durch eine Michael-Addition dargestellt werden. Abbildung 14 zeigt die retrosynthetische Analyse des für diesen Ansatz benötigten Eduktes 34:

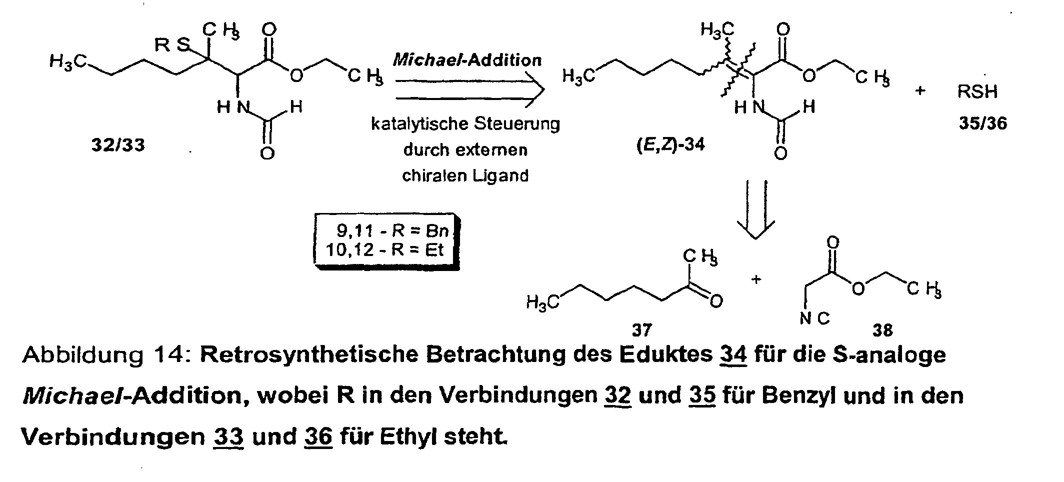

Der 2-Formylaminoacrylsäureester **34** soll in einer Olifinierungsreaktion aus dem Keton **37** und aus lsocyanessigsäureethylester **(38)** hergestellt. werden.

In Abbildung 15 ist der Syntheseweg zur Darstellung von **38** aufgezeigt:

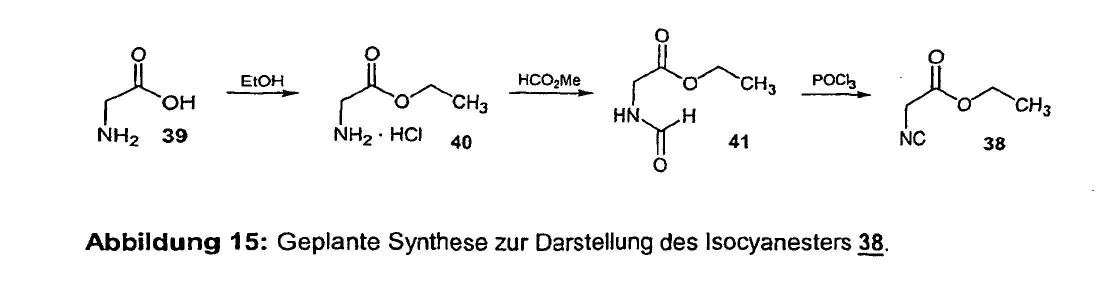

In der geplanten Synthese von **38** soll im ersten Schritt Glycin **(39)** mit Ethanol zum Glycinethylester **(40)** verestert werden. Dieser soll mit Methylformiat an der Aminofunktion unter Bildung des Formylaminoesters **41** formyliert werden. Unter Bildung des Isocyanessigsäureethylesters **(38)** soll die Formylamino-Funktion des entstandenen 2-Formylaminoessigsäureethylesters **(41)** mit Phosphorylchlorid in die Isocyan-Funktion überführt werden.

### Beispiel 2:

### Darstellung des chiralen Hilfsreagenzes: (S,S)-1,2-Dimethoxy-1,2-diphenylethan

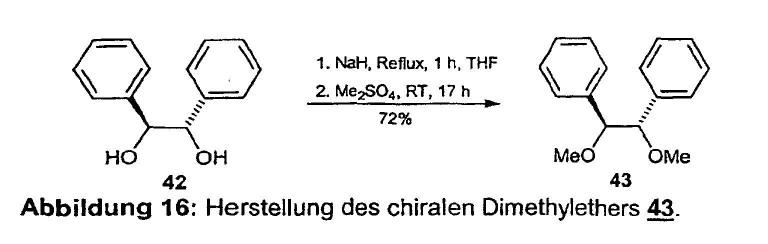

Die Darstellung des chiralen Dimethylethers **43** erfolgte nach einer Vorschrift von *K. Tomioka et al.* (siehe Abbildung 16).^{[34]} Dabei wurde gereinigtes NaH im Überschuß in THF vorgelegt, (*S,S*)-Hydrobenzoin **42** in THF bei RT zugegeben und kurz zum Rückfluß erhitzt. Die Lösung kühlte man auf 0 °C ab und tropfte Dimethylsulfat hinzu. Nach 30 min Rühren wurde die weiße, zähe Masse weitere 16 h bei RT gerührt. Nach Aufarbeitung und Umkristallisation aus Pentan erhielt man (**S,S**)-1,2-Dimethoxy-1,2-diphenylethan **(43)** in Form farbloser Nadeln und in Ausbeuten von 72%.

### Beispiel 3:

### Darstellung von Isocyanessigsäureethylester

Die Ausgangsverbindung der Synthese des Isocyanessigsäureethylesters (**38**) wurde nach dem in Abbildung 17 dargestellten Syntheseweg hergestellt:

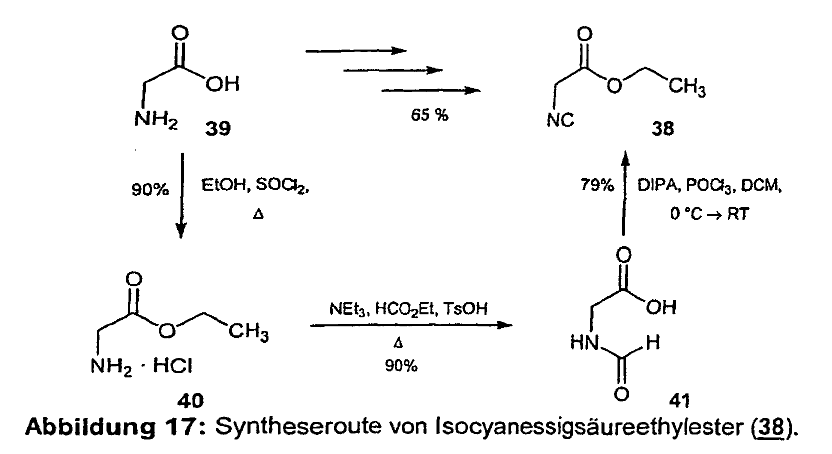

Dabei erhitze man Glycin **(39)** mit Thionylchlorid und Ethanol, welches gleichzeitig als Lösungsmittel fungierte, 2 Stunden unter Rückfluß. Nach dem Entfernen von überschüssigem Ethanol und Thionylchlorid blieb der rohe Ester als Feststoff zurück. Nach Umkristallisation aus Ethanol erhielt man den Glycinethylester als Hydrochlorid **(40)** in Ausbeuten von 90 - 97% in Form eines farblosen nadelförmigen Feststoffes.

Das Glycinethylester-Hydrochlorid **(40)** wurde nach einer leicht modifizierten Synthese von *C.-H. Wong et al*.^{[35]} an der Aminofunktion formyliert. Dabei wurde das Glycinester-Hydrochlorid **40** in Ameisensäuremethylester suspendiert und man gab Toluolsufonsäure in katalytischen Mengen hinzu. Es wurde zum Rückfluß erhitzt. Anschließend wurde Triethylamin zugetropft und die Reaktionsmischung weiter refluxiert. Nach Kühlung der Reaktionsmischung wurde das ausgefallene Ammoniumch lorid-Salze abfiltriert. Vom Filtrat wurden restlicher Ameisensäureethylester und Triethylamin abgezogen und es wurde der roher Ester als oranges Öl erhalten. Nach Destillation lag der 2-Formylaminoessigsäureethylester **(41)** in Ausbeuten von 73 - 90% als farblose Flüssigkeit vor.
Die Umwandlung der Formylamino-Gruppe zur Isocyan-Gruppe erfolgte nach einer Vorschrift von *I. Ugi* et *al.* ^{[}^{*36*}^{]} Dabei wurde der Formylaminoessigsäureethylester **(41)** in Diisopropylamin und Dichlormethan gegeben und unter Kühlung mit Phosphorylchlorid versetzt. Nach erfolgter Zugabe erwärmte man auf RT und hydrolysierte die Reaktionsmischung anschließend mit 20%iger Natriumhydrogencarbonat-Lösung. Den Isocyanessigsäureethylester **(38)** erhielt man nach Aufarbeitung und destillativer Reinigung in Ausbeuten von 73 - 79% als leicht gelbes und lichtempfindliches Öl.
Durch die Verwendung von Phosphorylchlorid konnte eine Umsetzung mit schwerhandhabbaren Phosgen umgangen werden. Dabei wurde in dieser Stufe ein Ausbeuteverlust von ca. 10% laut Literatur^{[37],[38]} in Kauf genommen.
Über drei Stufen wurde eine Gesamtausbeute von 65 % erreicht, wobei die ersten beiden Stufen ohne Probleme in großen Ansätzen bis zwei Mol durchgeführt werden konnten. Dagegen konnte die letzte Stufe wegen der großen Lösungsmittelmenge und der großen Reaktivität des Phosphorylchlorides nur in kleineren Ansätzen bis 0.5 Mol durchgeführt werden.

### Beispiel 4:

### Darstellung der (E)- und (Z)- 2-Formylamino-3-methyl-oct-2-ensäureethylester

Die Darstellung der (E)- und (Z)-2-Fomylamino-3-methyl-oct-2-ensäureethylester **(34)** erfolgte nach einer Vorschrift von *U. Schöllkopf et al*^{[39],[40]}, Dabei wurde der lsocyanessigsäureethylester **(38)** *in situ* bei niedrigen Temperaturen mit Kalium-*tert*.butanolat in α-Position deprotoniert. Anschließend wurde dann eine Lösung aus 2-Heptanon **(37)** in THF hinzugetropft. Nach 30 min Rühren erwärmte man auf Raumtemperatur. Durch Zugabe äquivalenter Mengen Eisessig wurde die Reaktion abgebrochen.
Der 2-Formylamino-3-methyl-oct-2-ensäureethylester **(34)** lag immer in (*E*/*Z*)-Gemischen vor, wobei diese leicht durch Chromatographie zu trennen waren. Die Gesamtausbeuten der gereinigten und getrennten (*E*)- und (*Z*)-Isomere beliefen sich auf 73% in Form farbloser Feststoffe.

Bei dieser Reaktion, die *Schöllkopf*^{[41]} "Formylaminomethylenierung von Carbonylverbindungen" nannte, werden in einem Zug der Sauerstoff des Ketons durch die (Formylamino-alkoxycarbonyl-methylen)-Gruppe ausgetauscht und direkt die β-substituierten α-Formylaminoacrylsäureester **34** gebildet. Der Reaktion liegt laut Schöllkopf der in Abbildung 18 aufgezeigte Mechanismus zugrunde .^{[42]}

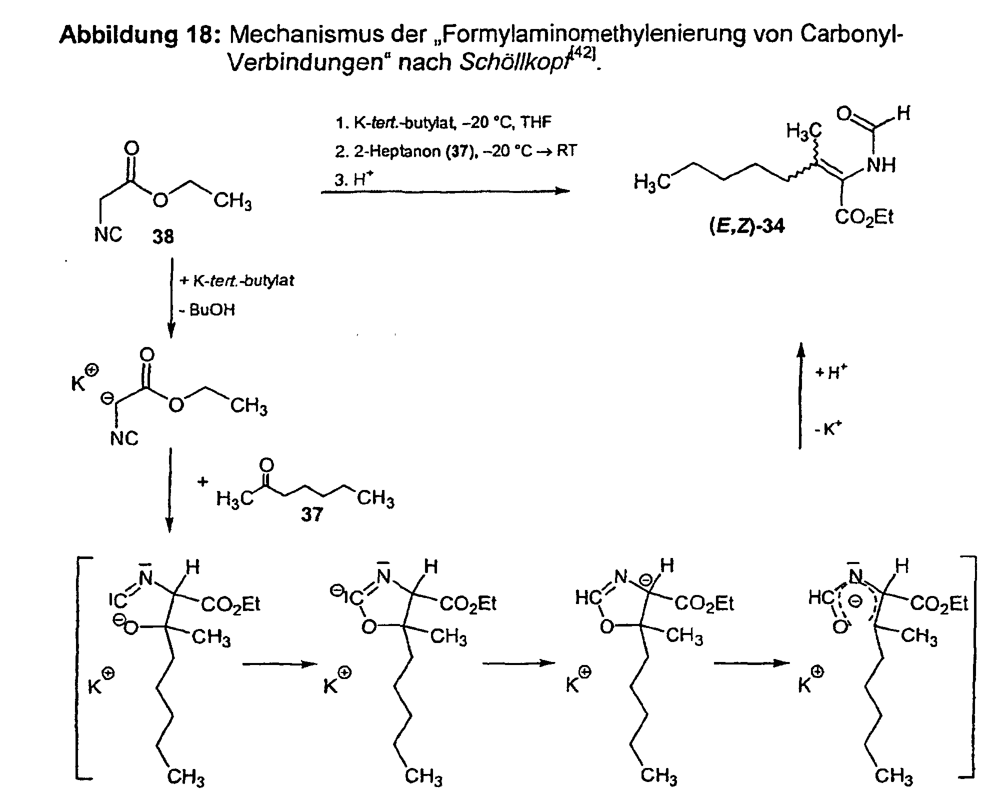

Dabei wird zuerst der lsocyanessigsäureestylester **38** mit Kalium-*tert*.-butylat in α-Position deprotoniert. Das Carbanion greift dann nucleophil am Carbonyl-C-Atom vom Keton **37** an. Nach mehreren intramolekularen Umlagerungen der negativen Ladung und anschließender Protonierung liegen die β-substituierten α-Formylaminoacrylsäureester **34** vor.
Da die 2-Formylamino-3-methyl-oct-2-ensäureethylester **(34)** immer in *(E*/*Z)* -Gemischen entstanden, stellte sich die Frage des Temperatureinflußes auf das (*E*/*Z*)-Verhältnis.

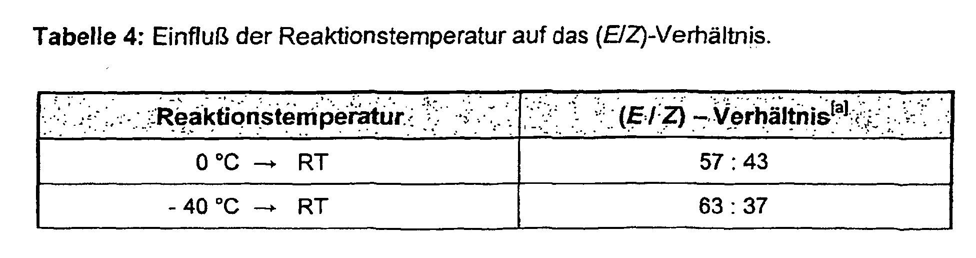

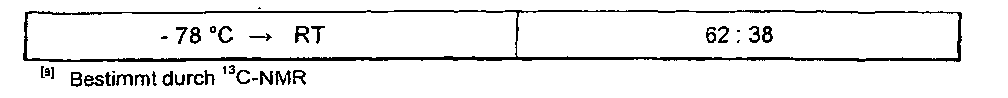

In Tabelle 4 sieht man den Einfuß der Temperatur auf die (*E*/*Z*)-Verhältnisse. Die Reaktionen wurden unter den oben beschriebenen Bedingungen durchführt. Es wurden nur die Anfangstemperaturen variiert.
Man erkennt, daß die Temperatur nur einen geringen Einfluß auf die (*E*/*Z*)-Verhältnisse hatte. Da aber für die Synthese beide Isomere benötigt werden, ist das ausgeglichene Verhältnis bei ca. 0 °C von Vorteil, da beide isomere in ungefähr gleichen Mengen durch Chromatographie gewonnen werden konnten.
Die (*E*/*Z*)-Zuordung erfolgte nach U. Schöllkopf^{[39]}, wonach die Protonen der β-ständigen Methylgruppe des (*Z*)-Isomeren bei höherem Feld absorbieren, als die des (*E*)-Isomeren^{[43]}

### Beispiel 5:

### Michael-Addition mit Thiolen als Donator

### A) Versuche mit Thiolaten als Katalysator

Da die *Michael*-Addition von Thiolen an 2-Formylamino-3-methyl-oct-2-ensäureethylester **(34)** nicht ohne Katalysator abläuft, wurde zunächst nach einer Vorschrift von *T. Naito et al.*^{[44]} vorgegangen. Dabei wurde zuerst ein Gemisch aus Thiol und

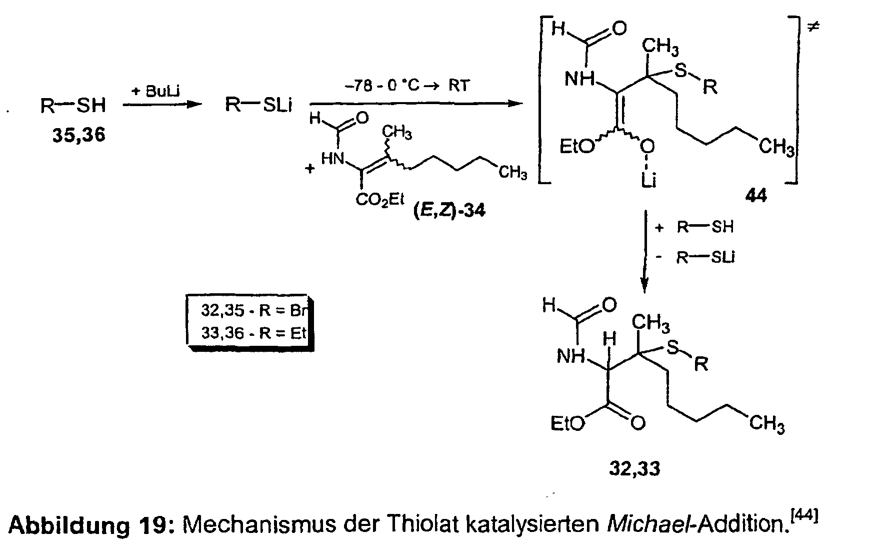

Lithiumthiolat im Verhältnis 10:1 hergestellt, bevor die Zugabe des 2-Formylaminoacrylsäureethylesters **34** erfolgte. Dabei legt man der Reaktion den in Abbildung 19 aufgezeigten Mechanismus zugrunde.^{[44]} Nach der Addition der Thiolate **35** bzw. **36** an die 2-Formylamino-3-methyl-oct-2-ensäure-ethylester **[(*E,Z*-34]** in β-Position, wird dieses Addukt **44** direkt durch das im Überschuß vorhandene Thiol protoniert, wobei das *Michael-*Addukt **32,33** entsteht.
Zur Darstellung der *Michael*-Addukte **32,33** wurden 0.1 Äquivalente BuLi in THF vorgelegt und bei 0 °C 10 Äquivalente Thiol zugegeben. Dann tropfte man bei niedrigen Temperatur die in THF gelösten (***E*)**- oder **(*Z*)-34** zu und erwärmte langsam auf RT. Nach Hydrolyse mit 5%iger NaOH und Säulenchromatographie erhielt man **32,33** als farblose, viskose Öle, welche als Diastereomeren-Gemische vorlagen.
Tabelle 5 listet die nach der beschriebenen Synthese hergestellten *Michael*-Addukte auf:

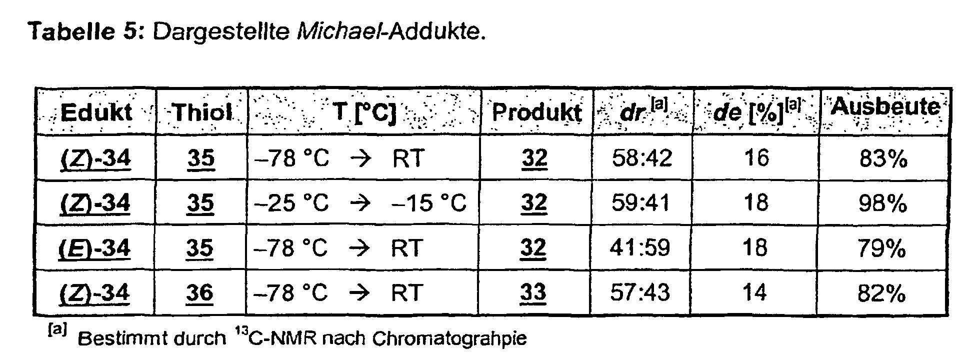

Wie aus Tabelle 5 ersichtlich gab es zwar eine Vorsteuerung durch die Wahl der Formylamino-3-methyl-oct-2-ensäureethylester **(*Z*)-34** oder **(*E*)-34**, jedoch wurde dadurch lediglich das Vorzugsdiastereoisomer bestimmt. Es war in THF nicht möglich, eine bessere Vorsteuerung mit de-Werten >18% zu erzielen, da die Reaktion in diesem Medium erst bei ≥ -20 °C startet und eine bessere Steuerung bei höheren Temperaturen nicht zu erwarten ist.
Die *threo*-*lerythro*-Diastereomeren **32** konnten zunächst durch präparative HPLC voneinander getrennt werden. Dadurch stellte man fest, daß das *threo*-Diastereomer **(*threo*)-32** ein Feststoff, das *erythro*-Diastereomer **(*erythro*)*-*32** eine viskose Flüssigkeit war.
So wurde versucht, die *threo*-*lerythro*-Diastereomeren **32** durch Kristallisation voneinander zu trennen. Die Diastereomerengemische **32** wurden in möglichst geringen Mengen Pentan/Ethanol (~10:1) gelöst und über mindestens 5 d auf -22 °C gekühlt, währenddessen das Diastereomer **(*threo*)-32** als Feststoff auskristallisierte. So erhielt man die angereicherten Diastereomeren **(*threo*)-32** bzw. **(*erythro*)-32** mit Diastereomenüberschüssen von 85 - 96 % für **(*threo*)-32** bzw. 62 - 83 % für **(*erythro*)-32.**

### B) Versuche mit Lewis-Säuren als Katalysator

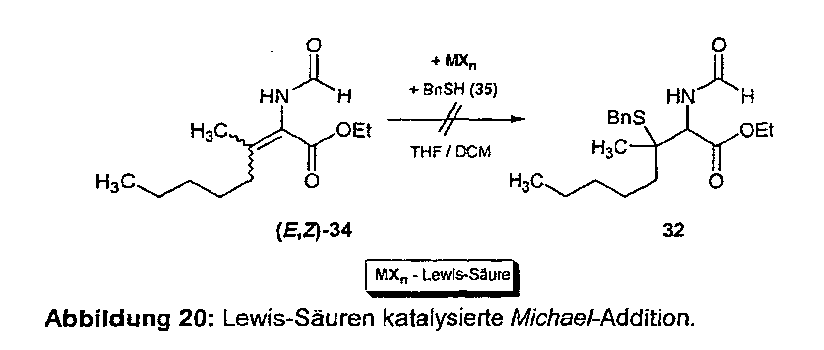

Wie aus Abbildung 20 ersichtlich, wurde versuchte die *Michael*-Addition von Benzytmercaptan an 2-Formylaminoacrylsäureethylester **34,** durch die Zugabe einer Lewis-Säure **MX**_{**n**} zu katalysieren. Es gibt viele Beispiele für die Aktivierung von α,β-ungesättigten Estern durch verschiedene Lewis-Säuren für die Addition von Thiolen. ^{[27]} In diesem Falle würde einer der postulierten Komplexe **A** oder **B** bilden, in dem das Metall am Carbonyl-Sauerstoff koordiniert (siehe Abbildung 21).

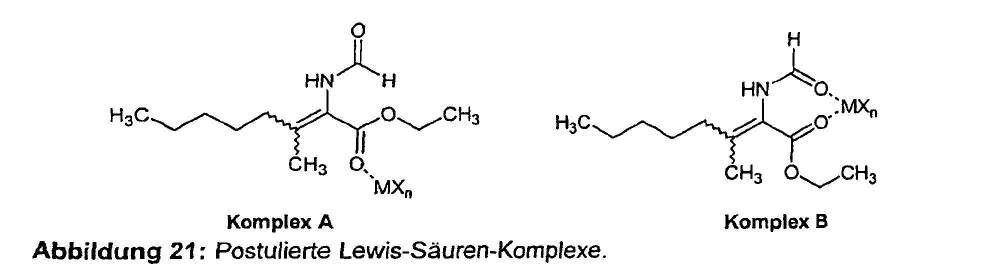

Durch diesen Komplex sollte die Doppelbindung so stark aktiviert werden, so daß die Reaktion direkt abläuft.
Es wurden die Tabelle 6 aufgelisteten Lewis-Säuren **MX**_{**n**} in unterschiedlichen Lösungsmitteln auf ihre katalytische Wirkung auf diese *Michael*-Reaktion getestet.
Dabei wurden jeweils ein Äquivalent der 2-Formylamino-3-methyl-oct-2-ensäureethylester **(34)** in THF oder DCM vorgelegt und ein Äquivalent der gelösten oder suspendierten Lewis-Säure bei 0 °C hinzugegeben. Danach wurden 1.2 Äquivalente Benzylmercaptan hinzugetropft und nach 2 h auf Raumtemperatur erwärmt. Zum Teil wurden die Ansätze auch zum Rückfluß erhitzt, wenn nach einem Tag keine Reaktion zu erkennen war.

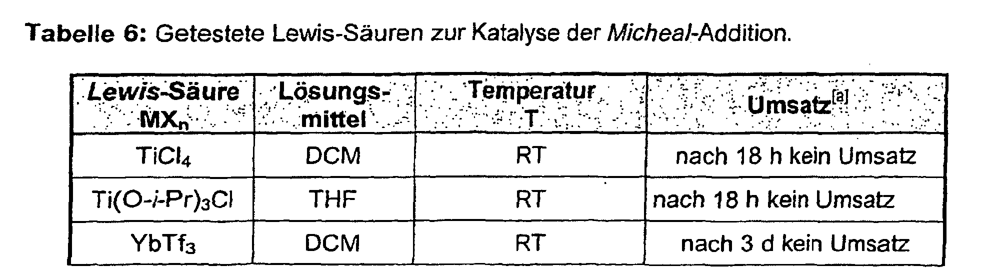

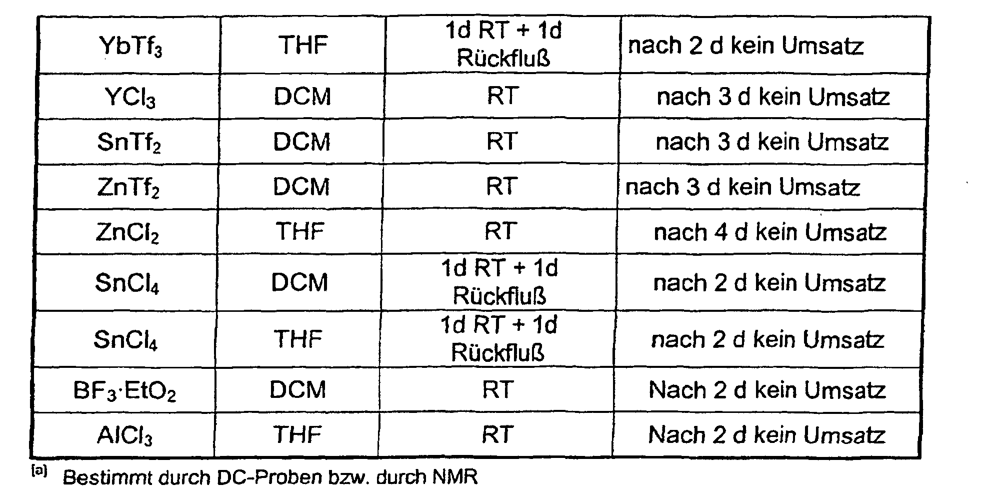

Nur bei TiCl₄ war eine Farbveränderung zu erkennen, was auf eine Komplexbildung schließen ließ. Bei allen anderen Lewis-Säuren war dagegen keine Komplexbildung durch Farbveränderung ersichtlich. Keine der ausprobierten Lewis-Säuren zeigte eine katalytische Wirkung, da in allen Fällen nach bis zu 3 Tagen Reaktionsdauer kein Umsatz zu erkennen war und die Edukte wieder vollständig zurückgewonnen werden konnten.

### C) Versuche zur Katalyse mit Lewis-Säuren unter Zusatz von Basen

Die *Michael*-Addition von Thiolen an α,β-ungesättigte Ketone kann wie in Kapitel 1.2.4 beschrieben durch Zugabe von Basen (z.B. Triethylamin) katalysiert werden.^{[45]} Dabei erhöht die Brønsted-Base die Nucleophilie des Thiols, welche nun ausreichend groß ist, um die Reaktion zu starten.
Bei der Reaktion von äquimolaren Mengen 2-Formylamino-3-methyl-oct-2-ensäureethylester **(34),** Benzylmercaptan **(35)** und Triethylamin in THF, konnte bei Reaktionstemperaturen bis 60 °C keine katalytische Wirkung beobachtet werden. Die Ausgangsstoffe konnten wieder zurückgewonnen werden.

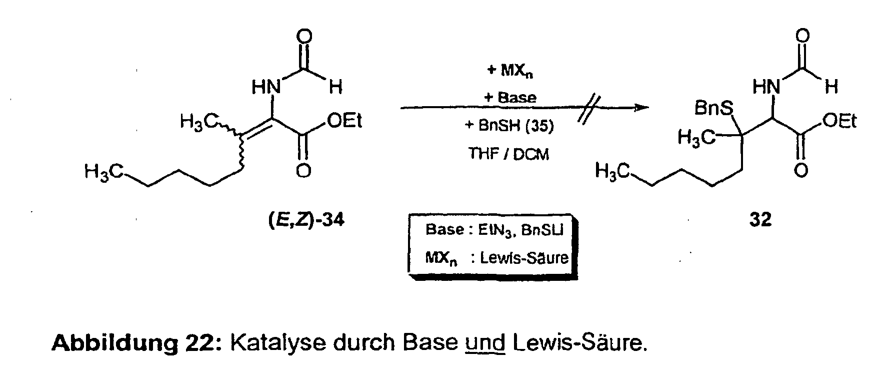

Daher kam die Idee die Lewis-Säuren-Katalyse (vorgestellt in Kapitel 2.6.2) mit der Basen-Katalyse zu verbinden (siehe Abbildung 22), weil die Katalyse mit Lewis-Säuren bzw. Brønsted-Basen je für sich alleine nicht funktioniert.
Bei den in Tabelle 7 aufgezeigten Kombinationen von Basen und Lewis-Säuren wurde jeweils ein Äquivalent 2-Formylamino-3-methyl-oct-2-ensäureethylester **(34)** im angegebenen Lösungsmittel vorgelegt und eine Lösung aus 1.2 Äquivalenten Benzylmercaptan **(35)** und 1 Äquivalent der angegebenen Base bei 0 °C zugetropft.
Nach 2 h wurde auf Raumtemperatur erwärmt und weitere 3 Tage gerührt. Bei allen Kombinationen von Basen und Lewis-Säuren war kein Umsatz zu erkennen. Selbst bei dem Ansatz, bei dem Benzyllithiumthiolat als Base in der Kombination mit TiCl₄ verwendet wurde, war kein Umsatz zu beobachten, obwohl ohne den Zusatz von TiCl₄ ein vollständiger Umsatz schon bei 0 °C erreicht werden konnte.

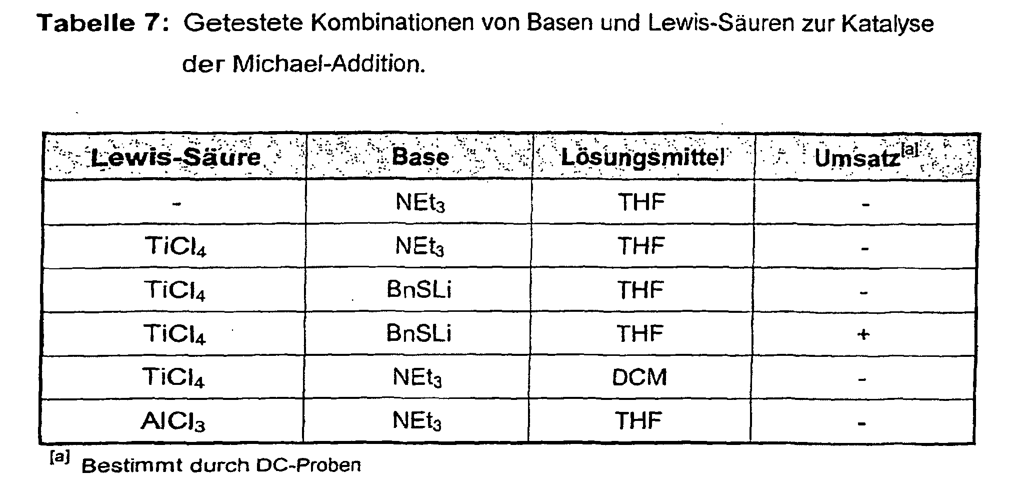

### D) Einfuß des Lösungsrnittels

Die Frage nach dem geeigneten Lösungsmittel stellte sich nun, um bei Reaktionsbedingungen wie unter Kapitel 2.6.1 beschrieben evtl. höhere de-Werte durch Variation des Lösungsmittels zu erzielen.

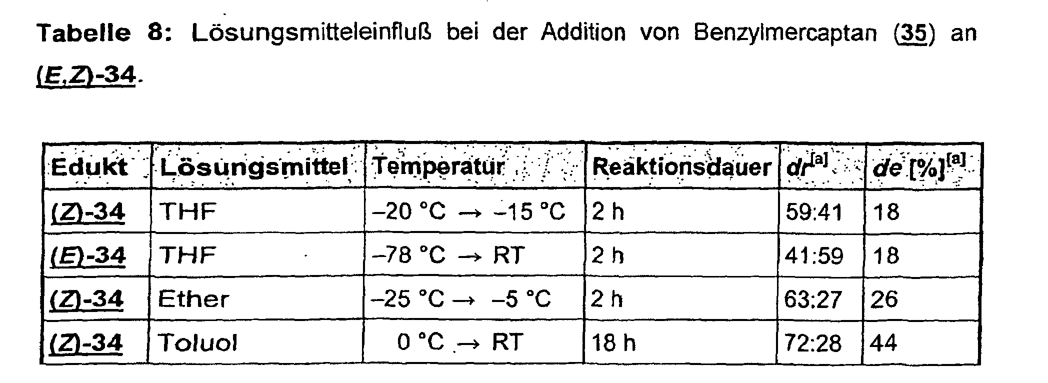

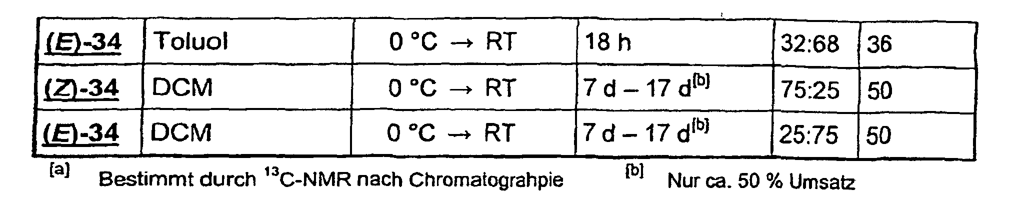

Wie aus Tabelle 8 ersichtlich war eine *de*-Wert-Steigerung durch die Wahl anderer Lösungsmittel möglich. Eine deutliche Steigerung war in den unpolaren Lösungsmitteln wie Toluol und DCM zu erkennen. Dort wurden *de-*Werte 50 % erreicht, jedoch stieg hierbei die Reaktionsdauer von 2 h in THF auf 17 d in DCM. Ebenfalls konnte mit DCM nach 7 - 17 d nur ein 50%iger Umsatz verzeichnet werden.

### E) Versuche zur Steuerung durch Komplexierung des Michael-Donators

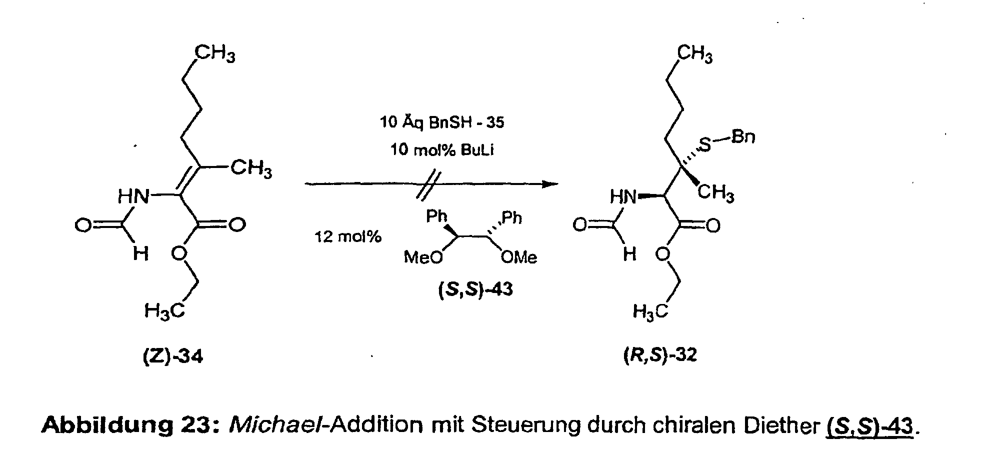

Ziel war es, die *Michael*-Reaktion durch die Zugabe einer chiralen Verbindung zu der mit Thiolat katalysierten Reaktion (siehe Kapitel 2.6.1) zu steuern (siehe Abbildung 23).
Die Steuerung erfolgte nach *Tomioka et al*.^{[33]} durch chirale Bi- oder Triether vorgegangen. Dabei wurde das Benzyllithiumthiolat nur in katalytischen Mengen eingesetzt. Durch die Zugabe des chiralen Dimethylethers **(*S,S*)-43** sollte das Lithiumthiolat komplexiert werden, um dessen Angriff zu steuern. So sollte anstatt des nach Kapitel 2.5.1 und 2.5.4 hergestellten Diastereomerengemisches, enantioselektiv nur ein Diastereomer gebildet werden.
Es wird angenommen, daß sich der in Abbildung 24 aufgezeigte Chelat bildet.^{[32]} Darin wird das Lithiumthiolat von den beiden Sauerstoffatomen des Dimethylethers komplexiert. Beim Angriff koordiniert auch der Carbonytsauerstoff des *Michael*-Akzeptors **34** an das zentrale Lithiumatom, wodurch die Reaktion gesteuert wird.

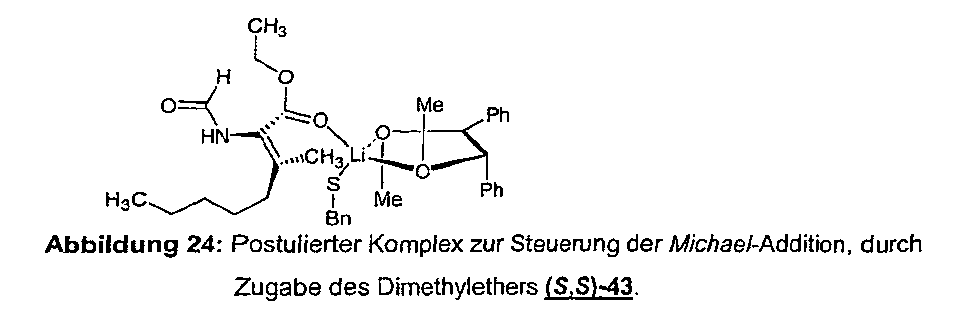

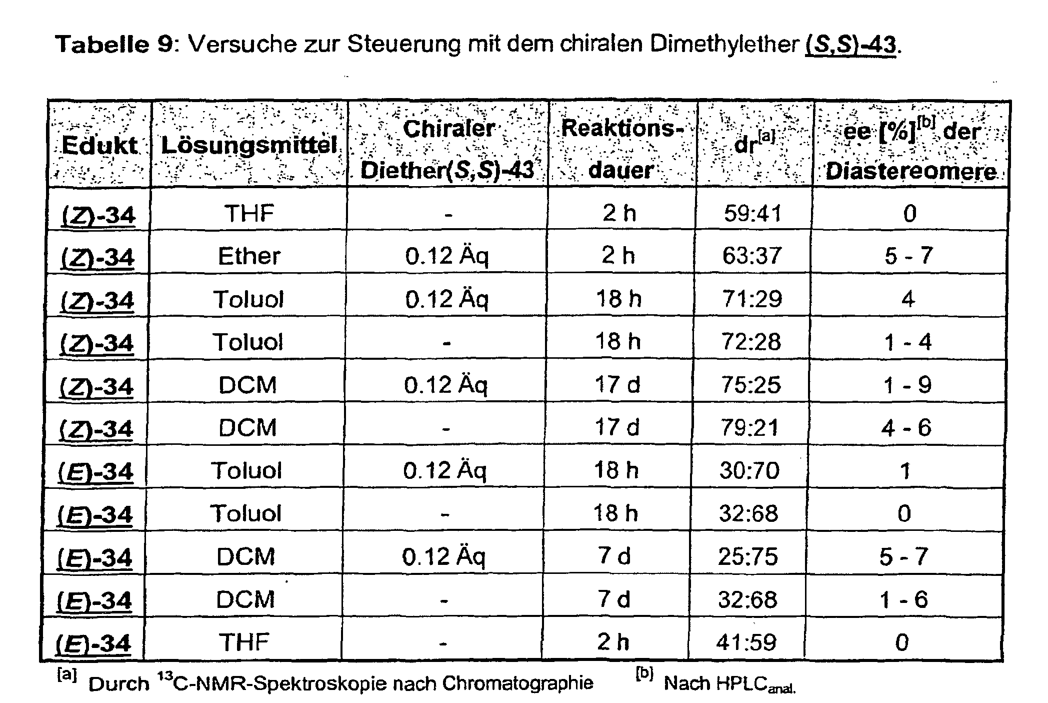

Die Versuche zur Steuerung durch den Dimethylether **(*S,S*)-43** wurden in Ether, DCM und Toluol durchgeführt. 0.1 Äquivalente BuLi wurden bei 0°C vorgelegt und 10 Äquivalente Benzylmercaptan 35 hinzugegeben. Dazu gab man 0.12 Äquivalente des gelösten Dimethylethers **(*S,S*)-43** zu. Es war jedoch keine Komplexbildung durch Farbveränderung zu erkennen. 30 min später wurde ein Äquivalent 2-Formylamino-3-methyl-oct-2-ensäureethylester **34** bei 0 °C zugetropft. Die Reaktion ist nach der jeweils angegebene Zeit durch die Zugabe von 5%iger NaOH beendet worden.
Die Diastereomerenüberschüsse wurden nach Chromatographie aus den ¹³C-NMR-Spektren nach säulenchromatographischer Aufreinigung bestimmt. Die Enantiomerenüberschüsse wurden nach Auskristallisation des Diastereomeren **(*threo*)**-**32** (Pentan/Ethanol) durch analytische HPLC an chiraler stationärer Phase ermittelt.

Wie aus Tabelle 9 zu erkennen ist, war durch die Zugabe des chiralen Dimethylethers keine chirale Induktion der Michael-Addition zu erkennen, da die gemessenen Enantiomerenüberschüsse innerhalb der Genauigkeit der HPLC liegen. Dies liegt daran, daß die aufgereinigten Diastereomere mit dem anderen Diastereomer verunreinigt sind und alle vier Isomere zusammen nicht Basistinien-getrennt vermessen werden konnten.

### Beispiel 6

### Zusammenfassung

Im Rahmen dieser Erfindung wurde zunächst eine Syntheseroute zur Darstellung von (*E*,*Z*)-2-Formylaminoacrylsäureestern **(*E*,*Z*)-34** erarbeitet werden. Dies gelang in einer vierstufigen Synthese, ausgehend von Glycin **(39).** Nach Veresterung, *N*-Formylierung, Kondensation der *N*-Formylaminofunktion und Olifinierung wurde **(*E*,*Z*)-34** in einer Gesamtausbeute von 47% und mit einem (*E*/*Z*)-Verhältnis von 1 : 1.3 erhalten. (siehe Abbildung 25)

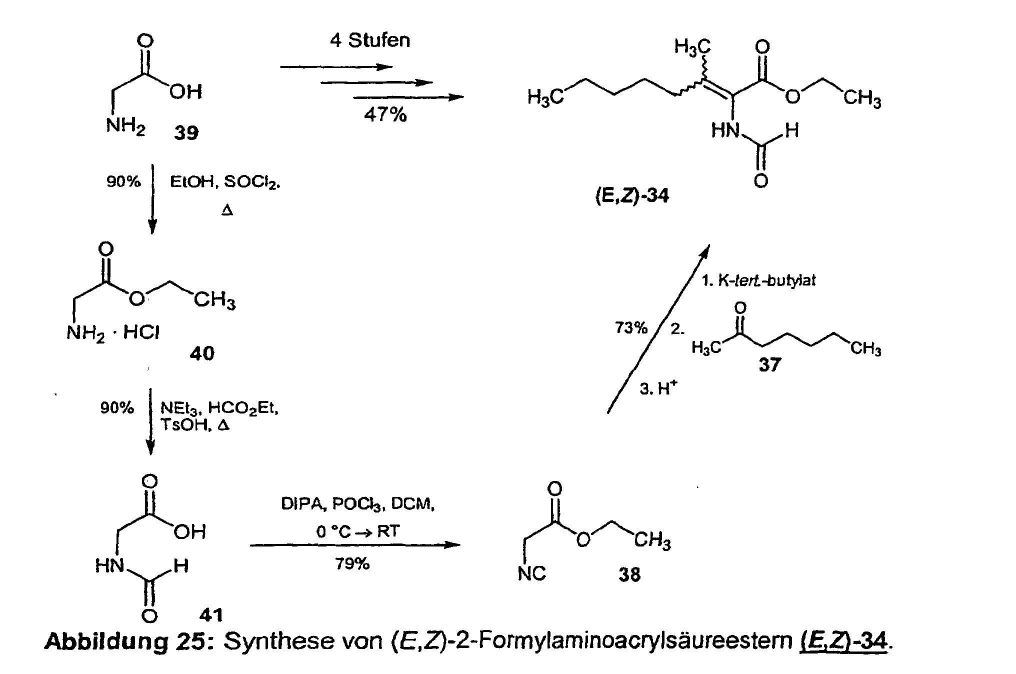

An die synthetisierten (*E*,*Z*)-2-Formylaminoacrylsäureester **(*E*,*Z*)-34** sollten in einer *Michael*-Addition Mercaptane addiert werden. Durch Zugabe von 0.1 Äquivalenten Lithiumthiolat konnte die Reaktion katalysiert werden.
Um eine enentioselektive Steuerung über chirale Katalysatoren zu ermöglichen, wurde der Einsatz verschiedener Katalysatoren untersucht, die später mit chiralen Liganden versehen werden können. Es wurden Lewis-Säuren, Brønsted-Basen und eine Kombination aus beiden in unterschiedlichen Lösungsmitteln auf ihre katalitische Wirkung getestet (siehe Abbildung 26). Es wurden jedoch bisher keine katalitischen Systeme für die angestrebte *Michael*-Addition gefunden.

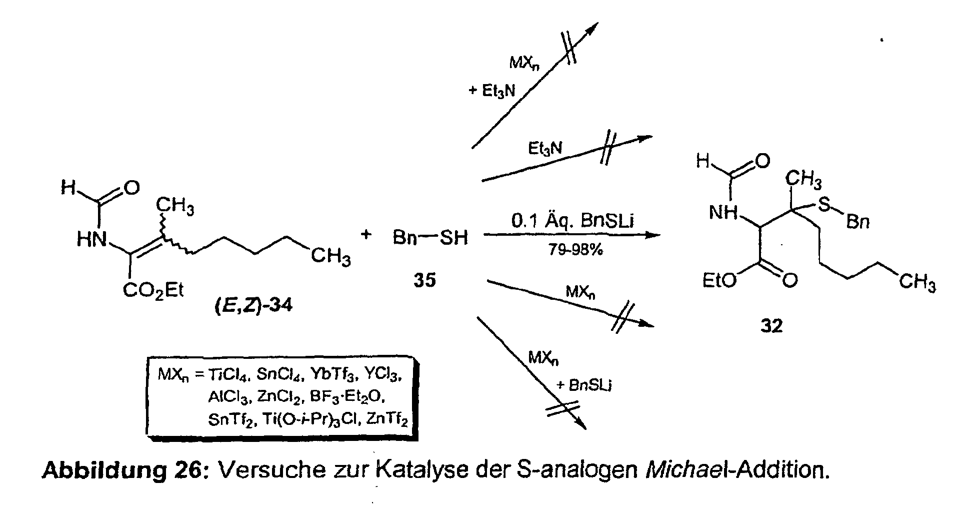

Bei der Thiolat-katalysierten *Michael*-Addition entstand eine Mischung beider Diastereomere. Durch den Wechsel des Lösungsmittels konnte der Diastereomerenüberschuß bei Einsatz von **(Z)-34** von 17% (THF) über 43% (Toluol) auf 50% (DCM) gesteigert werden. Ausgehend von **(*E*)-34** wurde vergleichbare de-Werte mit umgekehrtem Diastereomerenverhältnis erzielt. Allerdings stiegt mit dem de-Wert auch die Reaktionsdauer von 2 h (THF) bis auf 17 d (DCM), um befriedende Umsätze zu erreichen.
Durch Kristallisation des *threo*-Diastereomeren ***(threo)-32*** aus Pentan/Ethanol (10:1) konnte man die *threo-* und erythro-Diastereomere **32** weiter bis zu einem de-Wert von 96% für **(*threo*)-32** und 83% für **(*erythro*)-32** aufreinigen.
Aufgrund der erfolgreichen Katalyse mit 0.1 Äquivalenten Thiolat, wurde versucht den Angriff des Thiolats durch Zugabe des chiralen Diethers (S,S)-1,2-Dimethoxy-1,2-diphenylethan **[(*S,S*)-43]** zu steuern.^{[33]} Dabei wurde in den unpolaren Lösungsmitteln gearbeitet. Jedoch war bislang kein Einfluß des Diethers **(*S*,*S*)-43** auf die Steuerung der Reaktion zu beobachten.

### Beispiel 7:

### Einsatz von TMSCI

Da die in dieser Erfindung entwickelte Trennung der Diastereomeren gut funktioniert, kann man das Thiolat wie in Beispiel 5 A gezeigt stöchiometrisch einsetzen und das Addukt vorzugsweise mit TMSCl als Enolether **45** abfangen. Durch Protonierung dieses Adduktes **45** mit einem chiralen Protonendonator **R*-H** ist die Steuerung des zweiten Zentrums möglich (siehe Abbildung 27).

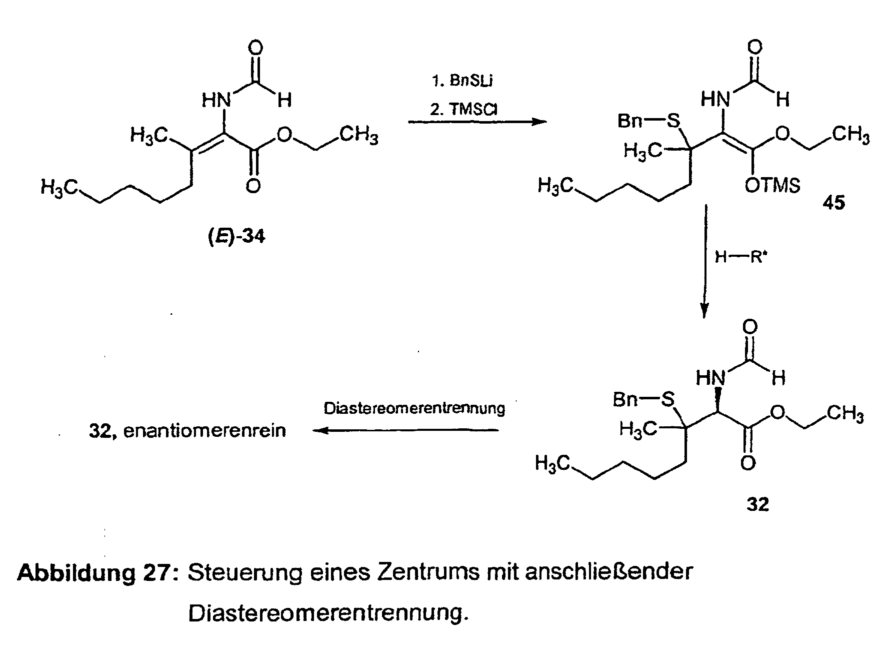

Die beiden gebildeten, enantiomerenreinen Diastereomere können, wie beschrieben, durch Kristallisation getrennt werden. Durch diese Art der Steuerung sind alle vier Stereoisomere einzeln zugänglich.

### Beispiel 8:

### Einsatz sterisch anspruchsvoller Gruppen:

Eine zweite Möglichkeit zur Steuerung der *Michael*-Addition stellt die intramolekulare Steuerung durch sterisch anspruchsvolle Gruppen, vorzugsweise der TBDMS-Gruppe, dar. Diese können nach einer Methode von *D. Enders und B. Lohray*^{[46],[47]} enantioselektiv eingeführt werden. Das von Aceton **(46)** ausgehend hergestellte α-Silylketon 47 würde dann mit Isocyanessigsäureethylester **(38)** zum 2-Formylamino-3-methyl-4-(*t*-butyldimethylsilyl)-2-octensäureethylester **(*E*)-48** und **(*Z*)-48** umgesetzt (siehe Abbildung 28).

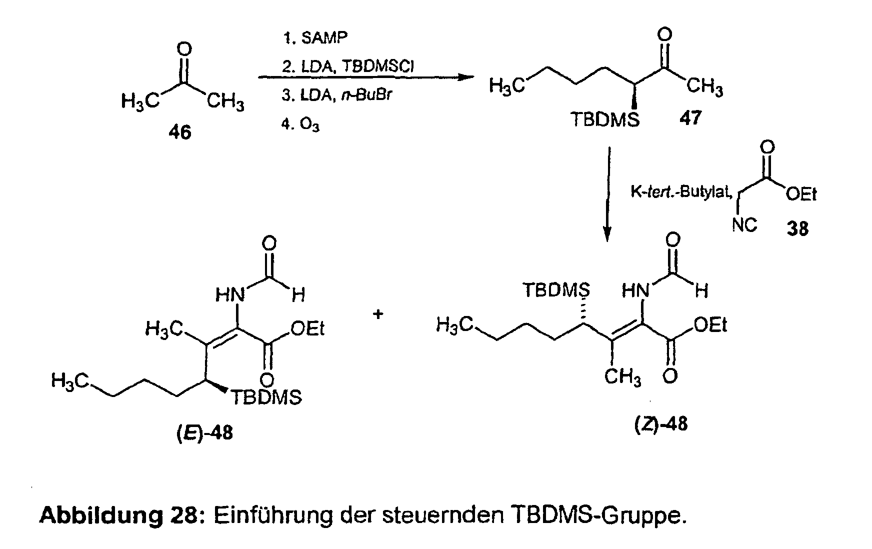

**(*E*)-48** bzw. **(*Z*)-48** werden dann mit einem Thiol in einer *Michael*-Addition umgesetzt, wobei durch die TBDMS-Gruppe und die (E/Z)-Isomere die Reaktion gesteuert wird. Die steuernde TBDMS-Gruppe kann nach der Methode von *T. Otten* ^{[12]} mit *n*-BuNF₄ /NH₄F / HF als Spaltreagenz wieder entfernt werden, wobei die Veröffentlichung von von *T. Otten*^{[12]} Teil der Offenbarung ist. Auch so besteht eine Möglichkeit, alle vier Stereoisomere unabhängig voneinander zu synthetisieren.
Da die zuerst vorgestellte, alternative Synthese immer noch die Möglichkeit der asymmetrischen Katalyse bei der Protonierung des Silylenolethers **45** bietet, ist dieser Weg die bessere Alternative. Bei der zweiten Alternativroute kommt evtl. auch noch das Problem der Silylgruppenabspaltung hinzu, da unter den Bedingungen der Abspaltung manchmal auch die *N*-Formylgruppe unter Bildung des Hydrofluorides abgespalten wird.

### Beispiel 8:

### Experimentelle Bedingungen:

### Anmerkungen zum präparativen Arbeiten

### A) Schutzgastechnik

Alle luft- und feuchtigkeitsempfindlichen Reaktionen wurden in evakuierten, ausgeheizten und mit Septen verschlossenen Kolben unter Argonatmosphäre durchgeführt. Flüssige Komponenten oder in Lösungsmittel gelöste Komponenten wurden mit Hilfe von Kunststoffspritzen mit V2A-Kanülen zugegeben. Feststoffe wurden im Argongegenstrom abgefüllt.

### B) Lösungsmittel

Zum Absolutieren wurden vorgetrocknete bzw. vorgereinigte Lösungsmittel verwendet:
- *Tetrahydrofuran:*: Vierstündiges Refluxieren über Calciumhydrid und anschließende Destillation.
- *abs. Tetrahydrofuran:*: Zweistündiges Refluxieren von vorbehandeltem THF über Natrium-Blei-Legierung unter Argon und anschließende Destillation.
- *Dichlormethan:*: Vierstündiges Refluxieren über Calciumhydrid und anschließende Destillation über eine 1 m Füllkörperkolonne.
- *abs. Dichlormethan:*: Ausschütteln des vorbehandelten Dichlormethan mit konz. Schwefelsäure, Neutralisation, Trocknung, zweistündiges Refluxieren über Calciumhydrid unter Argon und anschließende Destillation.
- *Pentan:*: Zweistündiges Refluxieren über Calciumhydrid und anschließende Destillation über eine 1 m Füllkörperkolonne.
- *Diethylether:*: Zweistündiges Refluxieren über Natrium-Blei-Legierung unter Argon und anschließende Destillation.
- *abs. Diethylether:*: Zweistündiges Refluxieren über KOH und anschließende Destillation über eine 1 m Füllkörperkolonne.
- *Toluol:*: Zweistündiges Refluxieren über Natriumdraht und Destillation über eine 0.5 m Füllkörperkolonne.
- *abs. Toluol:*: Zweistündiges Refluxieren über Natrium-Bleilegierung und anschließende Destillation.
- *Methanol:*: Zweistündiges Refluxieren über Magnesium/Magnesiummethanolat und anschließende Destillation.

### C) Verwendete Reagenzien

- Argon:: Argon wurde von der Firma Linde bezogen.
- *n*-Butyllithium:: *n*-BuLi wurde als 1.6 molare Lösung in Hexan von Firma Merck erworben.
- (S,*S*)-(-)-1,2-Diphenyl-1,2-ethandiol:: wurde von der Firma Aldrich bezogen.
- Benzylmercaptan:: wurde von der Firma Aldrich bezogen
- Ethylmercaptan:: wurde von der Firma Fluka bezogen.
- 2-Heptanon:: wurde von der Firma Fluka bezogen.

Alle übrigen Reagenzien wurden von den Firmen Aldrich, Fluka, Merck und Acros bezogen oder standen dem Arbeitskreis zur Verfügung.

### D) Reaktionskontrolle

Die Dünnschichtchromatographie wurde wurde zur Reaktionskontrolle sowie zur Detektion nach der Säulenchromatographie (siehe Kapitel 3.1.5) verwendet. Sie wurde auf kieselgelbeschichteten Glasplatten mit Fluoreszenzindikator (Merck, Kieselgel 60, Schicht 0.25 mm) durchgeführt. Die Detektion erfolgte durch Fluoreszenzlöschung (Absorption von UV-Licht der Wellenlänge 254 nm) sowie durch Eintauchen in Mostainlösung [5%ige Lösung von (NH₄)₆Mo₇O₂₄ in 10%iger Schwefelsäure (v/v) mit einem Zusatz von 0.3 % Ce(SO₄)₂] und anschließendem Erhitzen im Heißluftstrom.

### E) Produktreinigung

Die Reinigung der Substanzen erfolgte meistens durch Säulenchromatographie in Glassäulen mit eingebauter Glasfritte mit Kieselgel 60, (Firma Merck, Komgröße 0.040 - 0.063 mm). Dabei wurde mit einem Überdruck von 0.1 - 0.3 bar gearbeitet. Das Elutionsmittel wurden zumeist so gewählt, daß der R_{f}-Wert der zu isolierenden Substanz bei 0.35 lag. Die Zusammensetzung der Lösungsmittelgemische wurde volumetrisch abgemessen. Der Durchmesser und die Länge der Säule wurde dem Trennproblem und der Substanzmenge angepaßt.

Einige kristalline Substanzen wurden auch durch Umkristallisation in geeigneten Lösungsmitteln oder Gemischen gereinigt.

### F) Analytik

- *HPLC*_{*präparativ*}*:*: Gilson Abimed; Säule: Hibar® Fertigsäule (25 cm x 25 mm) von Merck und UV-Detektor.
- *HPLC*_{*analytisch*}*:*: Hewlett Packard, Säule: Daicel OD, UV-Detektor
- ^{*1*}*H-NMR-Spektroskopie:*: Varian GEMINI 300 (300 MHz) und Varian Inova 400 (400 MHz) mit Tetramethylsilan als internen Standard.
- ^{*13*}*C-NMR-Spektroskopie:*: Varian GEMINI 300 (75 MHz) und Inova 400 (100 MHz) mit Tetramethylsilan als internen Standard.
- *2D-NMR-Spektroskopie:*: Varian Inova 400.
- *Gaschromatographie:*: Siemens Sichromat 2 und 3; Detektor FID, Säulen: OV-17-CB (fused Silica, 25 m x 0,25 mm, ID); CP-Sil-8 (fused Silica, 30 m x 0,25 mm, ID).
- *IR-Spektroskopie:*: a) Messungen von KBr-Preßlingen:
   Perkin-Elmer FT/IR 1750.
b) Messungen in Lösung:
   Perkin-EImer FT/IR 1720 X.
- *Massenspektroskopie:*: Varian MAT 212 (EI 70 eV, CI 100 eV).
- *Elementaranalyse:*: Heraeus CHN-O-Rapid, Elementar Vario EI.
- *Schmelzpunkte:*: Tottoli-Schmelzpunktapperatur Büchi 535.

### G) Anmerkungen zu den analytischen Daten

- *Ausbeuten:*: Die angegebenen Ausbeuten beziehen sich auf die isolierten gereinigten Produkte
- *Siedepunkte*/*Drücke:*: Die angegebenen Siedetemperaturen wurden innerhalb der Apparatur mit Quecksilberthermometern gemessen und sind unkorrigiert. Die dazugehörigen Drücke wurden mit analogen Meßfühlern gemessen.
- ^{*1*}*H-NMR-Spektroskopie:*: Die chemischen Verschiebungen δ sind in ppm gegen Tetramethylsilan als internen Standard, und die Kopplungskonstanten J in Hertz (Hz) angegeben. Zur Beschreibung der Signalmultiplizitäten werden folgende Abkürzungen verwendet: s = Singulett, d = Dublett, t = Triplett, q = Quartett, quin = Quintett, m = Multiplett. Mit kB wird ein komplexer Bereich eines Spektrums bezeichnet. Ein vorangestelltes br bezeichnet ein breites Signal.
- ^{*13*}*C-NMR-Spektroskopie:*: Die chemischen Verschiebungen δ sind in ppm mit Tetramethylsilan als internen Standard angegeben.
- *de-Werte:*: Die Ermittlung der Diastereomerenüberschüsse *(de)* erfolgte mit Hilfe der ¹³C-NMR-Spektren der Verbindungen. Diese Methode nutzt die unterschiedlichen Verschiebungen diastereomerer Verbindungen im protonenentkoppelten ¹³C-Spektrum.
- *IR-Spektroskopie:*: Die Lage der Absorptionsbanden ( 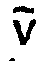 ist in cm⁻¹ angegeben. Zur Charakterisierung der Banden werden folgende Abkürzungen verwendet: vs = sehr stark, s = stark, m = mittel, w = schwach, vw = sehr schwach, br = breit.
- *Gaschromatographie:*: Es wird die Retentionszeit in Minuten der unzersetzten Verbindungen wiedergegeben. Danach werden die Angaben über die Meßbedingungen aufgelistet:
verwendete Säule, Starttemperatur-Temperatur-gradient-Endtemperatur (jeweils in °C) und die Injektionstemperatur Tₛ, falls diese von der Standardtemperatur abweicht. (Sil 8: Tₛ = 270 °C, OV-17: Tₛ = 280 °C
- *Massenspektroskopie:*: Die Angabe der Massen der Fragmentionen (m/z) erfolgt als dimensionslose Zahl, deren Intensität prozentual zum Basispeak ist (rel. Int). Es werden Signale mit hoher Intensität (> 5 %) oder charakteristische Signale angegeben.
- *Elementaranalyse:*: Die Angaben erfolgen in Massenprozenten [%] der angegebenen Elemente. Die Substanzproben wurde für Δ_{C,H,N} ≤ 0.5 % als authentisch betrachtet.

### Beispiel 10:

### Allgemeine Arbeitsvorschriften

### Darstellung von Glycinalkylester-Hydrochloriden [AAV 1]

1.2 Äquivalente Thionylchlorid werden zu 0.6 ml Alkohol pro mmol Glycin unter Eiskühlung bei -10 °C gegeben. Nach Entfernen des Eisbades wird 1 Äquivalent Glycin portionsweise hinzugegeben. Die Mischung wird 2 h unter Rückfluß gerührt. Nach dem Abkühlen auf Raumtemperatur wird am Rotationsverdampfer der überschüssige Alkohol und das Thionylchlorid entfernt. Der erhaltene weiße Feststoff wird zweimal mit dem Alkohol versetzt und dieser wiederum am Rotationsverdampfer entfernt, um anhaftendes Thionylchlorid vollständig zu entfernen.

### Darstellung von Formylaminoessigsäurealkylestern [AAV 2]

1 Äquivalent Glycinalkylester-Hydrochlorid wird in 0.8 ml Ethyl- oder Methylformiat pro mmol Glycinalkylester-Hydrochlorid suspendiert. 130 mg Toluolsulfonsäure pro mol Glycinalkylester-Hydrochlorid zugeben und zum Rückfluß erhitzt. Nun werden 1.1 Äquivalente Triethylamin zu der siedenen Lösung zugetropft und die Reaktionslösung über Nacht unter Rückfluß gerührt.
Nach Abkühlen auf RT wird das ausgefallene Ammoniumchlorid-Salz abfiltriert, das Filtrat auf ein ca. 20% des Ursprungvolumens eingeengt und auf -5 °C gekühlt. Das erneut ausgefallene Ammoniumchlorid-Salz wird abfiltriert, das Filtrat eingeengt und bei 1 mbar destilliert.

### Darstellung von lsocyanessigsäurealkylester [AAV 3]

1 Äquivalent Formylaminoessigsäurealkylester und 2.7 Äquivalente Diisopropyl-amin werden in DCM (10 ml pro mmol Formylaminoessigsäurealkylester) gegeben und mit einem Eisbad auf -3 °C gekühlt. Dann werden 1.2 Äquivalente Phosphorylchlorid hinzugetropft und anschließend eine weitere Stunde bei dieser Temperatur gerührt. Nachdem das Eisbad entfemt und Raumtemperatur erreicht wurde, wird vorsichtig mit 1 ml 20%iger Natriumcarbonat-Lösung pro mmol Formylaminoessigsäurealkylester hydrolysiert. Nach ca. 20 min ist eine starke Schaumbildung zu beobachten und der Kolben muß mit Eiswasser gekühlt werden. Nach 60 minütigem Rühren bei RT werden weiteres Wasser (1 ml pro mmol Formylaminoessigsäurealkylester) und DCM (0.5 ml pro mmol Formylaminoessigsäurealkylester) hinzugegeben. Die Phasen werden getrennt und die organische Phase zweimal mit 5%iger Na₂CO₃-Lösung gewaschen und über MgSO₄ getrocknet. Das Lösungsmittel wird am Rotationsverdampfer entfernt und das zurückbleibende braune Öl destilliert.

### Darstellung von (E)- und (Z)-2-Formylamino-3-dialkylprop-2-ensäurealkylestern [AAV 4]

1.05 Äquivalente Kalium-*tert*.-butanolat werden in 0.7 ml THF pro mmol Isocyanessigsäurealkylester werden auf -78 °C gekühlt. Hierzu wird eine Lösung aus 1.0 Äquivalenten lsocyanessigsäurealkylester in 0.25 ml THF pro mmol langsam hinzugegeben und 30 min bei dieser Temperatur gerührt (→ rosafarbene Suspension). Nun wird eine Lösung aus 1.0 Äquivalenten Keton in 0.125 ml THF pro mmol hinzugetropft. Nach 30 min Rühren bei -78 °C wird auf RT erwärmt (1 h) und es werden 1.05 Äquivalente Eisessig in einer Portion zugegeben (gelbe Lösung) und weitere 20 Minuten gerührt. Das Lösungmittel wird am Rotationsverdampfer (40 °C Badtemperatur) entfernt. Das Rohprodukt wird als Feststoff erhalten. Der Feststoff wird in 1.5 ml Diethylether pro mmol suspendiert und es werden 0.5 ml Wasser pro Äquivalent hinzugegeben. Die klaren Phasen werden getrennt und die wäßrige Phase zweimal mit Diethylether extrahiert. Die vereinigten organischen Phasen werden mit gesättigter NaHCO₃-Lösung gewaschen und über MgSO₄ getrocknet. Nach Entfemen des Lösungsmittels erhält man einen wachsartigen Feststoff. Durch Chromatographie mit Diethylether/Pentan (4:1) als Eluent können die (*E*)*-* und (*Z*)-Produkte getrennt werden.

### Darstellung von 2-Formylamino-3-dialkyl-3-alkylsulfanylpropansäure-alkylester [AAV 5]

0.1 Äquivalente Butyllithium werden in 50 ml THF pro mmol gegeben und auf 0 °C gekühlt. Nun werden 10 Äquivalente des Mercaptans hinzugetropft. Nach 20 minütigem Rühren wird die Lösung auf eine Temperatur zwischen -40 und 0 °C gekühlt und 1 Äquivalent des 2-Formylamino-3-dialkylprop-2-ensäurealkylesters in 5 ml THF pro mmol langsam hinzugegeben. Es wird 2 h bei der eingestellten Temperatur gerührt und danach auf 0 °C erwärmt und mit 5%iger Natriumhydroxid-Lösung hydrolysiert. Die Phasen werden getrennt und die wäßrige Phase zweimal mit DCM extrahiert. Die vereinigten organischen Phasen werden über MgSO₄ getrocknet und das Lösungsmittel am Rotationsverdampfer entfernt. Das im Überschuß eingesetzte Mercaptan kann mittels Chromatographie mit DCM/Diethylether (6:1) als Eluent abgetrennt werden.

### Beispiel 11:

### Spezielle Arbeitsvorschriften und analytische Daten

### A) (S,S)-(-)-1,2-Dimethoxy-1,2-diphenylethan ((S,S)-43)

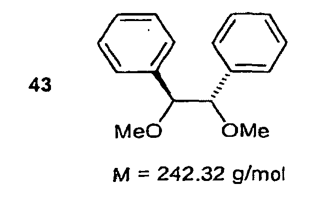

140 mg NaH (60%ig in Paraffin) wird dreimal mit Pentan gewaschen und im HV getrocknet. Dieses wird dann in 5 ml abs. THF suspendiert. Nun werden 250 mg (1.17 mmol) (*S,S*-(-)-2,2-Diphenyl-2,2-ethandiol **(42)** gelöst in 3 ml THF zugetropft. Nach der Zugabe wird 30 min unter Rückfluß gerührt und anschließend auf 5 °C gekühlt. Unter langsamen Zutropfen werden 310 mg Dimethylsulfat zugegeben und weitere 30 min unter Eiskühlung gerührt. Das Eisbad wird entfernt und die Reaktionsmischung auf RT erwärmt, wobei eine viskose weiße Masse entsteht, welche über Nacht bei RT gerührt wird. Die Reaktion wird durch Zugabe von 5 ml gesättigter NH₄Cl-Lösung beendet. Die Phasen werden getrennt und die wäßrige Phase zweimal mit Diethylether extrahiert. Die vereinigten organischen Phasen werden erst mit gesättigter NaHCO₃-Lösung und danach mit Brine gewaschen und über MgSO₄ getrocknet. Nach Entfernen des Lösungsmittels am Rotationsverdampfer erhält man einen farblosen Feststoff, welcher in Pentan (bei -22 °C) umkristallisiert wird. Der Dimethylether wird nun in Form von farblosen Nadeln erhalten.

| | | |
|---|---|---|
| **Ausbeute** | 204 mg | (0.84 mmol, 72% der Theorie) |
| **Smp** | 98.5 °C | (Lit: 99-100 °C)^{[39]} |
| **GC** | Rₜ = 3.08 min | (OV-17, 160-10-260) |

^{**1**}**H-NMR-Spektrum** (400 MHz, CDCl₃):
δ = 7.15 (m, 6 H, H_{Ar}), 7.00 (m, 4 H, H_{Ar}), 4.31 (s, 2 H, CHOCH₃), 3.27 (s, 6 H, CH₃) ppm.
^{**13**}**C-NMR-Spektrum** (100 MHz, CDCl₃):
δ = 138.40 (C_{Ar, quart.}), 128.06 (4xHC_{Ar}), 127.06 (HC_{Ar, para}), 87.98 (CH₃), 57.47 (HCOCH₃) ppm.
**IR-Spektrum (KBr-Preßling):**
~ = 3448 (br m), 3082 (vw), 3062 (m), 3030 (s), 2972 (s), 2927 (vs), 2873 (s), 2822 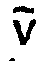 (vs), 2583 (vw), 2370 (vw), 2179 (vw), 2073 (vw), 1969 (br m), 1883 (m), 1815 (m), 1760 (w), 1737 (vw), 1721 (vw), 1703 (w), 1686 (vw), 1675 (vw), 1656 (w), 1638 (vw), 1603 (m). 1585 (w), 1561 (w), 1545 (w), 1525 (vw), 1492 (s), 1452 (vs), 1349 (s), 1308 (m), 1275 (w), 1257(vw), 1215 (vs), 1181 (m), 1154 (m), 1114 (vs), 1096 (vs), 1028 (m), 988 (s), 964 (s), 914 (m), 838 (s), 768 (vs), 701 (vs), 642 (m), 628 (s), 594 (vs), 515 (s) [cm⁻¹].
**Massenspektrum** (Cl, Isobutan): M/z [%J = 212 (M⁺ + 1 - OMe, 16), 211, (M⁺- MeOH, 100), 165 (M⁺- Ph, 2), 121 *(*½ M⁺, 15), 91 (Bn⁺, 3), 85 (M⁺- 157,8),81 (M⁺- 161, 7), 79 (M⁺-163, 6), 71 (M⁺-171,8).

| **Elementaranalyse:** | | |
|---|---|---|
| ber. | C = 79.31 | H = 7.49 |
| gef. | C= 79.12 | H = 7.41 |

Alle übrige analytischen Daten stimmen mit denen der Literatur überein. ^{[34]}

### B) Glycinethylester-Hydrochlorid (40)

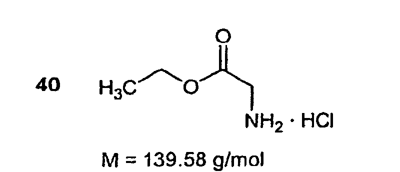

Nach **AAV** 1 werden 1000 ml Ethanol mit 130 g (1.732 mol) Glycin **39** und 247.3 g (2.08 mol) Thionylchlorid umgesetzt. Nach Umkristallisation aus Ethanol erhält man einen farblosen nadelförmigen Feststoff, der im HV getrocknet wird.

| | | |
|---|---|---|
| **Ausbeute** | 218.6 g | (1,565 mol, 90.4% der Theorie) |
| **GC** | Rₜ = 1,93 min | (OV-17, 60-10-260) |
| **Smp.** | 145 °C | (Lit.: 144 °C)^{[48]} |

^{**1**}**H-NMR-Spektrum** (300 MHz, CD₃OD):
δ = 4.30 (q, *J* = 7.14, 2 H, OCH₂), 3.83 (s, 2 H, H₂CNH₂), 1.32 (tr, *J* = 7.14, 3 H, CH₃) ppm.
^{**13**}**C-NMR-Spektrum** (75 MHz, CD30D):
δ = 167.53 (C=O), 63.46 (OCH₂), 41.09 (H₂CNH₂), 14.39 (CH₃) ppm.

Alle übrige analytischen Daten stimmen mit denen der Literatur überein.^{[48]}

### C) N-Formyl-Glycinethylester (41)

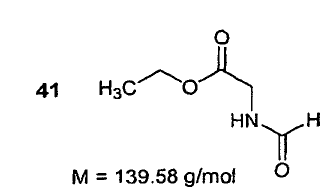

Nach **AAV 2** werden 218 g (1.553 mol) Glycinethylester-Hydrochlorid **40,** 223 mg Toluolsulfonsäure und 178 g Triethylamin in 1.34 l Ethylformiat umgesetzt. Nach Destillation bei 1 mbar erhält man eine farblose Füssigkeit.

| | | |
|---|---|---|
| **Ausbeute** | 184.0 g | (1.403 mol, 90.3% der Theorie) |
| **GC** | Rₜ = 6.95 min (CP-Sil 8, 60-10-300)^{[49]} 117 °C / 1 mbar (Lit.: 119 -120 °C / 1 mbar)^{[49]} | |
| **Sdp.** | | |

Es liegt ein Rotamerenverhättnis um die N-CHO-Bindung von 94:6 vor.
^{**1**}**H-NMR-Spektrum** (400 MHz, CDCl₃):
δ = 8.25, 8.04 (s, d, *J* = 11.81, 0.94 H, 0.06 H, HC=O), 4.22 (dq, *J* = 7.14, 3.05, 2 H, OCH₂), 4.07 (d, *J* = 5.50, 2 H, H₂CC=O), 1.29 (tr, *J* = 7.14, 3 H, CH₃) ppm.
^{**13**}**C-NMR-Spektrum** (100 MHz, CDCl₃):
δ = 169.40 (OC=O), 161.43 (HC=O), 61.55 (OCH₂), 39.90 (H₂CNH₂), 14.10 (CH₃) ppm.

Alle übrige analytischen Daten stimmen mit denen der Literatur überein. ^{[49]}

### D) Isocyanessigsäureethylester (38)

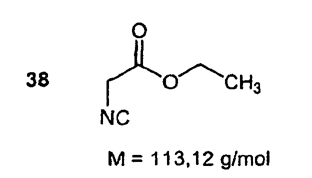

Nach **AAV 3** werden 50 g ( 381 mmol) Formylglycinethylester **41,** 104 g (1.028 mol) Diisopropylamin und 70.1 g (457 mmol) Phosphorylchlorid in 400 ml DCM umgesetzt. Nach Destillation bei 5 mbar erhält man eine leicht gelbe Flüssigkeit.

| | | |
|---|---|---|
| **Ausbeute** | 34.16 g | (302 mmol, 79.3% der Theorie) |
| **GC** | Rₜ = 1.93 min | (OV-17, 50-10-260) |
| **Sdp.** | 77 °C / 5 mbar | (Lit.: 89 - 91 °C / 20 mbar)^{[50]} |

^{**1**}**H-NMR-Spektrum** (300 MHz, CDCl₃):
δ = 4.29 (q, *J* = 7.14, 2 H, OCH₂), 4.24 (d, *J* = 5.50, 2 H, H₂CC=O), 1.33 (tr, *J* = 7.14, 3 H, CH₃) ppm.
^{**13**}**C-NMR-Spektrum** (75 MHz, CDCl₃):
δ = 163.75 (OC=O), 160.87 (NC), 62.72 (OCH₂), 43.58 (H₂CNH₂), 14.04 (CH₃) ppm.
**IR-Spektrum** (kapillar):
= 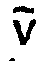 2986 (s), 2943 (w), 2426 (br vw), 2164 (vs, NC), 1759 (vs, C=O), 1469 (w), 1447 (w), 1424 (m), 1396 (vw), 1375 (s), 1350 (s), 1277 (br m), 1213 (vs), 1098 (m), 1032 (vs), 994 (m), 937 (vw), 855 (m), 789 (br m), 722 (vw), 580 (m), 559 (w) [cm⁻ ¹].
**Massenspektrum** (Cl, Isobutan):
M/z [%] = 171 (M⁺ + Isobutan, 6), 170 (M⁺ + Isobutan -1, 58), 114 (M⁺ + 1, 100), 113 (M⁺, 1), 100 (M⁺- 13, 2), 98 (M ⁺- CH₃, 2), 87 (M⁺ - C₂H₅+1, 1), 86 (M⁺ - C₂H₅, 18), 84 (M⁺ - 29, 2).

Alle übrige analytischen Daten stimmen mit denen der Literatur überein. ^{[50]}

### E) (E)- und (Z)-2-Formylamino-3-methyloct-2-ensäureethytester ((E,Z)-34)

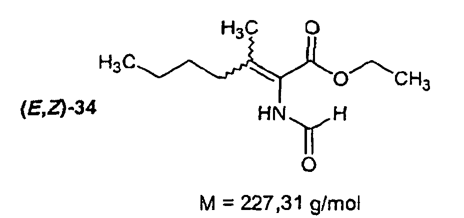

Nach **AAV 4** werden 15 g (132 mmol ) lsocyanessigsäureethylester **38,** 15.6 g (139 mmol) Kalium-*tert*.-Butanolat, 15.1 g (132 mmol) Heptan-2-on **37** und 8.35 g (139 *mmol*) Eisessig umgesetzt.
Durch Chromatographie mit Diethylether/Pentan (4:1) als Eluent werden die (*E*)- und (*Z*)-Produkte voneinander getrennt:

| | | |
|---|---|---|
| **Ausbeute** | 11.52 g | (50.7 mmol, 38.0 % der Theorie) (*Z*)-Produkt |
| | 9.07 g | (39.9 mmol, 30.2 % der Theorie) (*E*)-Produkt |
| | 1.32 g | (5.8 mmol, 4.4 % der Theorie) Mischfraktion |

### F) (Z)-2-Formylamino-3-methyloct-2-ensäureethylester ((Z)-34)

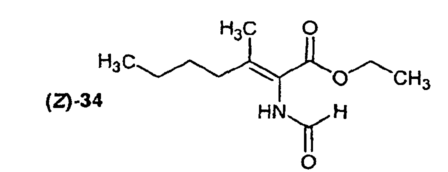

| | | |
|---|---|---|
| **GC** | Rₜ = 12.96 min | (CP-Sil 8, 80-10-300) |
| **Smp.** | 57 °C | (farblos, amorph) |
| **DC** | R_{f} = 0.32 | (Ether:Pentan - 4:1) |
| | R_{f} = 0.34 | (DCM:Ether - 4:1) |

Es liegt ein Rotamerenverhältnis um die N-CHO-Bindung von 65:35 vor.
^{**1**}**H-NMR-Spektrum** (400 MHz, CDCl₃):
δ = 8.21, 7.95 (d, d, *J* = 1.38, 11.40,0.65,0.35 H, HC=O), 6.80, 6.69 (br s, br d, *J* = 11.40, 0.65, 0.35 H, HN), 4.22 (dq, *J* = 1.10, 7.14, 2 H, OCH₂), 2.23 (dtr, *J* = 7.97, 38.73, 2 H, C=CCH₂), 2.20 (dd, *J* = 1.10, 21.7, 3 H, C=CCH₃), 1.45 (dquin, *J* = 1.25, 7.97, 2 H, CCH₂CH₂), 1.30 (dquin, *J* = 4.12, 7.14, 4 H, CH₃CH₂CH₂), 1.30 (m, 3 H, OCH₂CH₃), 0.89 (tr. *J* = 7.00, 3 H, CH₂CH₃) ppm.
^{**13**}**C-NMR-Spektrum** (100 MHz, CDCl₃):
δ = 164.82, 164.36 (OC=O), 159.75 (HC=O), 152.72, 150.24 (C=CNH), 120.35, 119.49 (C=CCH₃), 61.11, 60.89 (OCH₂), 35.82, 35.78 (CH₂), 31.80, 31.72 (CH₂), 27.21, 26.67 (CH₂), 22.45, 22.42 (CH₂), 19.53. 19.17 (C=CCH₃), 14.18 (OCH₂CH₃), 13.94, 13.90 (CH₂CH₃) ppm.
**IR-Spektrum (KBr-Preßling):**
= 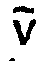 3256 (vs), 2990 (w), 2953 (w), 2923 (m), 2872 (w), 2852 (w), 2181 (br vw), 1711 (vs, C=O), 1659 (vs, OC=O), 1516 (s), 1465 (s), 1381 (s), 1310 (vs), 1296 (vw), 1269 (m), 1241 (s), 1221 (s), 1135 (w), 1115 (vw), 1032 (vs), 1095 (s), 1039 (m), 884 (m), 804 (m), 727 (vw), 706 (vw), 590 (w), 568 (vw) [cm⁻¹].
**Massenspektrum** (El, 70 eV):
M/z [%] = 227 (M⁺, 19), 182 (M⁺ - EtOH+1, 24), 181 (M⁺ - EtOH, 100) 170 (M⁺ - 57, 9), 166 (M⁺ - 61, 8), 156 (M⁺ - 71, 5), 154 (M⁺ - HCO₂Et+1, 6), 153 (M⁺- HCO₂Et, 13), 152 (M⁺- HCO₂Et-1, 13), 142 (M⁺ - 85, 15), 139 (M⁺ - HCO₂Et- CH₃ + 1, 8), 138 (M⁺ - HCO₂Et - CH₃, 65), 126 (M⁺- HCO₂Et - CHO + 2, 16), 125 (M⁺- HCO₂Et - CHO + 1, 34), 124 (M⁺ - HCO₂Et - CHO, 51 ), 114 (M⁺- 113, 36), 111 (M⁺ - HCO₂Et - HNCHO + 1, 17), 110 (M⁺- HCO₂Et - HNCHO, 36), 109 (M⁺- HCO₂Et - HNCHO - 1, 20), 108 (M⁺- HCO₂Et - HNCHO - 2, 10), 98 (M⁺- 129.6), 97 (M⁺- 130, 9), 96 (M⁺ - 131, 12), 82 (M⁺ - 145, 10), 68 (M⁺ - 159, 48), 55 (M⁺ - 172, 12).
**Elementaranalyse:**

| | | | |
|---|---|---|---|
| ber. | C = 63.41 | H=9.31 | N=6.16 |
| gef. | C = 63.51 | H = 9.02 | N=6.15 |

### G) (E)-2-Formylamino-3-methyloct-2-ensäureethylester ((E)-34)

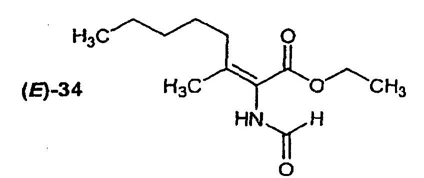

| | | |
|---|---|---|
| **GC** | Rₜ = 13.71 min | (CP-Sil 8, 80-10-300) |
| **Smp.** | 53 °C | (farblos, amorph) |
| **DC** | R_{f} = 0.20 | (Ether:Pentan - 4:1) |
| | R_{f} = 0.26 | (DCM:Ether-4:1) |

Es liegt ein Rotamerenverhältnis um die N-CHO-Bindung von 65:35 vor.
^{**1**}**H-NMR-Spektrum** (400 MHz, CDCl₃):
δ = 8.16, 7.96 (dd, *J* = 1.64, 11.68, 0.65, 0.35 H, HC=O), 6.92, 6.83 (br s, br d, *J* = 11.68, 0.65, 0.35 H, HN), 4.23 (dq, *J* = 0.82, 7.14, 2 H, OCH₂), 2.56 (dtr, *J* = 7.96, 18.13, 2 H, C=CCH₂), 1.90 (dd, *J* = 0.55, 39.55, 3 H, C=CCH₃), 1.51 (m, 2 H, CCH₂CH₂), 1.32 (dquin, *J* = 2.48, 7.14, 4 H, CH₃CH₂CH₂), 1.32 (m, 3 H, OCH₂CH₃), 0.90 (dtr, *J* = 3.57, 7.14, 3 H, CH₂CH₃) ppm.
^{**13**}**C-NMR-Spektrum** (100 MHz, CDCl₃):
δ = 164.75,164.14 (OC=O), 158.96 (HC=O), 151.38, 150.12 (C=CNH), 120.74, 119.48 (C=CCH₃), 61.10, 60.90 (OCH₂), 35.59 (CH₂), 31.90 (CH₂), 28.09, 28.04 (CH₂), 22.48 (CH₂), 20.89 (C=CCH₃), 14.17 (OCH₂CH₃), 13.99 (CH₂CH₃) ppm.
**IR-Spektrum** (KBr-Preßling):
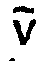 = 3276 (vs), 2985 (w), 2962 (w), 2928 (m), 2859 (m), 2852 (w), 1717 (vs, C=O), 1681 (s, OC=O), 1658 (vs, OC=O), 1508 (s), 1461 (s), 1395 (s), 1368 (vw), 1301 (vs), 1270 (w), 1238 (m), 1214 (s), 1185 (m), 1127 (m), 1095 (s), 1046 (m), 1027 (w), 932 (m), 886 (s), 793 (m), 725 (br s), 645 (m), 607 (m), 463 (w) [cm⁻¹].
**Massenspektrum** (EI, 70 eV):
M/z [%] = 227 (M⁺, 19), 182 (M⁺- EtOH + 1, 20), 181 (M⁺- EtOH, 100), 170 (M⁺ - 57, 8), 166 (M⁺ - 61, 8), 156 (M⁺ - 71, 7), 154 (M⁺- HCO₂Et + 1, 6), 153 (M⁺- HCO₂Et, 14), 152 (M⁺- HCO₂Et - 1, 12), 142 (M⁺- 85, 151), 139 (M⁺- HCO₂Et - CH₃ + 1, 8), 138 (M⁺- HCO₂Et - CH₃, 58), 126 (M⁺- HCO₂Et - CHO + 2, 13), 125 (M⁺- HCO₂Et - CHO +1, 32), 124 (M⁺ - HCO₂Et - CHO, 46), 114 (M⁺-113, 31), 111 (M⁺- HCO₂Et - HNCHO + 1, 16), 110 (M⁺ - HCO₂Et - HNCHO, 34), 109 (M⁺ - HCO₂Et - HNCHO - 1, 18), 108 (M⁺ - HCO₂Et - HNCHO - 2, 9), 98 (M⁺ - 129, 5), 97 (M⁺-130, 7), 96 (M⁺ - 131, 11), 93 (M⁺- 134, 7), 82 (M⁺- 145, 9), 69 (M⁺- 158, 6), 68 (M⁺- 159, 43), 55 (M⁺ - 172, 10).
**Elementaranalyse**:

| | | | |
|---|---|---|---|
| ber. | C = 63.41 | H=9.31 | N=6.16 |
| gef. | C = 63.23 | H = 9.38 | N = 6.10 |

### H) 3-Benzylsulfanyl-2-formylamino-3-methyloctansäure-ethylester (32)

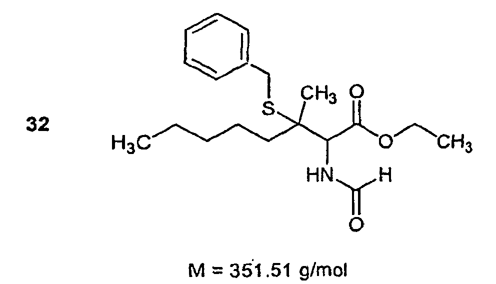

Nach **AAV 5** werden 0.28 ml (0.44 mmol) n-Butyllithium, 5.5 g (44 mmol) Benzylmercaptan **35** und 1 g (4.4 mmol) 2-Formylamino-3-methyloct-2-ensäureethylester (**34**) in 40 ml abs. THF umgesetzt (-78 °C → RT). Das erhaltene farblose Öl wird mit DCM/Ether (6:1) säulenchromatographisch gereinigt, wobei man ein farbloses, zähflüssiges Öl erhält.

| | | |
|---|---|---|
| **Ausbeute** | 1.51 g | (43 mmol, 98% der Theorie) |
| **DC** | R_{f} = 0.51 | (DCM:Ether- 6:1) |

Die entstandenen Diastereomeren können durch präparative HPLC oder durch Kristallisation in Pentan/Ethanol (10:1) voneinander getrennt werden.

### J) threo-Diastereomer ((threo)-32):

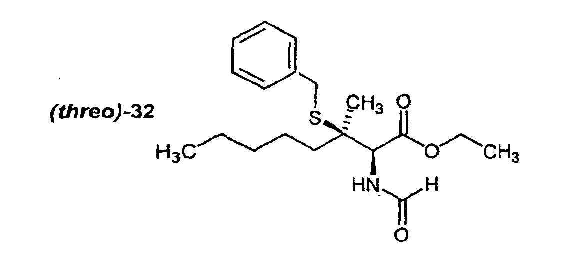

| | | |
|---|---|---|
| **Smp.** | 75 °C | (farblos, nadelförmig, kristallin) |
| **de** | > 96% | (nach ¹³C-NMR) |
| **HPLC**_{**präp.**} | 19.38 min | (Ether:Pentan - 85:15) |

Es liegt ein Rotamerenverhältnis um die N-CHO-Bindung von 91:9 vor.
^{**1**}**H-NMR-Spektrum** (400 MHz, CDCl₃):
δ = 8.22, 7.98 (s, d, *J* = 11.54, 0.91, 0.09 H, HC=O), 7.21 - 7.32 (kB, 5 H, CHₐᵣ),
6.52, 6.38 (dm, *J* = 8.66, 0.91, 0.09 H, HN), 4.74 (d, *J* = 8.66, 1 H, CHNH), 4.24 (ddq, J = 17.85, 10.71, 7.14, 2 H, OCH₂), 3.71 (s, 2 H, SCH₂), 1.56 (m, 3H, SCCH₃). 1.45 (dquin, 1.25, 7.97, 2 H, CCH₂CH₂), 1.20 - 1.45 (kB, 11 H, CH3CH₂CH₂CH₂CH₂+OCH₂CH₃), 0.89 (dtr, J = 3.3, 7.00, 3 H, CH₂CH₃) ppm.
^{**13**}**C-NMR-Spektrum** (100 MHz, CDCl₃):
δ = 170.37 (OC=O), 160.90 (HC=O), 137.31 (C_{Ar}, _{quart}), 129.31 (HC_{Ar}), 128.81 (HC_{Ar}), 127.41 (HC_{Ar}, ₚₐᵣₐ), 61.94 (OCH₂), 57.00 (CHNH). 52.30 (CS), 38.59 (CH₂), 33.31 (CH₂), 32.42 (CH₂), 24.00 (CH₂), 22.92 (CH₂), 22.51 (SCCH₃). 14.54 (OCH₂CH₃), 14.42 (CH₂CH₃) ppm.
**IR-Spektrum** (KBr-Preßling):
= 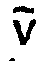 3448 (m), 3184 (br vs), 3031 (m), 2975 (m), 2929 (s), 2899 (w), 2862 (m), 1954 (w), 1734 (vs, C=O), 1684 (vs, OC=O), 1601 (w), 1561 (s), 1495 (m), 1468 (s), 1455 (m), 1296 (vw), 1441 (w), 1381 (vs), 1330 (s), 1294 (m), 1248 (s), 1195 (vs), 1158 (w), 1126 (s), 1096 (s), 1070 (w), 1043 (vw), 1028 (w), 1008 (s), 958 (m), 919 (w), 854 (s), 833 (m), 783 (s), 715 (vs), 626 (vw), 626 (m), 567 (vw) 483 (s) [cm⁻¹].
**Massenspektrum** (El, 70 eV):
M/z [%] = 351 (M⁺, 1), 324 (M⁺- C₂H₅, 1), 306 (M⁺- C₂H₅OH - 1, 1), 278 (M⁺- 73,1), 250 (M⁺- HCO₂Et - HCO, 1), 223 (M⁺ - 128, 5), 222 (M⁺ - 129, 16), 221 (M⁺ - EtO₂CCHNHCHO, 100), 184 (M⁺- 167,6),91 (M⁺- 260, 71).
**Elementaranalyse**

| | | | |
|---|---|---|---|
| ber. | C = 64.92 | H = 8.32 | N=3.98 |
| gef. | C = 64.88 | H = 8.40 | N = 3.92 |

### K) erythro-Diastereomer ((erythro)-32):

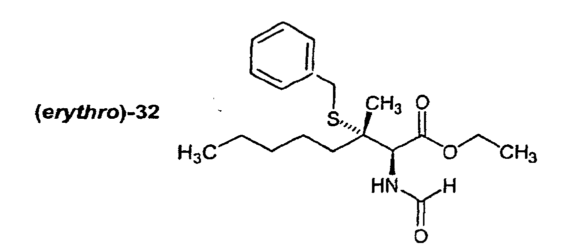

Klare ölige Flüssigkeit

| | | |
|---|---|---|
| ***de*** | 82% | (nach ¹³C-NMR) |
| **HPLC**_{**präp**}**.** | 20.61 min | (Ether:Pentan - 85:15) |

Es liegt ein Rotamerenverhältnis um die N-CHO-Bindung von 91:9 vor.
^{**1**}**H-NMR-Spektrum** (400 MHz, CDCl₃):
δ = 8.22, 7.97 (s, d, *J* = 11.54, 0.91, 0.09 H, HC=O), 7.20 - 7.34 (kB, 5 H, CHₐᵣ),
6.61, 6.43 (br dm, *J* = 9.34, 0.91, 0.09 H, HN), 4.74 (d, *J* = 9.34, 1 H, CHNH), 4.24 (ddq, *J* = 17.85, 10.71, 7.14, 2 H, OCH₂), 3.77 (d, *J* = 11.53, 1 H, SCHH), 3.69 (d, *J* = 11.53, 1 H, SCHH), 1.70 (m, 2 H, CH₂), 1.52 (m, 2 H, CH₂), 1.17-1.40 (kB, 10 H, CH₃C + 2 × CH₂ + OCH₂CH₃), 0.90 (tr, *J* = 7.14, 3 H, CH₂CH₃) ppm.
^{**13**}**C-N MR-Spektrum** (100 MHz, CDCl₃):
δ = 169.87 (OC=O), 160.49 (HC=O), 137.05 (C_{Ar, quart}), 128.91 (HC_{Ar}), 128.40 (HC_{Ar}), 126.99 (HC_{Ar}, ₚₐᵣₐ), 61.52 ( OCH₂), 56.81 (CHNH), 51.91 (CS), 37.51 (CH₂), 32.83 (CH₂), 32.13 (CH₂), 23.65 (CH₂), 23.19 (CH₂), 22.55 (SCCH₃), 14.11 (OCH₂CH₃), 14.03 (CH₂CH₃) ppm.
**IR-Spektrum** (kapillar):
= 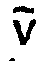 3303 (br vs), 3085 (vw), 3062 (w), 3029 (m), 2956 (vw), 2935 (vw), 2870 (w), 2748 (w), 1949 (br w), 1880 (br w), 1739 (vs, C=O), 1681 (vs, OC=O), 1603 (m), 1585 (vw), 1496 (br vs), 1455 (vs), 1381 (br vs), 1333 (s), 1197 (br vs), 1128 (w), 1095 (m), 1070 (s), 1030 (vs), 971 (br w), 918 (m), 859 (s), 805 (vw), 778 (m), 714 (vs), 699 (vw), 621 (w), 569 (w) 484 (s) [cm⁻¹].
**Massenspektrum (El, 70 eV):**
M/z [%] = 351 (M⁺, 1), 324 (M⁺ - C₂H₅, 1), 306 (M⁺ - C₂H₅OH-1, 1), 278 (M⁺- 73, 1), 250 (M⁺ - HCO₂Et- HCO, 1), 223 (M⁺ - 128, 6), 222 (M⁺- 129, 17), 221 (M⁺-EtO₂CCHNHCHO, 100), 184 (M⁺ - 167, 6), 91 (M⁺ - 260, 70).
**Elementaranalyse:**

| | | | |
|---|---|---|---|
| ber. | C = 64.92 | H = 8.32 | N = 3.98 |
| gef. | C = 64.50 | H = 8.12 | N = 4.24 |

### L) 3-Ethylsulfanyl-2-formylamino-3-methyloctansäureethylester (33)

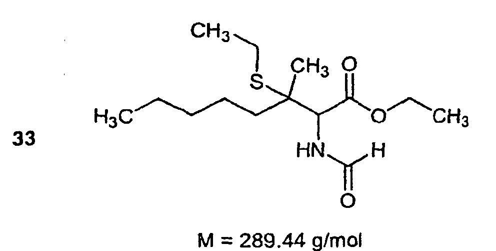

Nach **AAV 5** werden 0.28 ml (0.44 mmol) *n*-Butyllithium, 2.73 g (44 mmol) Ethylmercaptan **36** und 1 g (4.4 mmol) (*E*)-2-Formylamino-3-methyloct-2-ensäureethylester **(E)-34** in 40 ml abs. THF umgesetzt (-78 °C → RT). Es wird ein farbloses Öl erhalten, welches mit DCM/Ether (6:1) säulenchromatographisch gereinigt wird., Das Produkt wird als eine farbloses, viskoses Öl erhalten.

| | | |
|---|---|---|
| **Ausbeute** | 1.05 g | (36.3 mmol, 82 % der Theorie) |
| **de** | 14% | (nach ¹H- und ¹³C-NMR) |
| **DC** | R_{f}=0.49 | (DCM:Ether-4:1) |

Es liegt ein Rotamerenverhältnis um die N-CHO-Bindung von 91:9 vor.
^{**1**}**H-NMR-Spektrum** (400 MHz, CDCl₃, Diastereomerengemisch):
δ = 8.26 (s, 0.91 H, HC=O), 8.02 (d, *J* = 11.82 + d, *J* = 11.81, 0.09 H, HC=O), 6.79 (d, *J* = 9.34 + d, *J* = 8.71, 0.91 H, HN), 6.55 (m, 0.09 H, HN), 4.77 (d, *J* = 9.34, 0.57 H, CHNH), 4.64 (d, *J* = 8.71, 0.43 H, CHNH), 4.22 (m, 2 H, OCH₂), 2.50 (m, 2 H, SCH₂), 1.43 - 1.73 (kB, 4 H, 2 × CH₂), 1.20 - 1.37 (kB, 10 H), 1.18 (tr, *J* = 7.42 + tr, *J* = 7.00, 3H, SCH₂CH₃), 0.90 (dtr, *J* = 4.71, 7.14, 3H, CH₂CH₃) ppm.
^{**13**}**C-NMR-Spektrum** (100 MHz, CDCl₃, Diastereomerengemisch):
δ = 170.36, 170.25 (OC=O), 160.98, 160.93 (HC=O), 61.74, 61.70 (OCH₂), 57.15, 57.02 (CHNH), 51.19 (SC_{quart}), 38.66, 37.86 (CH₂), 32.51, 32.42 (CH₂), 23.94 (CH₂), 23.45, 22.50 (SCCH₃), 22.90, 22.85 (CH₂), 22.17, 22.11 (CH₂), 14.44, 14.41 (OCH₂CH₃), 14.38, 14.36 (SCH₂CH₃), 14.27, 14.25 (CH₂CH₃) ppm.
**IR-Spektrum** (kapillar):
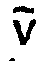 = 3310 (br s), 2959 (s), 2933 (vs), 2871 (s), 2929 (s), 2746 (br w), 1739 (vs, C=O), 1670 (vs, OC=O), 1513 (br s), 1460 (m), 1468 (m), 1381 (s), 1333 (m), 1298 (vw), 1262 (w), 1196 (vs), 1164 (vw), 1127 (m), 1096 (m), 1070 (w), 1030 (s), 978 (w), 860 (m), 833 (m), 727 (br m) [cm⁻¹].
**Massenspektrum** (EI, 70 eV):
M/z [%] = 289 (M⁺, 1), 260 (M⁺ - C₂H₅, 1), 244 (M⁺ - C₂H₅OH-1, 1), 228 (M⁺ - SC₂H₅, 1), 188 (M⁺ - HCO₂Et-HCO, 1), 161 (M⁺ - 128, 5), 160 (M⁺ - 129, 11), 159 (M⁺-EtO₂CCHNHCHO, 100), 97 (M⁺ - 192, 11), 89 (M⁺- 200, 11), 75 (M⁺ - 214, 5), 55 (M⁺ - 214, 14).
**Elementaranalyse:**

| | | | |
|---|---|---|---|
| ber.z | C = 58.10 | H = 9.40 | N = 4.84 |
| gef. | C=57.97 | H = 9.74 | N=5.13 |

Durch Kristallisation in Pentan/Ethanol konnten nach 30 Tagen Kristalle das threo-Diastereoisomere **(*threo*)-33** in hoher Reinheit erhalten werden:

### M) threo-Diastereomer ((threo)-33):

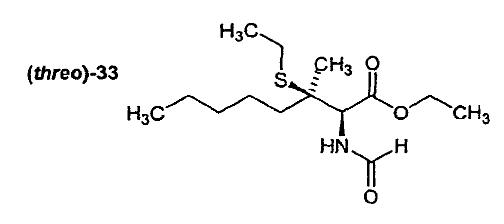

| | | |
|---|---|---|
| ***de*** | 86 % | (nach ¹³C-NMR) |
| **Smp** | 45.5 °C | (farblos, kristallin) |

Es liegt ein Rotamerenverhältnis um die N-CHO-Bindung von 91:9 vor.
^{**1**}**H-NMR-Spektrum** (300 MHz, CDCl₃):
δ = 8.26, 8.01 (br s, dd, *J* = 11.81 H, 0.91, 0.09 H, HC=O), 6.61, 6.40 (dm, *J* = 9.06, 0.91, 0.09 H, HN), 4.77 (d, *J* = 9.34, 0.57 H, CHNH), 4.22 (ddq, *J* = 7.14, 10.72, 17.79, 2 H, OCH₂), 2.50 (ddq, *J* = 7.42, 10.72, 27.36, 2 H, SCH₂), 1.42 - 1.76 (kB, 4 H, 2 × CH₂), 1.24 - 1.38 (kB, 10 H), 1.18 (dtr, *J* = 3.3, 7.42, 3 H, SCH₂CH₃), 0.90 (tr, *J* = 7.14, 3 H, CH₂CH₃) ppm.
^{**13**}**C-NMR-Spektrum** (75 MHz, CDCl₃):
δ = 170.13 (OC=O), 160.71 (HC=O), 61.50 (OCH₂), 56.85 (CHNH), 50.97 (SC_{quart.})_{,} 37.64 (CH₂), 32.22 (CH₂), 23.66 (CH₂), 23.47 (SCCH₃), 22.60 (CH₂), 21.81 (CH₂), 14.09 (OCH₂CH₃), 14.07 (SCH₂CH₃), 13.93 (CH₂CH₃) ppm.
**IR-Spektrum** (KBr-Preßling):
= 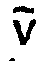 3455 (m), 3289 (br s), 3036 (w), 2981 (s), 2933 (vs), 2860 (vs), 2829 (s), 2755 (br m), 2398 (vw), 2344 (vw), 2236 (vw), 2062 (w), 1737 (vs, C=O), 1662 (vs, OC=O), 1535 (s), 1450 (m), 1385 (s), 1373 (s), 1334 (vs), 1267 (m), 1201 (vs), 1154 (m), 1132 (s), 1118 (w), 1065 (m), 1050 (w),1028 (s), 1016 (m), 978 (m), 959 (vw), 929 (w), 896 (m), 881 (m), 839 (w), 806 (m), 791 (m), 724 (s), 660 (m), 565 (m) [cm⁻¹].
**Massenspektrum** (Cl, Isobutan):
M/z [%] = 346 (M⁺ + Isobutan - 1, 2), 292 (M⁺ + 3, 6), 291 (M⁺ + 2, 17), 290 (M⁺ + 1, 100), 245 (M⁺ - C₂H₅OH, 1), 228 (M⁺- SC₂H₅, 6), 159 (M⁺ - EtO₂CCHNHCHO, 8).
**Elementaranalyse:**

| | | | |
|---|---|---|---|
| ber. | C=58.10 | H = 9.40 | N = 4.84 |
| gef. | C = 58.05 | H = 9.73 | N = 4.76 |

Diastereoisomer **(*erythro*)-33** konnte durch Kristallisation von **(*threo*)-33** bisher nur mit einem de von 50% erhalten werden, wovon keine getrennte Analytik angefertigt wurde.

### Abkürzungsverzeichnis

- AAV: Allgemeine Arbeitsvorschrift
- abs.: absolut
- Äq.: Äquivalente
- AcCl: Essigsäurechlorid
- Ar: Aromat
- ber.: berechnet
- Bn: Benzyl
- Brine: gesättigte NaCl-Lösung
- BuLi: Butyllithium
- DC: Dünnschichtchromatographie
- DIPA: Diisopropylamin
- DCM: Dichlormethan
- *de*: diastereomeric excess
- DMSO: Dimethylsulfoxid
- *dr*: diastereomeric ratio
- *ee*: enantiomeric excess
- Et: Ethyl
- et al.: et altera
- GC: Gaschromatographie
- gef.: gefunden
- ges.: gesättigt
- HPLC: High Pressure Liquid Chromatography
- IR: Infrarot
- konz.: konzentriert
- Lit.: Literatur
- Me: Methyl
- min: Minute
- MS: Massenspektroskopie
- NMR: Nuclear magnetic resonance
- quart.: Quartär
- Pr: Propyl
- R: organischer Rest
- RT: Raumtemperatur
- Sdp.: Siedepunkt
- Smp.: Schmelzpunkt
- TBS: *tert*-Butyldimethylsilyl
- Tf: Triflat
- THF: Tetrahydrofuran
- TMS: Trimethylsilyl
- TsOH: Toluolsulfonsäure
- v: Volumen

### Literaturverzeichnis

1. ^{[1]} D. Enders, R. W. Hoffmann, *Ch. i. u. Z.* **1985,** 19,177.
2. ^{[1]} L. Pasteur, *Ann. Chim.* **1848,** 24, 442.
3. ^{[1]} J. A. Le Bel, *Bull Soc. Chim. Fr.* **1874,** 22,337.
4. ^{[1]} J. H. van't Hoff, *Bull. Soc. Chim. Fr.* **1875,** 23, 295.
5. ^{[1]} T. Laue, A. Plagens, *Namen- und Schlagwort-Reaktionen der Organischen Chemie,* B. G. Teubner Verlag Stuttgart **1998.**
6. ^{[1]} R. Brückner, *Reakfionsmechanismen,* Spektrum Akademischer Verlag Heidelberg **1996.**
7. ^{[1]} E. E. Bergmann, D. Ginsburg, R. Rappo, *Org. React.* **1959,** *10,* 179.
8. ^{[1]} T. Hudlicky, J. D. Price, *Chem. Rev.* **1989,** *89*, 1467.
9. ^{[1]} H. Scherer, *Dissertation,* RWTH Aachen, **1991.**
10. ^{[1]} S. G. Pyne, P. Bloem, S. L. Chapman, C. E. Dixon, R. Griffith, *J. Org. Chem* **1990,** *55*, 1086.
11. ^{[1]} E. S. Gubnitskaya, L. P. Peresypkina, L. I. Samarai, *Russ. Chem. Rev.* **1990,** *59*, 807.
12. ^{[1]} T. Otten, *Dissertation,* RWTH Aachen, **2000.**
13. ^{[1]} D. Enders, H. Wahl, W. Bettray, *Angew. Chem.* **1995,** *107*, 527.
14. ^{[1]} V. S. Martin, M. T. Ramirez, M. S. Soler, *Tetrahedron Lett.* **1990,** *31*, 763.
15. ^{[1]} W. Amberg, D. Seebach, *Chem. Ber.* **1990,** *123*, 2250.
16. ^{[1]} D. Enders, K. Heider, G. Raabe, *Angew. Chem.* **1993,** *105*, 592.
17. ^{[1]} A. Kamimura, H. Sasatani, T. Hashimoto, T. Kawai, K. Hori, N. Ono, *J. Org. Chem.* **1990,** *55*, 3437.
18. ^{[1]} W. D. Rudorf, R. Schwarz, Wiss. Z.-Martin-Luther Univ. Halle-Wittenberg, Math. Naturwiss. Reihe **1989,** *38*, 25.
19. ^{[1]} K. Tomioka, A. Muraoka, M. Kanai, *J. Org. Chem.* **1995,** *60*, 6188.
20. ^{[1]} T. Naito, O. Miyata, T. Shinada, I. Ninomiya, *Tetrahedron* **1997,** *53*, 2421.
21. ^{[1]} a) D. A. Evans, D. M. Ennis, D. J. Mathre, *J. Am. Chem. Soc.* **1982,** *104,* 1737.
   b) D. A. Evans, K. T. Chapman, J. Bisaha, *J. Am. Chem.* Soc. **1988,** *110*, 1238.
22.^{[1]} A. A. Schleppnik, F. B. Zienty, *J*. *Org. Chem.* **1964,** *39*,1910.
23. ^{[1]} T. Mukaiyama, K. Suzuki, I. Ikegawa, *Bull. Chem. Soc. Jpn.* **1982,** *55*, 3277.
24. ^{[1]} CD Römpp Chemie Lexikon - Version 1.0, Stuttgart/New York: Georg Thieme Verlag **1995.**
25. ^{[1]} A. Kumar, R. V. Salunkhe, R. A. Rane, S. Y. Dike, *J*. *Chem. Soc., Chem. Commun.* **1991,** 485.
26. ^{[1]} H. Wynberg, H. Hiemstra, *J. Am. Chem. Soc.* **1981,** *103,417.*
27. ^{[1]} T. Mukaiyama, T. lzawa, K. Saigo, H. Takei, *Chem. Lett.* **1973,** 355.
28. ^{[1]} D. A. Evans, M. C. Willis, J. N. Johnston, Org. *Lett.* **1999,** *1*, 865.
29. ^{[1]} S. Kanemasa, Y. Oderaotoshi, E. Wada, J. *Am. Chem.* Soc. **1999,** *121*, 8675.
30. ^{[1]} B. L. Feringa, E. Keller, N. Veldman, A. L. Speck, *Tetrahedron Asymmetry* **1997,** *8*, 3403.
31. ^{[1]} M. Shibasaki, T. Arai, H. Sasai, *J. Am. Chem.* Soc. **1998,** *120,* 4043.
32. ^{[1]} K. Tomioka, *Synthesis* **1990,** 541.
33.^{[1]} K. Tomioka, K. Nishimura, M. Ono, Y. Nagaoka, J. *Am. Chem. Soc.* **1997,** *119*, 12974.
34. ^{[1]} K. Tomioka, M. Shindo, K. Koga, J. Org. *Chem.* **1998,** *63*, 9351.
35. ^{[1]} C. H. Wong, W. K. C. Park, M. Auer, H. Jasche, *J. Am. Chem. Soc.* **1996,** *118*, 10150.
36.^{[1]} I . Ugi, R. Obrecht, R. Herrmann, *Synthesis* **1985,** 400.
37.^{[1]} I. Ugi, U. Fetzer, U. Eholzer, H. Knupper, K. Offermann, *Angew. Chem.* **1965,** *11*, 492.
38. ^{[1]} I. Maeda, K. Togo, R. Yoshida, *Bull. Chem. Soc. Jpn.* **1971**, *44*,1407.
39.^{[1]} U. Schöllkopf, R. Meyer, *Liebigs Ann. Chem.* **1981,** 1469.
40. ^{[1]} U. Schöllkopf, R. Meyer, *Liebigs Ann. Chem.* **1977,** 1174.
41. ^{[1]} U. Schöflkopf, F. Gerhart, R. Schröder, *Angew. Chem.* **1969,** *81*, 701.
42. ^{[1]} U. Schöllkopf, F. Gerhart, R. Schröder, D. Hoppe, *Liebigs Ann. Chem.* **1972**, **766**, 1174.
43. ^{[1]} R. K. Olsen, A. Srinivasan, K. D. Richards, *Tetrahedron Lett.* **1976, 12**, 891.
44.^{[1]} T. Naito, O. Miyata, T. Shinada, I. Ninomiya, T. Date, K. Okamura, S. Inagaki, *J*. *Org. Chem.* **1991, 56**, 6556.
45. ^{[1]} D. N. Reinhoudt, V. van Axel Castelli, A. Dalla Cort, L. Mandolini, L. Schiaffino, *Chem. Eur. J.* **2000, 6**, 1193.
46. ^{[1]} D. Enders, B. B. Lohray, *Angew. Chem.* **1987, 99**, 360.
47. ^{[1]} D. Enders, B. B. Lohray, *Angew. Chem.* **1988,** *100, 594.*
48.^{[1]} *Beilstein* **4**, *I,* 342.
49.^{[1]} R. G. Jones, J. *Am. Chem. Soc.* **1949,** *71*, 644.
50.^{[1]} I. Maeda, K. Togo, R. Yoshida, *Bull. Chem. Soc. Jpn.* **1971,** *44*,1407.

## Patentansprüche

1. Verfahren zur Herstellung einer Verbindung der allgemeinen Formel **31** gemäß Anspruch 13 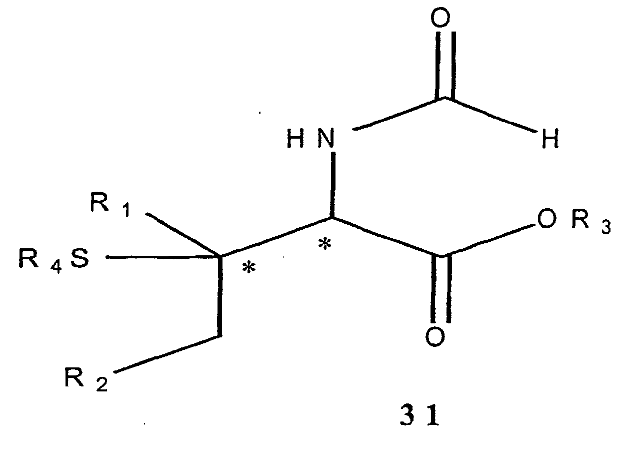 , worin
R1, R2, R3 unabhängigen voneinander ausgewälht sind aus
C₁₋₁₀-Alkyl, gesättigt oder ungesättigt, verzweigt oder unverzweigt, einfach oder mehrfach substituiert oder unsubstituiert; und
* ein stereoselektives Zentrum markiert,
R4 ausgewälht ist aus;
C1-10-Alkyl, gesättigt oder ungesättigt, verzweigt oder unverzweigt, einfach oder mehrfach substituiert oder unsubstituiert; C3-8-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C1-3-Alkyl gebundenem Aryl, C3-8-Cycloalkyl oder Heteroaryl, jeweils unsubstituiert oder einfach oder mehrfach substituiert;
bei dem eine Verbindung der allgemeinen Formel **30**, unter Bedingungen einer Michael-Addition mit einer Verbindung der allgemeinen Formel R₄SH, nach nachfolgender Reaktion I umgesetzt wird: 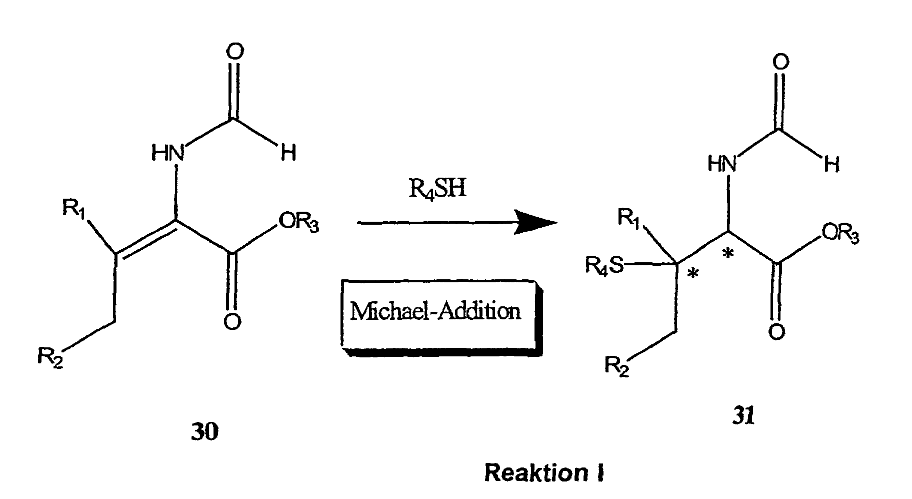 , wobei die Verbindungen der Formel R₄SH als Lithiumthiolate eingesetzt oder in oder vor der Reaktion I zu Lithiumthiolaten umgesetzt werden und/oder chirale Katalysatoren, ausgewählt aus: chiralen Hilfsreagenzien, insbesondere dem Diether (*S,S*)-1,2-Dimethoxy-1,2-diphenylethan; Lewis-Säuren und/oder Brønsted-Basen oder Kombinationen von diesen, eingesetzt werden und gegebenenfalls mit Basen, insbesondere NaOH, anschließend hydrolysiert und gegebenenfalls, vorzugsweise durch Säulenchromatographie, gereinigt wird.

2. Verfahren gemäß Anspruch 1, **dadurch gekennzeichnet, daß** die Verbindungen der Formel R₄SH als Lithiumthiolate eingesetzt oder in oder vor der Reaktion I zu Lithiumthiolaten umgesetzt werden.

3. Verfahren gemäß einem der Ansprüche 1 oder 2, **dadurch gekennzeichnet, daß** vor der Reaktion I zur Umsetzung der Verbindungen der Formel R₄SH zu Lithiumthiolaten Butyl-Lithium (BuLi) eingesetzt, vorzugsweise in einem Äquilvalenten-Verhältnis BuLi : R4SH zwischen 1 : 5 und 1:20, insbesondere 1 : 10, und mit R₄SH umgesetzt wird und/oder die Umsetzung bei Temperaturen ≤ 0°C und/oder in organischem Lösungsmittel, insbesondere Toluol, Ether, THF oder DCM, besonders THF; stattfindet.

4. Verfahren gemäß einem der Ansprüche 1 bis 3, **dadurch gekennzeichnet, daß** zu Beginn der Reaktion I die Reaktionstemperatur bei Temperaturen ≤ 0°C, vorzugsweise bei zwischen -70 und -80°C, insbesondere -78°C, liegt und im Verlauf der Reaktion I die Temperatur auf Raumtemperatur gebracht wird oder die Reaktionstemperatur zu Beginn der Reaktion I bei Temperaturen ≤ 0°C, vorzugsweise bei zwischen -30 und -20°C, insbesondere -25°C, liegt und im Verlauf der Reaktion I die Temperatur auf zwischen - 20°C und -10°C, insbesondere -15°C, gebracht wird.

5. Verfahren gemäß einem der Ansprüche 1 bis 4, **dadurch gekennzeichnet, daß** die Reaktion I in einem organischen Lösungsmittel, vorzugsweise Toluol, Ether, THF oder DCM, insbesondere in THF, bzw. einem unpolaren Lösungsmittel, insbesondere in DCM oder Toluol, stattfindet.

6. Verfahren gemäß einem der Ansprüche 1 bis 5, **dadurch gekennzeichnet, daß** die Diastereomeren nach der Reaktion I getrennt wurden, vorzugsweise durch präparative HPLC oder Kristallisation, insbesondere unter Einsatz des Lösungsmittels Pentan/Ethanol (10:1) und Kühlung.

7. Verfahren gemäß einem der Ansprüche 1 bis 6, **dadurch gekennzeichnet, daß** die Enantiomerentrennung vor der Diastereomerentrennung erfolgt.

8. Verfahren gemäß einem der Ansprüche 1 bis 7, **dadurch gekennzeichnet, daß** R¹ C₁₋₆-Alkyl, gesättigt oder ungesättigt, verzweigt oder unverzweigt, einfach oder mehrfach substituiert oder unsubstituiert; bedeutet und R² C₂₋₉-Alkyl, gesättigt oder ungesättigt, verzweigt oder unverzweigt, einfach oder mehrfach substituiert oder unsubstituiert; bedeutet,
vorzugsweise
R¹ C₁₋₂-Alkyl, einfach oder mehrfach substituiert oder unsubstituiert, insbesondere Methyl oder Ethyl; bedeutet und R² C₂₋₉-Alkyl, vorzugsweise C₂₋₇-Alkyl, gesättigt oder ungesättigt, verzweigt oder unverzweigt, einfach oder mehrfach substituiert oder unsubstituiert, insbesondere Ethyl, Propyl, n-Propyl, i-Propyl, Butyl, n-Butyl, i-Butyl, tert.-Butyl, Pentyl, Hexyl oder Heptyl; bedeutet
insbesondere
der Rest R¹ Methyl bedeutet und R² n-Butyl.

9. Verfahren gemäß einem der Ansprüche 1 bis 8, **dadurch gekennzeichnet, daß** R³ ausgewählt ist aus C₁₋₃-Alkyl, gesättigt oder ungesättigt, verzweigt oder unverzweigt, einfach oder mehrfach substituiert oder unsubstituiert; vorzugsweise Methyl oder Ethyl.

10. Verfahren gemäß einem der Ansprüche 1 bis 9, **dadurch gekennzeichnet, daß** R⁴ ausgewählt ist aus C₁₋₆-Alkyl, gesättigt oder ungesättigt, verzweigt oder unverzweigt, einfach oder mehrfach substituiert oder unsubstituiert; Phenyl oder Thiophenyl, unsubstituiert oder einfach (vorzugsweise mit OCH₃, CH₃, OH, SH, CF₃, F, Cl, Br oder I) substituiert; oder über gesättigtes CH₃-gebundenem Phenyl, unsubstituiert oder einfach (vorzugsweise mit OCH₃, CH₃, OH, SH, CF₃, F, Cl, Br oder I) substituiert;
vorzugsweise R⁴ ausgewählt ist aus C₁₋₆-Alkyl, gesättigt, unverzweigt und unsubstituiert, insbesondere Methyl, Ethyl, Propyl, n-Propyl, i-Propyl, Butyl, n-Butyl, i-Butyl, tert.-Butyl, Pentyl oder Hexyl; Phenyl oder Thiophenyl, unsubstituiert oder einfach (vorzugsweise mit OCH₃, CH₃, OH, SH, CF₃, F, Cl, Br oder I) substituiert; oder über gesättigtes CH₃-gebundenem Phenyl, unsubstituiert oder einfach (vorzugsweise mit OCH₃, CH₃, OH, SH, CF₃, F, Cl, Br oder I) substituiert,
insbesondere R⁴ ausgewählt ist aus Methyl, Ethyl oder Benzyl, unsubstituiert oder einfach (vorzugsweise mit OCH₃, CH₃, OH, SH, CF₃, F, Cl, Br oder I) substituiert.

11. Verfahren gemäß einem der Ansprüche 1 bis 10, **dadurch gekennzeichnet, daß** das Thiolat stöchiometrisch eingesetzt wird, TMSCI verwendet wird und/oder dann ein chiraler Protonendonator R*-H eingesetzt wird,
oder
daß die Verbindung **30** vor der Reaktion I mit einer sterisch anspruchsvollen (großen) Gruppe, vorzugsweise TBDMS modifiziert wird.

12. Verfahren gemäß einem der Ansprüche 1 bis 11, **dadurch gekennzeichnet, daß** die Verbindung nach der allgemeinen Formel **31** 3-Ethylsulfanyl-2-formylamino-3-methyloctansäureethylester oder 3-Benzylsulfanyl-2-formylamino-3-methyloctansäure-ethylester ist, die Verbindung nach der allgemeinen Formel **30** 2-Formylamino-3-methyloct-2-ensäureethylester ist und R₄SH Ethylmercaptan oder Benzylmercaptan ist.

13. Verbindung der allgemeinen Formel **31** 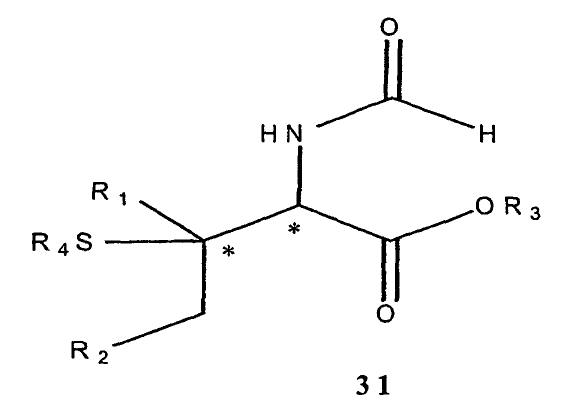 , worin
R1, R2 und R3 unabhängig voneinander ausgewählt sind aus C₁₋₁₀-Alkyl, gesättigt oder ungesättigt, verzweigt oder unverzweigt, einfach oder mehrfach substituiert oder unsubstituiert;
* ein stereoselektives Zentrum markiert, und
R⁴ ausgewählt ist aus:
C₁₋₁₀-Alkyl, gesättigt oder ungesättigt, verzweigt oder unverzweigt, einfach oder mehrfach substituiert oder unsubstituiert; C₃₋₈-Cycloalkyl, gesättigt oder ungesättigt, unsubstituiert oder einfach oder mehrfach substituiert; Aryl-, oder Heteroaryl, jeweils unsubstituiert oder einfach oder mehrfach substituiert; oder über gesättigtes oder ungesättigtes C₁₋₃-Alkyl gebundenem Aryl, C₃₋₈-Cycloalkyl oder Heteroaryl, jeweils unsubstituiert oder einfach oder mehrfach substituiert;
in Form ihrer Razemate; Enantiomere, Diastereomere, insbesondere Mischungen ihrer Enantiomere oder Diastereomere oder eines einzelnen Enantiomers oder Diastereomers; in Form ihrer physiologisch verträglichen sauren und basischen Salze bzw. Salze mit Kationen bzw. Basen oder mit Anionen bzw. Säuren oder in Form der freien Säuren oder Basen.

14. Verbindung gemäß Anspruch 13, **dadurch gekennzeichnet, daß** R¹ C₁₋₆-Alkyl, gesättigt oder ungesättigt, verzweigt oder unverzweigt, einfach oder mehrfach substituiert oder unsubstituiert; bedeutet und R² C₂₋₉-Alkyl, gesättigt oder ungesättigt, verzweigt oder unverzweigt, einfach oder mehrfach substituiert oder unsubstituiert; bedeutet,
vorzugsweise
R¹ C₁₋₂-Alkyl, einfach oder mehrfach substituiert oder unsubstituiert, insbesondere Methyl oder Ethyl; bedeutet und R² C₂₋₉-Alkyl, vorzugsweise C₂₋₇-Alkyl, gesättigt oder ungesättigt, verzweigt oder unverzweigt, einfach oder mehrfach substituiert oder unsubstituiert, insbesondere Ethyl, Propyl, n-Propyl, i-Propyl, Butyl, n-Butyl, i-Butyl, tert.-Butyl, Pentyl, Hexyl oder Heptyl; bedeutet
insbesondere
der Rest R¹ Methyl bedeutet und R² n-Butyl.

15. Verbindung gemäß einem der Ansprüche 13 oder 14, **dadurch gekennzeichnet, daß** R³ ausgewählt ist aus C₁₋₃-Alkyl, gesättigt oder ungesättigt, verzweigt oder unverzweigt, einfach oder mehrfach substituiert oder unsubstituiert; vorzugsweise Methyl oder Ethyl.

16. Verbindung gemäß einem der Ansprüche 13 bis 15, **dadurch gekennzeichnet, daß** R⁴ ausgewählt ist aus C₁₋₆-Alkyl, gesättigt oder ungesättigt, verzweigt oder unverzweigt, einfach oder mehrfach substituiert oder unsubstituiert; Phenyl oder Thiophenyl, unsubstituiert oder einfach (vorzugsweise mit OCH₃, CH₃, OH, SH, CF₃, F, Cl, Br oder I) substituiert; oder über gesättigtes CH₃-gebundenem Phenyl, unsubstituiert oder einfach (vorzugsweise mit OCH₃, CH₃, OH, SH, CF₃, F, Cl, Br oder I) substituiert;
vorzugsweise R⁴ ausgewählt ist aus C₁₋₆-Alkyl, gesättigt, unverzweigt und unsubstituiert, insbesondere Methyl, Ethyl, Propyl, n-Propyl, i-Propyl, Butyl, n-Butyl, i-Butyl, tert.-Butyl, Pentyl oder Hexyl; Phenyl oder Thiophenyl, unsubstituiert oder einfach (vorzugsweise mit OCH₃, CH₃, OH, SH, CF₃, F, Cl, Br oder I) substituiert; oder über gesättigtes CH₃-gebundenem Phenyl, unsubstituiert oder einfach (vorzugsweise mit OCH₃, CH₃, OH, SH, CF₃, F, Cl, Br oder I) substituiert,
insbesondere R⁴ ausgewählt ist aus Methyl, Ethyl oder Benzyl, unsubstituiert oder einfach (vorzugsweise mit OCH₃, CH₃, OH, SH, CF₃, F, Cl, Br oder I) substituiert.

17. Verbindung gemäß einem der Ansprüche 13 bis 16, **dadurch gekennzeichnet, daß** die Verbindung ausgewählt ist aus
• 3-Ethylsulfanyl-2-formylamino-3-methyloctansäureethylester oder
• 3-Benzylsulfanyl-2-formylamino-3-methyloctansäure-ethylester.

## Claims

1. A process for the production of a compound of the general formula **31** according to claim 13, 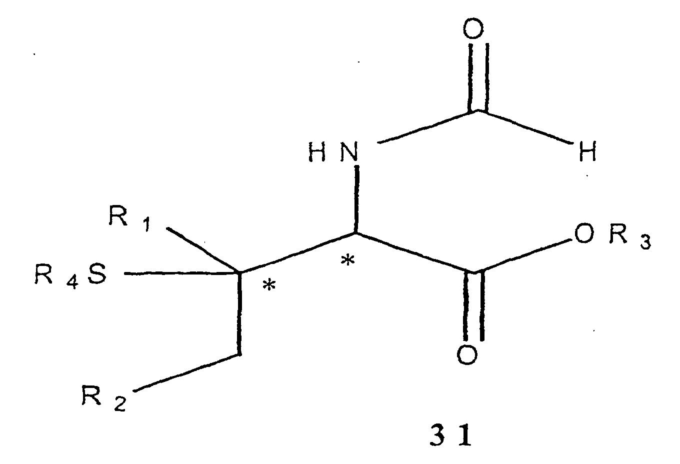 in which
R1, R2 and R3 are mutually independently selected from among
C₁₋₁₀ alkyl, saturated or unsaturated, branched or unbranched, mono- or polysubstituted or unsubstituted; and
* indicates a stereoselective centre,
R4 is selected from among:
C1-10 alkyl, saturated or unsaturated, branched or unbranched, mono- or polysubstituted or unsubstituted; C3-8 cycloalkyl, saturated or unsaturated, unsubstituted or mono- or polysubstituted; aryl or heteroaryl, in each case unsubstituted or mono- or polysubstituted; or aryl, C3-8 cycloalkyl or heteroaryl, in each case unsubstituted or mono- or polysubstituted, attached via saturated or unsaturated C1-3 alkyl;
in which a compound of the general formula **30,** is reacted under *Michael* addition conditions with a compound of the general formula R₄SH, in accordance with reaction I below: 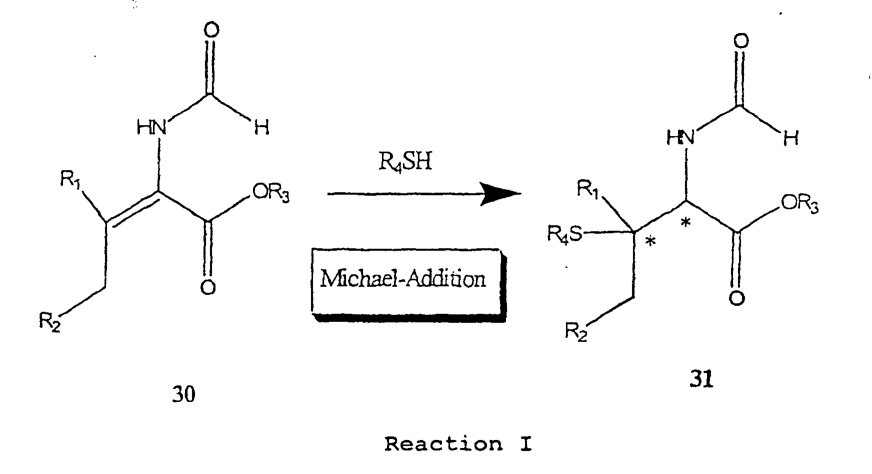 wherein the compounds of the formula R₄SH are used as lithium thiolates or are converted into lithium thiolates during or before reaction I and/or chiral catalysts, selected from among: chiral auxiliary reagents, in particular the diether (*S,S*)-1,2-dimethoxy-1,2-diphenylethane; Lewis acids and/or Brønsted bases or combinations thereof are used, and are optionally then hydrolysed with bases, in particular NaOH, and optionally purified, preferably by column chromatography.

2. A process according to claim 1, **characterised in that** the compounds of the formula R₄SH are used as lithium thiolates or are converted into lithium thiolates during or before reaction I.

3. A process according to one of claims 1 or 2, **characterised in that** butyllithium (BuLi) is used before reaction I to convert the compounds of the formula R₄SH into lithium thiolates, preferably in an equivalent ratio of BuLi:R₄SH of between 1:5 and 1:20, in particular 1:10, and is reacted with R₄SH and/or the reaction proceeds at temperatures of ≤ 0°C and/or in an organic solvent, in particular toluene, ether, THF or DCM, especially THF.

4. A process according to one of claims 1 to 3, **characterised in that**, at the beginning of reaction I, the reaction temperature is at temperatures of ≤ 0°C, preferably at between -70 and -80°C, in particular -78°C, and, over the course of reaction I, the temperature is adjusted to room temperature or the reaction temperature at the beginning of reaction I is at temperatures of ≤ 0°C, preferably at between -30 and -20°C, in particular -25°C, and, over the course of reaction I, the temperature is adjusted to between -20°C and -10°C, in particular -15°C.

5. A process according to one of claims 1 to 4, **characterised in that** reaction I proceeds in an organic solvent, preferably toluene, ether, THF or DCM, in particular in THF, or a nonpolar solvent, in particular in DCM or toluene.

6. A process according to one of claims 1 to 5, **characterised in that** the diastereomers are separated after reaction I, preferably by preparative HPLC or crystallisation, in particular using the solvent pentane/ethanol (10:1) and cooling.

7. A process according to one of claims 1 to 6, **characterised in that** the separation of the enantiomers proceeds before the separation of the diastereomers.

8. A process according to one of claims 1 to 7, **characterised in that** R¹ means C₁₋₆ alkyl, saturated or unsaturated, branched or unbranched, mono- or polysubstituted or unsubstituted; and R² means C₂₋₉ alkyl, saturated or unsaturated, branched or unbranched, mono- or polysubstituted or unsubstituted,
preferably
R¹ means C₁₋₂ alkyl, mono- or polysubstituted or unsubstituted, in particular methyl or ethyl and R² means C₂₋₉ alkyl, preferably C₂₋₇ alkyl, saturated or unsaturated, branched or unbranched, mono- or polysubstituted or unsubstituted, in particular ethyl, propyl, n-propyl, i-propyl, butyl, n-butyl, i-butyl, *tert*.-butyl, pentyl, hexyl or heptyl;
in particular
residue R¹ means methyl and R² means n-butyl.

9. A process according to one of claims 1 to 8, **characterised in that** R³ is selected from among C₁₋₃ alkyl, saturated or unsaturated, branched or unbranched, mono- or polysubstituted or unsubstituted, preferably methyl or ethyl.

10. A process according to one of claims 1 to 9, **characterised in that** R⁴ is selected from among C₁₋₆ alkyl, saturated or unsaturated, branched or unbranched, mono- or polysubstituted or unsubstituted; phenyl or thiophenyl, unsubstituted or monosubstituted (preferably with OCH₃, CH₃, OH, SH, CF₃, F, Cl, Br or I); or phenyl attached via saturated CH₃, unsubstituted or monosubstituted (preferably with OCH₃, CH₃, OH, SH, CF₃, F, Cl, Br or I);
R⁴ is preferably selected from among C₁₋₆ alkyl, saturated, unbranched and unsubstituted, in particular methyl, ethyl, propyl, n-propyl, i-propyl, butyl, n-butyl, i-butyl, *tert*.-butyl, pentyl or hexyl; phenyl or thiophenyl, unsubstituted or monosubstituted (preferably with OCH₃, CH₃, OH, SH, CF₃, F, Cl, Br or I); or phenyl attached via saturated CH₃, unsubstituted or monosubstituted (preferably with OCH₃, CH₃, OH, SH, CF₃, F, Cl, Br or I),
in particular R⁴ is selected from among methyl, ethyl or benzyl, unsubstituted or monosubstituted (preferably with OCH₃, CH₃, OH, SH, CF₃, F, Cl, Br or I).

11. A process according to one of claims 1 to 10, **characterised in that** the thiolate is used stoichiometrically, TMSCl is used and/or a chiral proton donor R*-H is then used,
or
that compound **30** is modified before reaction I with a sterically demanding (large) group, preferably TBDMS.

12. A process according to one of claims 1 to 11, **characterised in that** the compound of the general formula **31** is 3-ethylsulfanyl-2-formylamino-3-methyloctanoic acid ethyl ester or 3-benzylsulfanyl-2-formylamino-3-methyloctanoic acid ethyl ester, the compound of the general formula **30** is 2-formylamino-3-methyl-2-octenoic acid ethyl ester and R₄SH is ethyl mercaptan or benzyl mercaptan.

13. A compound of the general formula **31** 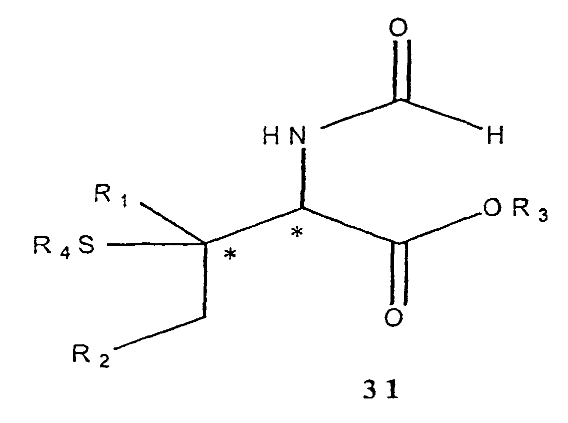 in which
R1, R2 and R3 are mutually independently selected from among C₁₋₁₀ alkyl, saturated or unsaturated, branched or unbranched, mono- or polysubstituted or unsubstituted;
* indicates a stereoselective centre, and
R⁴ is selected from among:
C₁₋₁₀ alkyl, saturated or unsaturated, branched or unbranched, mono- or polysubstituted or unsubstituted; C₃₋₈ cycloalkyl, saturated or unsaturated, unsubstituted or mono- or polysubstituted; aryl or heteroaryl, in each case unsubstituted or mono- or polysubstituted; or aryl, C₃₋₈ cycloalkyl or heteroaryl, in each case unsubstituted or mono- or polysubstituted, attached via saturated or unsaturated C₁₋₃ alkyl;
in the form of the racemates, enantiomers, diastereomers thereof, in particular mixtures of the enantiomers or diastereomers thereof or of a single enantiomer or diastereomer; in the form of their physiologically acceptable acidic and basic salts or salts with cations or bases or with anions or acids or in the form of the free acids or bases.

14. A compound according to claim 13, **characterised in that** R¹ means C₁₋₆ alkyl, saturated or unsaturated, branched or unbranched, mono- or polysubstituted or unsubstituted; and R² means C₂₋₉ alkyl, saturated or unsaturated, branched or unbranched, mono- or polysubstituted or unsubstituted,
preferably
R¹ means C₁₋₂ alkyl, mono- or polysubstituted or unsubstituted, in particular methyl or ethyl and R² means C₂₋₉ alkyl, preferably C₂₋₇ alkyl, saturated or unsaturated, branched or unbranched, mono- or polysubstituted or unsubstituted, in particular ethyl, propyl, n-propyl, i-propyl, butyl, n-butyl, i-butyl, *tert*.-butyl, pentyl, hexyl or heptyl;
in particular
residue R¹ means methyl and R² means n-butyl.

15. A compound according to one of claims 13 or 14, **characterised in that** R³ is selected from among C₁₋₃ alkyl, saturated or unsaturated, branched or unbranched, mono- or polysubstituted or unsubstituted; preferably methyl or ethyl.

16. A compound according to one of claims 13 to 15, **characterised in that** R⁴ is selected from among C₁₋₆ alkyl, saturated or unsaturated, branched or unbranched, mono- or polysubstituted or unsubstituted; phenyl or thiophenyl, unsubstituted or monosubstituted (preferably with OCH₃, CH₃, OH, SH, CF₃, F, Cl, Br or I); or phenyl attached via saturated CH₃, unsubstituted or monosubstituted (preferably with OCH₃, CH₃, OH, SH, CF₃, F, Cl, Br or I);
R⁴ is preferably selected from among C₁₋₆ alkyl, saturated, unbranched and unsubstituted, in particular methyl, ethyl, propyl, n-propyl, i-propyl, butyl, n-butyl, i-butyl, *tert*.-butyl, pentyl or hexyl; phenyl or thiophenyl, unsubstituted or monosubstituted (preferably with OCH₃, CH₃, OH, SH, CF₃, F, Cl, Br or I); or phenyl attached via saturated CH₃, unsubstituted or monosubstituted (preferably with OCH₃, CH₃, OH, SH, CF₃, F, Cl, Br or I),
in particular R⁴ is selected from among methyl, ethyl or benzyl, unsubstituted or monosubstituted (preferably with OCH₃, CH₃, OH, SH, CF₃, F, Cl, Br or I).

17. A compound according to one of claims 13 to 16, **characterised in that** the compound is selected from among
• 3-ethylsulfanyl-2-formylamino-3-methyloctanoic acid ethyl ester or
• 3-benzylsulfanyl-2-formylamino-3-methyloctanoic acid ethyl ester.

## Revendications

1. Procédé de production d'un composé de formule générale 31 suivant la revendication 13 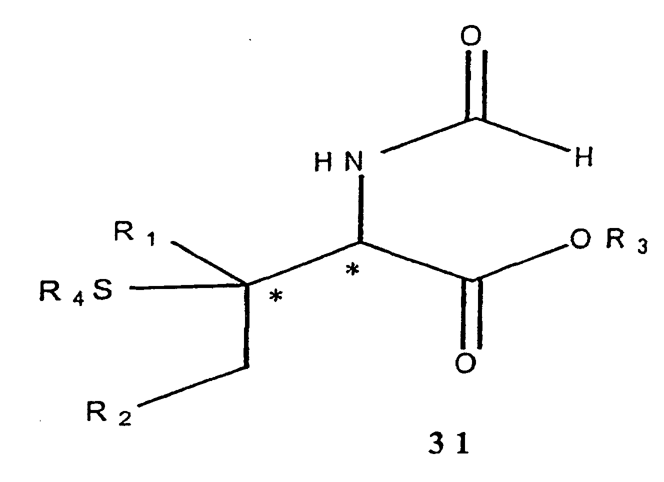 formule dans laquelle
R₁, R₂ et R₃ sont choisis, indépendamment les uns des autres, entre
des radicaux alkyle en C₁ à C₁₀, saturés ou non saturés, ramifiés ou non ramifiés, substitués une ou plusieurs fois ou non substitués ; et
le signe * désigne un centre stéréosélectif,
R₄ est choisi entre :
un reste alkyle en C₁ à C₁₀, saturé ou non saturé, ramifié ou non ramifié, substitué une ou plusieurs fois ou non substitué ; un reste cycloalkyle en C₃ à C₈, saturé ou non saturé, non substitué ou substitué une ou plusieurs fois ; un reste aryle ou hétéroaryle, chacun non substitué ou substitué une ou plusieurs fois ; ou bien un reste aryle, cycloalkyle en C₃ à C₈
ou hétéroaryle lié par l'intermédiaire d'un groupe alkyle en C₁ à C₃ saturé ou non saturé, chacun étant non substitué ou substitué une ou plusieurs fois ;
procédé dans lequel un composé de formule générale 30 est amené à réagir, dans les conditions d'une addition de Michael, avec un composé de formulé générale R₄SH, d'après le schéma réactionnel I suivant : 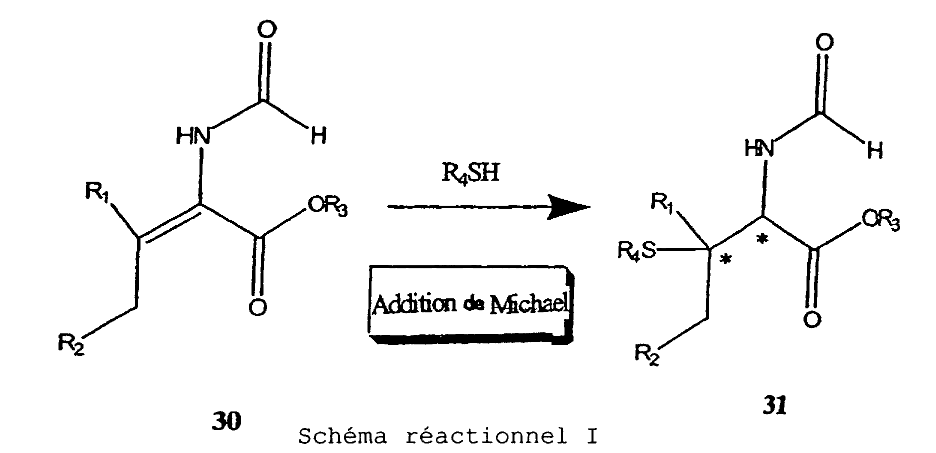 les composés de formule R₄SH étant utilisés sous forme de thiolates de lithium ou transformés pendant ou avant la réaction I en thiolates de lithium, et/ou des catalyseurs chiraux, choisis entre : des réactifs auxiliaires chiraux, en particulier le diéther (S,S)-1,2-diméthoxy-1,2-diphényléthane ; des acides de Lewis et/ou des bases de Brønsted ou des combinaisons de ces composés, sont utilisés, puis, le cas échéant, une hydrolyse est effectuée avec des bases, en particulier NaOH, et une purification est effectuée le cas échéant, avantageusement par chromatographie sur colonne.

2. Procédé suivant la revendication 1, **caractérisé en ce que** les composés de formule R₄SH sont utilisés sous forme de thiolates de lithium ou convertis en thiolates de lithium pendant ou avant la réaction I.

3. Procédé suivant l'une des revendications 1 ou 2, **caractérisé en ce que** du butyllithium (BuLi) est utilisé avant la réaction I pour transformer les composés de formule R₄SH en thiolates de lithium, avantageusement dans un rapport d'équivalents BuLi : R₄SH compris entre 1 : 5 et 1 : 20, notamment égal à 1 : 10, et est amené à réagir avec R₄SH, et/ou la réaction a lieu à des températures inférieures ou égales à 0°C et/ou dans des solvants organiques, en particulier dans le toluène, l'éther, le THF ou le DCM, notamment le THF.

4. Procédé suivant l'une des revendications 1 à 3, **caractérisé en ce que** la température réactionnelle au début de la réaction I a des valeurs inférieures ou égales à 0°C, avantageusement comprises entre - 70 et - 80°C, notamment la valeur - 78°C, et au cours de la réaction I, la température est ramenée à la température ambiante, ou bien la température de réaction au début de la réaction I se situe à des valeurs inférieures ou égales à 0°C, avantageusement entre - 30 et - 20°C, en particulier à - 25°C, et au cours de la réaction I, la température est réglée entre - 20°C et - 10°C, en particulier à - 15°C.

5. Procédé suivant l'une des revendications 1 à 4, **caractérisé en ce que** la réaction I a lieu dans un solvant organique, avantageusement dans le toluène, l'éther, le THF ou le DCM, notamment dans le THF, ou dans un solvant non polaire, en particulier le DCM ou le toluène.

6. Procédé suivant l'une des revendications 1 à 5, **caractérisé en ce que** les diastéréo-isomères sont séparés après la réaction I, avantageusement par CLHP préparative ou par cristallisation, en utilisant en particulier le solvant pentane/éthanol (10 : 1) et avec refroidissement.

7. Procédé suivant l'une des revendications 1 à 6, **caractérisé en ce que** la séparation des énantiomères est effectuée avant la séparation des diastéréo-isomères.

8. Procédé suivant l'une des revendications 1 à 7, **caractérisé en ce que** R¹ représente un reste alkyle en C₁ à C₆, saturé ou non saturé, ramifié ou non ramifié, substitué une ou plusieurs fois ou non substitué ; et R² représente un reste alkyle en C₂ à C₉, saturé ou non saturé, ramifié ou non ramifié, substitué une ou plusieurs fois ou non substitué ;
avantageusement,
R¹ désigne un reste alkyle en C₁ ou C₂, substitué une ou plusieurs fois ou non substitué,
en particulier le reste méthyle ou éthyle ; et R² est un reste alkyle en C₂ à C₉, avantageusement un reste alkyle en C₂ à C₇, saturé ou non saturé, ramifié ou non ramifié, substitué une ou plusieurs fois ou non substitué, en particulier le reste éthyle, propyle, n-propyle, isopropyle, butyle, n-butyle, isobutyle, tertiobutyle, pentyle, hexyle ou heptyle ;
en particulier
R¹ est le reste méthyle et R² est le reste n-butyle.

9. Procédé suivant l'une des revendications 1 à 8, **caractérisé en ce que** R³ est choisi entre des restes alkyle en C₁ à C₃, saturés ou non saturés, ramifiés ou non ramifiés, substitués une ou plusieurs fois ou non substitués, avantageusement le reste méthyle ou éthyle.

10. Procédé suivant l'une des revendications 1 à 9, **caractérisé en ce que** R⁴ est avantageusement choisi entre un reste alkyle en C₁ à C₆, saturé ou non saturé, ramifié ou non ramifié, substitué une ou plusieurs fois ou non substitué ; un reste phényle ou thiophényle, non substitué ou substitué une fois (avantageusement par un groupe OCH₃, CH₃, OH, SH, CF₃, F, Cl, Br ou I) ; ou un reste phényle lié par l'intermédiaire d'un groupe CH₃ saturé, non substitué ou substitué une fois (avantageusement par un groupe OCH₃, CH₃, OH, SH, CF₃, F, Cl, Br ou I),
R⁴ est avantageusement choisi entre un reste alkyle en C₁ à C₆, saturé, non ramifié et non substitué, en particulier méthyle, éthyle, propyle, n-propyle, isopropyle, butyle, n-butyle, isobutyle, tertiobutyle, pentyle ou hexyle ; un reste phényle ou thiophényle, non substitué ou substitué une fois (avantageusement par OCH₃, CH₃, OH, SH, CF₃, F, Cl, Br ou I) ; ou un reste phényle, lié par l'intermédiaire d'un groupe saturé CH₃, non substitué ou substitué une fois (avantageusement par OCH₃, CH₃, OH, SH, CF₃, F, Cl, Br ou I),
R⁴ étant choisi en particulier entre un reste méthyle, éthyle ou benzyle, non substitué ou substitué une fois (avantageusement par OCH₃, CH₃, OH, SH, CF₃, F, Cl, Br ou I).

11. Procédé suivant l'une des revendications 1 à 10, **caractérisé en ce que** le thiolate est utilisé en proportion stoechiométrique, du TMSCl est utilisé et/ou un donneur chiral de protons R*-H est utilisé ensuite, ou **en ce que** le composé 30 est modifié avant la réaction I avec un (grand) groupe à encombrement stérique, avantageusement TBDMS.

12. Procédé suivant l'une des revendications 1 à 11, **caractérisé en ce que** le composé de formule générale 31 est l'ester éthylique d'acide 3-éthylsulfanyl-2-formylamino-3-méthyloctanoïque ou l'ester éthylique d'acide 3-benzylsulfanyl-2-formylamino-3-méthyloctanoïque, le composé de formule générale 30 est l'ester éthylique d'acide 2-formylamino-3-méthyloct-2-énoïque et R₄SH est l'éthylmercaptan ou le benzylmercaptan.

13. Composé de formule générale 31 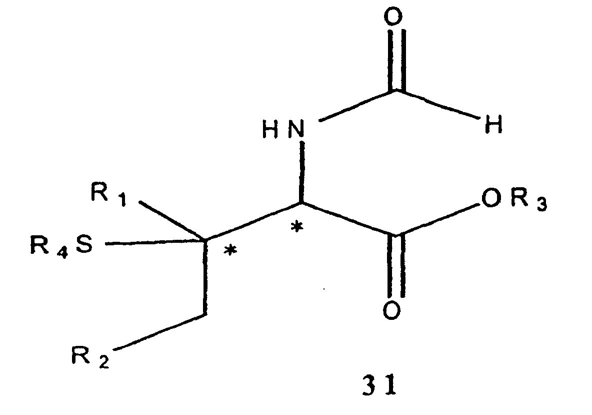 dans laquelle
R₁, R₂ et R₃ sont choisis, indépendamment les uns des autres, entre
des restes alkyle en C₁ à C₁₀, saturés ou non saturés, ramifiés ou non ramifiés, substitués une ou plusieurs fois ou non substitués,
le signe * désigne un centre stéréosélectif, et
R₄ est choisi entre :
un reste alkyle en C₁ à C₁₀, saturé ou non saturé, ramifié ou non ramifié, substitué une ou plusieurs fois ou non substitué ; un reste cycloalkyle en C₃ à C₈, saturé ou non saturé, non substitué ou substitué une ou plusieurs fois ; un reste aryle ou hétéroaryle, chacun non substitué ou substitué une ou plusieurs fois ; ou bien un reste aryle, cycloalkyle en C₃ à C₈ ou hétéroaryle lié par l'intermédiaire d'un groupe alkyle en C₁ à C₃ saturé ou non saturé, chacun non substitué ou substitué une ou plusieurs fois ;
sous forme de ses racémates ; énantiomères, diastéréo-isomères, en particulier mélanges de ses énantiomères ou diastéréo-isomères ou d'un énantiomère ou diastéréo-isomère individuel ; sous forme de ses sels acides ou basiques acceptables du point de vue physiologique ou de ses sels formés avec des cations ou des bases ou avec des anions et des acides ou sous forme des acides ou des bases libres.

14. Composé suivant la revendication 13, **caractérisé en ce que** R¹ est un reste alkyle en C₁ à C₆, saturé ou non saturé, ramifié ou non ramifié, substitué une ou plusieurs fois ou non substitué ; et R² est un reste alkyle en C₂ à C₉, saturé ou non saturé, ramifié ou non ramifié, substitué une ou plusieurs fois ou non substitué ; avantageusement
R¹ est un reste alkyle en C₁ ou C₂, substitué une ou plusieurs fois ou non substitué, en particulier méthyle ou éthyle ; et R² est un reste alkyle en C₂ à C₉, avantageusement un reste alkyle en C₂ à C₇, saturé ou non saturé, ramifié ou non ramifié, substitué une ou plusieurs fois ou non substitué, en particulier éthyle, propyle, n-propyle, isopropyle, butyle, n-butyle, isobutyle, tertiobutyle, pentyle, hexyle ou heptyle ; notamment
R¹ est le reste méthyle et R² est le reste n-butyle.

15. Composé suivant l'une des revendications 13 ou 14, **caractérisé en ce que** R³ est choisi entre des restes alkyle en C₁ à C₃, saturés ou non saturés, ramifiés ou non ramifiés, substitués une ou plusieurs fois ou non substitués ; avantageusement le reste méthyle ou éthyle.

16. Composé suivant l'une des revendications 13 à 15, **caractérisé en ce que** R⁴ est choisi entre un reste alkyle en C₁ à C₆, saturé ou non saturé, ramifié ou non ramifié, substitué une ou plusieurs fois ou non substitué ; un reste phényle ou thiophényle, non substitué ou substitué une fois (avantageusement par OCH₃, CH₃, OH, SH, CF₃, F, Cl, Br ou I) ; ou un reste phényle, lié par l'intermédiaire d'un groupe saturé CH₃, non substitué ou substitué une fois (avantageusement par un groupe OCH₃, CH₃, OH, SH, CF₃, F, Cl, Br ou I) ;
R⁴ est avantageusement choisi entre un reste alkyle en C₁ à C₆, saturé, non ramifié et non substitué, en particulier méthyle, éthyle, propyle, n-propyle, isopropyle, butyle, n-butyle, isobutyle, tertiobutyle, pentyle ou hexyle ; un reste phényle ou thiophényle, non substitué ou substitué une fois (avantageusement par un groupe OCH₃, CH₃, OH, SH, CF₃, F, Cl, Br ou I) ; ou un reste phényle, lié par l'intermédiaire d'un groupe saturé CH₃, non substitué ou substitué une fois (avantageusement par un groupe OCH₃, CH₃, OH, SH, CF₃, F, Cl, Br ou I),
R⁴ étant choisi en particulier entre un reste méthyle, éthyle ou benzyle, non substitué ou substitué une fois (avantageusement par un groupe OCH₃, CH₃, OH, SH, CF₃, F, Cl, Br ou I).

17. Composé suivant l'une des revendications 13 à 16, **caractérisé en ce que** le composé est choisi entre
• l'ester éthylique d'acide 3-éthylsulfanyl-2-formylamino-3-méthyloctanoïque ou
• l'ester éthylique d'acide 3-benzylsulfanyl-2-formylamino-3-méthyloctanoïque.
